(19) Europäisches Patentamt
European Patent Office
Office européen des brevets

(11) EP 4 565 562 B1

(12) **EUROPEAN PATENT SPECIFICATION**

(45) Date of publication and mention of the grant of the patent:
**27.05.2026 Bulletin 2026/22**

(21) Application number: **23754385.5**

(22) Date of filing: **02.08.2023**

(51) International Patent Classification (IPC):
***C07D 207/16*** (2006.01) ***C07D 209/52*** (2006.01)
***A61K 31/40*** (2006.01) ***A61K 31/403*** (2006.01)
***A61P 1/00*** (2006.01) ***A61P 1/04*** (2006.01)
***A61P 1/06*** (2006.01) ***A61P 1/10*** (2006.01)
***A61P 1/12*** (2006.01) ***A61P 11/00*** (2006.01)
***A61P 11/06*** (2006.01) ***A61P 11/08*** (2006.01)
***A61P 11/14*** (2006.01)

(52) Cooperative Patent Classification (CPC):
**C07D 207/16; A61P 1/00; A61P 1/04; A61P 1/06; A61P 1/10; A61P 1/12; A61P 11/00; A61P 11/06; A61P 11/08; A61P 11/14; C07D 209/52**

(86) International application number:
**PCT/GB2023/052049**

(87) International publication number:
**WO 2024/028603 (08.02.2024 Gazette 2024/06)**

(54) **PYRROLIDINE-2-CARBOXAMIDE DERIVATIVES AS PROSTAGLANDIN E2 RECEPTOR 4 (EP4) AGONISTS FOR THE TREATMENT OF GASTROINTESTINAL AND PULMONARY DISEASES**

PYRROLIDINE-2-CARBOXAMID-DERIVATE ALS PROSTAGLANDIN E2 RECEPTOR 4 (EP4) AGONISTEN ZUR BEHANDLUNG VON MAGEN-DARM- UND LUNGEN-ERKRANKUNGEN

DÉRIVÉS DE PYRROLIDINE-2-CARBOXAMIDE EN TANT QU'AGONISTS DU RÉCÉPTEUR 4 DE PROSTAGLANDIN E2 (EP4) POUR LE TRAITEMENT DE MALADIES GASTRO-INTESTINALES ET PULMONAIRES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **02.08.2022 GB 202211233**

(43) Date of publication of application:
**11.06.2025 Bulletin 2025/24**

(73) Proprietor: **Nxera Pharma UK Limited**
**Cambridge, Cambridgeshire CB21 6DG (GB)**

(72) Inventors:
- **SWAIN, Nigel Alan**
**Cambridge Cambridgeshire CB21 6DG (GB)**
- **WHITEHURST, Benjamin**
**Cambridge Cambridgeshire CB21 6DG (GB)**
- **CONGREVE, Miles Stuart**
**Cambridge Cambridgeshire CB21 6DG (GB)**
- **BROWN, Giles Albert**
**Cambridge Cambridgeshire CB21 6DG (GB)**

(74) Representative: **Gill Jennings & Every LLP**
**The Broadgate Tower**
**20 Primrose Street**
**London EC2A 2ES (GB)**

(56) References cited:
**WO-A1-2013/004291**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 565 562 B1

## Description

TECHNICAL FIELD OF THE INVENTION

This application relates to novel compounds and their use as prostaglandin $E_2$ receptor 4 ($EP_4$) agonists. Compounds described herein may be useful in the treatment or prevention of diseases in which $EP_4$ receptors are involved. The application is also directed to pharmaceutical compositions comprising these compounds and the manufacture and use of these compounds and compositions in the prevention or treatment of such diseases in which $EP_4$ receptors are involved.

BACKGROUND OF THE INVENTION

Prostanoids including prostaglandins and thromboxanes are metabolite derivatives of arachidonic acid that play key roles in cellular physiological function. Arachidonic acid is an integral component of membrane phospholipids which is released via the activity of phospholipase A2 ($PLA_2$). The biosynthesis of prostaglandins is mediated via cyclooxygenase (COX) which catalyses conversion of arachidonic acid to an unstable intermediate ($PGH_2$) and leads to the generation of prostaglandins including $PGE_2$. $PGE_2$ represents the most widely produced prostanoid whose activity is mediated through action at 4 functionally distinct subtypes of receptors, EP1-4.

EP receptors belong to a family of G protein coupled receptors (GPCR) which are integral membrane proteins with seven-transmembrane domains. This receptor class can broadly be classified according to their signaling pathways: i) Gs coupled receptors (adenylate cyclase activation and cyclic adenosine monophosphate (cAMP) production), EP2 and EP4 ii) Gq coupled receptor (PLC activation), EP1 and iii) Gi coupled receptor (inhibition of adenylate cyclase), EP3.

EP4 receptor signals through Gs and couples positively to adenylate cyclase to increase cAMP levels. The receptor was originally described in 1993 with the identification of an EP2 like receptor which couples positively to adenylate cyclase but does not bind butaprost (A Honda et al. J. Biol. Chem. 1993, 268, 7759-7762).

The EP4 receptor plays key roles in diverse physiological functions including gastrointestinal homeostasis, regulation of vascular tone, renal function, inflammation, fever and carcinogenesis. The potent biological action of PGE2 has fuelled interest in developing subtype selective EP4 agonists and antagonists for the treatment of a broad range of indications.

Functional gastrointestinal diseases (FGIDs), including chronic constipation, are common gastrointestinal conditions encountered by primary care physicians and gastroenterologists. The prevalence of chronic constipation ranges from 1% to 8% and can negatively impact the quality of life (QoL), resulting in major social and economic burden. Chronic constipation can bring discomfort to patients and affect their daily lives. Symptoms include hard and lumpy stools, straining during defecation and a sensation of incomplete evacuation. The use of laxatives and stool softners remains high, despite many patients not obtaining substantial benefit. There remains a need for treatments that can provide complete and sustainable symptomatic relief.

The intestinal mucosa plays an important role in the homeostasis of anion and body fluid maintenance. These functions are mediated through coordinated ion transport via membrane bound transporters and channels localized on the apical and basolateral membrane of intestinal epithelial cells. PGE2 is a well-established secretagogue that may directly promote chloride secretion from intestinal epithelial cells. The secretory effects of PGE2 are in part mediated via the EP4 receptor which can stimulate anion chloride secretion across the gut mucosa. Lubiprostone, a bicyclic fatty acid derivative of PGE1 clinically approved for the treatment of chronic constipation and IBS-C has been shown to promote fluid secretion and gastrointestinal tract motility through the activation of prostaglandin (EP4) receptors. EP4 selective agonists may have therapeutic value in promoting intestinal fluid homeostasis in chronic constipation conditions.

Inflammatory bowel disease (IBD) is a chronic debilitating gastrointestinal disorder that includes ulcerative colitis and Crohn's disease. Patients with IBD commonly present with symptoms that include diarrhoea, abdominal pain, weight loss, rectal bleeding and fever. Clinical management involves strategies to control the abnormal dysregulated immune response in the intestinal mucosa. A wide range of agents are employed to induce and maintain remission, depending on the severity of the disease. These include aminosalicylates, corticosteroids, immunosuppressive agents, antibiotics and biologic agents. This may be given in a stepwise approach, with intensification of therapy according to the severity and progression of the disease. It is clear, however, that some patients are refractory to medical therapy or fail to tolerate it due to systemic side effects. Only a subset of patients achieve long term remission with current therapeutic strategies, suggesting the need for improved therapies.

Intestinal barrier dysfunction plays a key pathogenic role in IBD and there is emerging interest in the development of agents that restore barrier function (mucosal healing). EP4 is expressed in a number of cell types including gastro-intestinal (GI) epithelial cells, lamina propria mononuclear cells and colon innervating sensory neurones and may offer benefits in dampening the inappropriate mucosal immune response as well as protecting the GI mucosal barrier. PGE2 signalling through EP4 promotes cellular differentiation to a wound-associated epithelial cell phenotype which is important

for wound repair (Miyoshi, H. et al. EMBO J. 2017, 36, 5-24). Administration of EP4 agonists provide benefit in chemically induced colitis models (Kabashima, K. et al. J. Clin. Invest. 2002, 109, 883-893; Watanabe, Y. et al. Eur. J. Pharmacol. 2015, 754, 179-189; Nitta, M. et al. Scand. J. Immunol. 2002, 56, 66-75) while mutant mice lacking EP4 develop severe dextran sodium sulfate induced colitis characterised by impaired mucosal barrier function, increased epithelial cell loss, crypt damage and increased immune cell infiltration (Kabashima, K. et al. J. Clin. Invest. 2002, 109, 883-893). In a small PhII study, the EP4 agonist, ONO-4819CD was evaluated in patients with mild to moderate ulcerative colitis refractory to 5-ASA (Nakase, H. et al. Inflamm. Bowel Dis. 2010, 16, 731-733). Although the study was not powered for efficacy, patients treated with ONO-4819CD showed signs of improved disease activity index (DAI) score and histological scores. These data support a potential clinical benefit of EP4 agonist therapy in IBD.

Asthma and chronic obstructive pulmonary disease are inflammatory diseases of the airway characterised by limitations in airflow. It is estimated that approximately 300 million people have asthma and represents the most common chronic disease in children. Despite the advances in the management of asthma, a significant proportion of patients have uncontrolled disease which can lead to mortality and morbidity. Therapies that can achieve effective control of asthma symptoms and reduce risk of future exacerbations for long term management are still needed. PGE2 is known to have bronchodilator and anti-inflammatory effects in rodent and human isolated airway smooth muscle. Inhaled PGE2 has been shown to be beneficial on inflammation and airway calibre in patients with chronic bronchitis and asthma. PGE2 however, also induces a reflex cough, potentially via EP3 receptor mediated irritancy of the upper airway. This has led to efforts to discover EP receptor selective agents for the treatment of airway disorders. Interestingly, key species differences have been reported in the receptor subtype responsible for mediating airway smooth muscle relaxation. In guinea pig, monkey and mouse, EP2 agonists could induce relaxation of airway smooth muscle, whilst in human, this is mediated via the EP4 receptor (Buckley, J. et al. Thorax 2011, 66, 1029-1035). Selective EP4 receptor agonists may have potential therapeutic value in airway diseases.

EP4 receptor has been shown to exert roles in the regulation of blood pressure. EP4 is expressed on smooth muscle and endothelial cells and may induce vasodilatory effects via endothelial nitric oxide synthase (eNOS) mediated nitric oxide (NO) production. PGE2 has been shown to dose dependently relax smooth muscle in aortic rings, an effect that is abolished in EP4 knockout mouse. In halothane anesthetised dogs, the EP4 selective agonist, ONO-AE1-329 induces a vasodepressor response (Honda, A. et al. Eur. J. Pharmacol. 2016, 775, 130-137) and similarly, hypotension was reported as one of the adverse drug reactions in IBD patients receiving ONO-4819CD (Nakase, H. et al. Inflamm. Bowel Dis. 2010, 16, 731-733).

WO 2013/004291 A1 discloses a group of cyclic amine derivatives having EP4 receptor agonistic activity.

EP4 agonists that can be used for the treatment of a range of gastrointestinal and airway disorders without cardiovascular systemic side effects may have potential therapeutic value. In particular, EP4 agonists that can be used for the treatment of a range of gastrointestinal disorders without cardiovascular systemic side effects may have potential therapeutic value. The discovery of safe and effective EP4 selective agents are needed.

## SUMMARY OF THE INVENTION

The present invention provides compounds having activity as prostaglandin $E_2$ receptor 4 ($EP_4$) agonists.

In one aspect the invention provides a compound of Formula I:

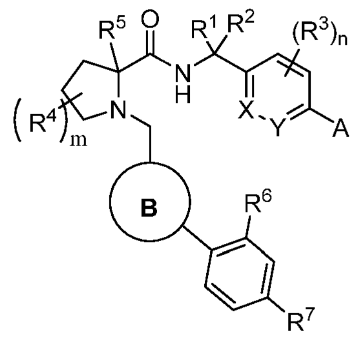

I

or a pharmaceutically acceptable salt, solvate, hydrate, tautomer or optical isomer thereof, wherein:

A is OR', C(O)R', $CO_2R'$, C(O)N(R')$_2$, C(O)N(R')S(O)$_2$R', S(O)$_2$R', S(O)$_2$OR', $SO_2N(R')_2$, $C_{1-8}$ alkyl, cycloalkyl, heterocycloalkyl, aryl, or heteroaryl;

Ring B is aryl or heteroaryl;

X and Y are each independently CR" or N, wherein at least one of X and Y is CH;

$R^1$ and $R^2$ are each independently H, $C_{1-6}$ alkyl, $C_{1-6}$ alkoxy, or $R^1$ and $R^2$, together with the carbon atom to which they are attached, form a $C_{3-6}$ cycloalkane-1,1-diyl;
each $R^3$ is independently selected from H, OR', COOR', C(O)R', halo, or $C_{1-6}$ alkyl;
$R^4$ is H, $C_{1-6}$ alkyl, halo, CN, $NO_2$, or OR';
$R^5$ is H or $C_{1-6}$ alkyl; or $R^4$ and $R^5$, together with the pyrrolidine ring to which they are attached, form a $C_{1-6}$ alkylene linker;
$R^6$ is H, $C_{1-6}$ alkyl, $C_{1-3}$ alkoxy optionally substituted with 1-3 fluorine atoms, halo, CN, $NO_2$, OR', COOR', or C(O)R',
$R^7$ is OR', OC(O)R', OC(O)OR', $CO_2R'$, $CON(R')_2$, $SO_2N(R')_2$, $SO_2R'$, $OSO_2R'$, or $OSO_2N(R')_2$;
each R' is independently H, $C_{1-6}$ alkyl, or $C_{3-6}$ cycloalkyl;
each R" is H, $C_{1-6}$ alkyl, halo, or OR': and
n and m are each independently 0, 1, 2, or 3;
wherein at each occurrence, alkyl, alkylene, and cycloalkyl are each optionally and independently substituted with up to 3 instances of OH', SH, CN, $NO_2$, COOH, halo, or $COOC_{1-4}$ alkyl;
wherein heterocycloalkyl, aryl, and heteroaryl are each optionally and independently substituted with up to 3 instances of OR', SR', CN, $NO_2$, COOR', halo, $C_{1-4}$ alkyl, or oxo.

In another aspect, the invention provides a compound of Formula (1):

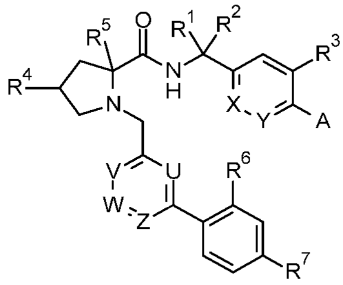
(1)

or a pharmaceutically acceptable salt, solvate, hydrate, tautomer or optical isomer thereof, wherein;
A is selected from the group consisting of:

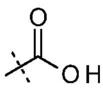, 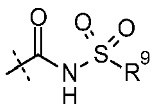, 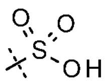, 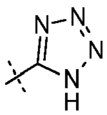, 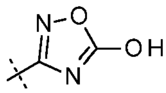, 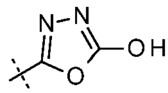,

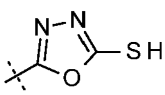, 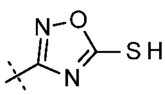, 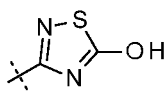, 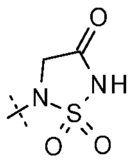, 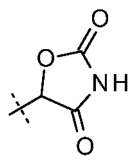;

U, V, W, and Z are each independently selected from the group consisting of CH, COH, N or $N^+-O^-$, wherein at least three of U, V, W, and Z are CH;
X and Y are each independently selected from the group consisting of CH, CF, COH or N; $R^1$ is H, $C_{1-3}$ alkyl optionally substituted with 1-3 fluorine atoms or is joined to $R^2$ to form a $C_{3-6}$ cycloalkyl ring which is optionally substituted with 1-3 fluorine atoms;
$R^2$ is H, $C_{1-3}$ alkyl optionally substituted with 1-3 fluorine atoms or is joined to $R^1$ to form a $C_{3-6}$ cycloalkyl ring which is optionally substituted with 1-3 fluorine atoms;

$R^3$ is H, OH or F;
$R^4$ is H, OH, F or is joined to $R^5$ to form a $CH_2$ bridge;
$R^5$ is H or is joined to $R^4$ to form a $CH_2$ bridge;
$R^6$ is H, OH, CN, halo, $C_{1-3}$ alkoxy optionally substituted with 1-3 fluorine atoms or $C_{13}$ alkyl optionally substituted with 1-3 fluorine atoms;
$R^7$ is OH, $CO_2H$, $CONH_2$, $SO_2NH_2$ or $OSO_2NH_2$; and

$R^9$ is $C_{1-3}$ alkyl or a $C_{3-6}$ cycloalkyl ring.

In another aspect, the invention includes a pharmaceutical composition comprising a compound described herein, and a pharmaceutically acceptable excipient.

In another aspect, the invention includes a kit comprising a compound described herein and at least one additional therapeutic agent selected from the group consisting of aminosalicylates, corticosteroids, immunomodulators and combinations thereof.

In another aspect, the invention includes a compound described herein, a composition described herein, or a kit described herein for use as a medicament.

In another aspect, the invention includes a compound described herein, a composition described herein, or a kit described herein for use in the treatment of an EP4 receptor mediated disease.

In another aspect, the invention includes a compound or composition described herein for use in a method of modulating EP4 receptor agonist activity in a biological sample, the method comprising contacting said EP4 receptor with a compound or composition described herein.

In another aspect, the invention includes a compound or composition described herein for use in a method of treating an EP4 receptor mediated disease, the method comprising administering to a patient in need thereof a compound or composition described herein.

The compounds herein may be used as $EP_4$ receptor agonists. The compounds herein may selectively act at $EP_4$ receptor. The compounds may be used in the manufacture of compositions or medicaments. The compounds, compositions or medicaments may be used in treating, preventing, ameliorating, controlling or reducing the risk of diseases or disorders in which $EP_4$ receptors are involved. The compounds, compositions or medicaments may be used in treating, preventing, ameliorating, controlling or reducing the risk of gastrointestinal disorders and conditions, including but not limited to constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease and ischemic colitis.

The compounds, compositions or medicaments may also be used in treating, preventing, ameliorating, controlling or reducing the risk of pulmonary diseases and conditions such as chronic obstructive pulmonary diseases, asthma, chronic bronchitis, cystic fibrosis, emphysema, chronic idiopathic cough, hyperactive airway disorder and idiopathic pulmonary fibrosis.

## DETAILED DESCRIPTION OF THE INVENTION

The invention relates to novel compounds. The invention also relates to the use of novel compounds as agonists of the $EP_4$ receptor. The invention further relates to the use of novel compounds in the manufacture of medicaments for use as $EP_4$ receptor agonists and the compounds of the invention for use in methods of treatment comprising administering a compound of the invention as an $EP_4$ receptor agonist.

The compounds of Formula I may be used in treating, preventing, ameliorating, controlling or reducing the risk of diseases or disorders in which $EP_4$ receptors are involved. The compounds of Formula I may be used in treating, preventing, ameliorating, controlling or reducing the risk of gastrointestinal disorders and conditions, including but not limited to constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease and ischemic colitis.

The compounds of Formula I may also be used in treating, preventing, ameliorating, controlling or reducing the risk of pulmonary diseases and conditions such as chronic obstructive pulmonary diseases, asthma, chronic bronchitis, cystic fibrosis, emphysema, chronic idiopathic cough, hyperactive airway disorder and idiopathic pulmonary fibrosis.

Certain novel compounds of the invention show particularly high activities as $EP_4$ receptor agonists.

The compounds of the invention have been demonstrated to have activity as $EP_4$ receptor agonists. Compounds of the invention also possess low gastrointestinal permeability, as demonstrated by Caco-2 studies. It is therefore believed that the compounds of the invention exhibit low systemic bioavailability when administered orally. Functional agonism of $EP_4$ receptors expressed in the gastrointestinal tract has the potential to treat a range of gastrointestinal disorders. The

combination of $EP_4$ receptor agonist activity and low gastrointestinal permeability suggests that compounds of the invention are useful for the treatment of a range of gastrointestinal disorders without cardiovascular side effects arising from systemic distribution.

In one aspect the invention provides a compound of Formula I:

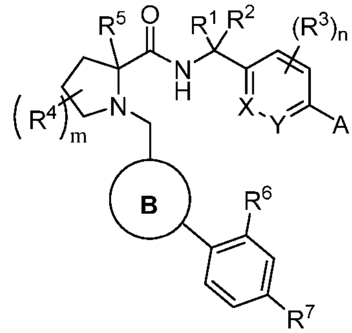

I

or a pharmaceutically acceptable salt, solvate, hydrate, tautomer or optical isomer thereof, wherein;

A is OR', C(O)R', $CO_2R'$, $C(O)N(R')_2$, $C(O)N(R')S(O)_2R'$, $S(O)_2R'$, $S(O)_2OR'$, $SO_2N(R')_2$, $C_{1-8}$ alkyl, cycloalkyl, heterocycloalkyl, aryl, or heteroaryl;
Ring B is aryl or heteroaryl;
X and Y are each independently CR" or N, wherein at least one of X and Y is CH;
$R^1$ and $R^2$ are each independently H, $C_{1-6}$ alkyl, $C_{1-6}$ alkoxy, or $R^1$ and $R^2$, together with the carbon atom to which they are attached, form a $C_{3-6}$ cycloalkand-1,1-diyl;
each $R^3$ is independently selected from H, OR', COOR', C(O)R', halo, or $C_{1-6}$ alkyl;
$R^4$ is H, $C_{1-6}$ alkyl, halo, CN, $NO_2$, or OR';
$R^5$ is H or $C_{1-6}$ alkyl alkyl; or $R^4$ and $R^5$, together with the pyrrolidine ring to which they are attached, form a $C_{1-6}$ alkylene linker;
$R^6$ is H, $C_{1-6}$ alkyl, $C_{1-3}$ alkoxy optionally substituted with 1-3 fluorine atoms, halo, CN, $NO_2$, OR', COOR', or C(O)R',
$R^7$ is OR', OC(O)R', OC(O)OR', $CO_2R'$, $CON(R')_2$, $SO_2N(R')_2$, $SO_2R'$, $OSO_2R'$, or $OSO_2N(R')_2$;
each R' is independently H, $C_{1-6}$ alkyl, or $C_{3-6}$ cycloalkyl;
each R" is H, $C_{1-6}$ alkyl, halo, or OR': and
n and m are each independently 0, 1, 2, or 3;
wherein at each occurrence, alkyl, alkkylene, and cycloalkyl are each optionally and independently substituted with up to 3 instances of OH', SH, CN, $NO_2$, COOH, halo, or $COOC_{1-4}$ alkyl;
wherein heterocycloalkyl, aryl, and heteroaryl are each optionally and independently substituted with up to 3 instances of OR', SR', CN, $NO_2$, COOR', halo, $C_{1-4}$ alkyl, or oxo.

In some embodiments, the compound is a compound of Formula I:

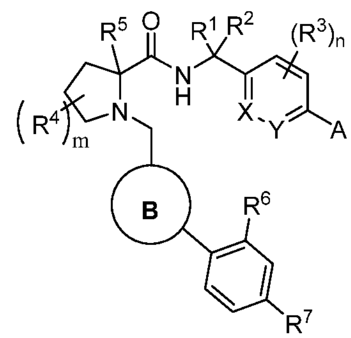

I

or a pharmaceutically acceptable salt, solvate, hydrate, tautomer or optical isomer thereof, wherein;

A is OR', C(O)R', $CO_2R'$, $C(O)N(R')_2$, $C(O)N(R')S(O)_2R'$, $S(O)_2R'$, $S(O)_2OR'$, $SO_2N(R')_2$, $C_{1-8}$ alkyl, cycloalkyl, heterocycloalkyl, aryl, or heteroaryl;
Ring B is aryl or heteroaryl;

X and Y are each independently CR" or N, wherein at least one of X and Y is CH;
$R^1$ and $R^2$ are each independently H, $C_{1-6}$ alkyl, $C_{1-6}$ alkoxy, or $R^1$ and $R^2$, together with the carbon atom to which they are attached, form a $C_{3-6}$ cycloalkand-1,1-diyl;
each $R^3$ is independently selected from H, OR', COOR', C(O)R', halo, or $C_{1-6}$ alkyl;
$R^4$ is $C_{1-6}$ alkyl, halo, CN, $NO_2$, or OR';
$R^5$ is H or $C_{1-6}$ alkyl alkyl; or $R^4$ and $R^5$, together with the pyrrolidine ring to which they are attached, form a $C_{1-6}$ alkylene linker;
$R^6$ is H, $C_{1-6}$ alkyl, halo, CN, $NO_2$, OR', COOR', or C(O)R',
$R^7$ is OR', OC(O)R', OC(O)OR', $CO_2R'$, $CON(R')_2$, $SO_2N(R')_2$, $SO_2R'$, $OSO_2R'$, or $OSO_2N(R')_2$;
each R' is independently H, $C_{1-6}$ alkyl, or $C_{3-6}$ cycloalkyl;
each R" is H, $C_{1-6}$ alkyl, halo, or OR': and
n and m are each independently 0, 1, 2, or 3;
wherein at each occurrence, alkyl, alkkylene, and cycloalkyl are each optionally and independently substituted with up to 3 instances of OH', SH, CN, $NO_2$, COOH, halo, or $COOC_{1-4}$ alkyl;
wherein heterocycloalkyl, aryl, and heteroaryl are each optionally and independently substituted with up to 3 instances of OR', SR', CN, $NO_2$, COOR', halo, $C_{1-4}$ alkyl, or oxo.

In some embodiments, the compound is a compound of Formula I, Formula IIa, Formula IIb, Formula IIc, Formula IId, Formula IIe, Formula (1), Formula (1a), Formula (1b), Formula (2), Formula (2a), Formula (2b), Formula 3, Formula (3a), Formula (3b), Formula (4), Formula (4a), Formula (4b), Formula (5), Formula (5a), Formula (5b), Formula (6), Formula (6a), Formula (6b), Formula (7), Formula (7a), Formula (7b), Formula (8), Formula (8a), Formula (8b) , Formula (9), Formula (9a), Formula (9b), Formula (10), Formula (10a), Formula (10b) or a pharmaceutically acceptable salt or tautomer thereof.

In some embodiments, the compound is a compound of Formula I, Formula IIa, Formula IIb, Formula IIc, Formula IId, Formula IIe, Formula (1), Formula (1a), Formula (1b), Formula (2), Formula (2a), Formula (2b), Formula 3, Formula (3a), Formula (3b), Formula (4), Formula (4a), Formula (4b), Formula (5), Formula (5a), Formula (5b), Formula (6), Formula (6a), Formula (6b), Formula (7), Formula (7a), Formula (7b), Formula (8), Formula (8a), Formula (8b), Formula (9), Formula (9a), Formula (9b), Formula (10), Formula (10a), Formula (10b) or a pharmaceutically acceptable salt thereof.

In some embodiments, A is $CO_2R'$, $C(O)N(R')S(O)_2R'$, $S(O)_2R'$, or heteroaryl.

In some embodiments, A is COOH or 5-membered heteroaryl optionally substituted with OR' or SR'.

In some embodiments, Ring B is a 5-6 membered aryl or a 5-6 membered heteroaryl, each of which is optionally and independently substituted with up to 3 instances of OR', SR', CN, $NO_2$, $CO_2R'$, halo, or $C_{1-4}$ alkyl.

In some embodiments, Ring B is a 5-6 membered aryl or a 5-6 membered heteroaryl.

In some embodiments, Ring B is phenyl, which is optionally substituted with up to three instances of OH.

In some embodiments, Ring B is a 6 membered heteroaryl comprising one or two nitrogen atoms, and each nitrogen is optionally substituted with oxo.

In some embodiments, Ring B is pyridine-N-oxide.

In some embodiments, each $R^3$ is independently selected from OR', halo, or $C_{1-6}$ alkyl.

In some embodiments, each $R^3$ is independently selected from OR' or alkyl; and n is 0 or 1. In some embodiments, n is 0.

In some embodiments, n is 1.

In some embodiments, n is 0 or 1.

In some embodiments, $R^4$ is independently selected from OR' or halo and; m is 0 or 1. In some embodiments, m is 0.

In some embodiments, m is 1.

In some embodiments, m is 0 or 1.

In some embodiments, $R^5$ is H.

In some embodiments, $R^4$ and $R^5$, together with the pyrrolidine ring to which they are attached, form a $CH_2$ linker.

In some embodiments, $R^4$ is bonded to the carbon atom to which $R^5$ is bonded, to form, together with the pyrrolidine ring to which $R^4$ is attached, a $C_5$ bridged bicyclic ring.

In some embodiments, $R^4$ and $R^5$, together with the pyrrolidine ring to which they are attached, form a $C_5$ bridged bicyclic ring.

In some embodiments, $R^6$ is H, $C_{1-6}$ alkyl, halo, or OR'.

In some embodiments, $R^6$ is H, methyl, or OH.

In some embodiments, $R^7$ is OR', OC(O)R', $CO_2R'$, $CON(R')_2$, $SO_2N(R')_2$, $SO_2R'$, or $OSO_2N(R')_2$.

In some embodiments, $R^7$ is OR', $CO_2R'$, $CON(R')_2$, $SO_2N(R')_2$, or $OSO_2N(R')_2$. In some embodiments, R' is H.

In some embodiments, $R^7$ is OH, $CO_2H$, $CONH_2$, $SO_2NH_2$, or $OSO_2NH_2$. In some embodiments, $R^7$ is $CO_2H$, $CONH_2$, $SO_2NH_2$, or $OSO_2NH_2$.

In some embodiments, $R^7$ is $CONH_2$.

In some embodiments, when $R^7$ is OH then U, V and Z are CH and W is COH.

In some embodiments, provided herein is a compounds of Formula IIa, IIb, IIc, IId, and IIe:

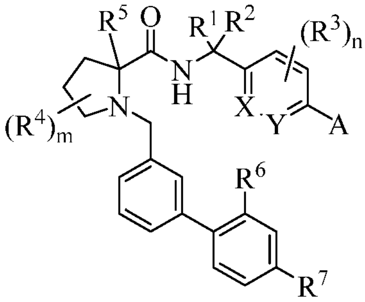
IIa,

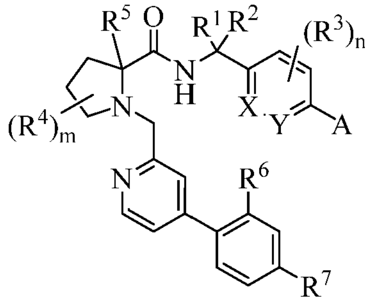
IIb,

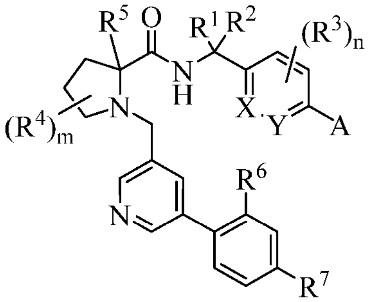
IIc,

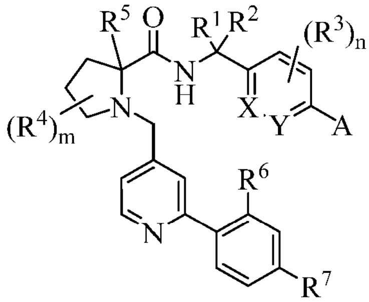
IId,

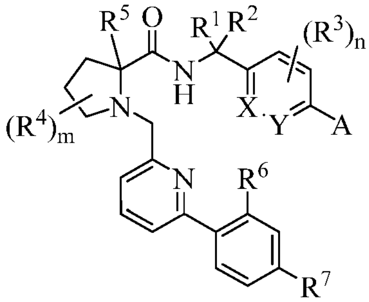
IIe,

or a pharmaceutically acceptable salt, solvate, hydrate, tautomer or optical isomer thereof, wherein A, X, Y, $R^1$, $R^2$, $R^3$, $R^4$, $R^5$, $R^6$, and $R^7$ are as defined above.

In one embodiment, provided herein is a compound of Formula (1):

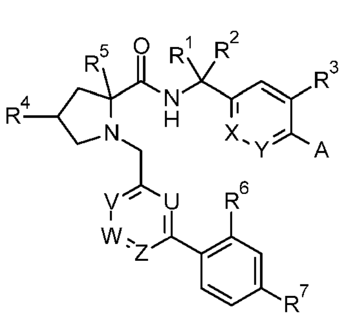
(1)

or a pharmaceutically acceptable salt, solvate, hydrate, tautomer or optical isomer thereof, wherein U, V, W, and Z are each independently selected from the group consisting of CH, COH, N or $N^+$-$O^-$ , and at least three of U, V, W, and Z are CH.

In some embodiments, $R^4$ is H, OH, F or is joined to $R^5$ to form a $CH_2$ bridge.

In some embodiments, $R^5$ is H or is joined to $R^4$ to form a $CH_2$ bridge.

In some embodiments, A is selected from the group consisting of:

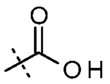, 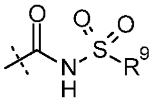, 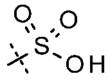, 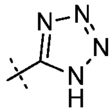, 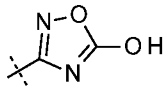, 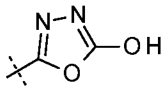,

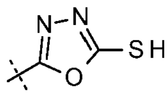, 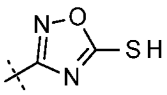, 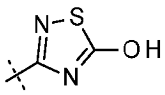, 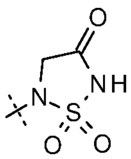, 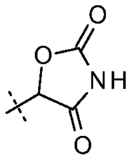;

wherein $R^9$ is $C_{1-3}$ alkyl or a $C_{3-6}$ cycloalkyl ring.

In one embodiment, provided herein is a compound of Formula (1):

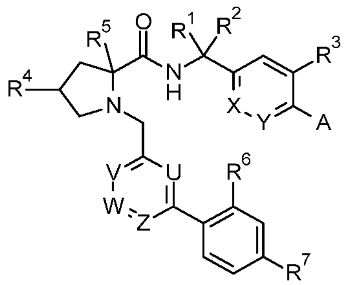

(1)

or a pharmaceutically acceptable salt, solvate, hydrate, tautomer or optical isomer thereof, wherein;

A is selected from the group consisting of:

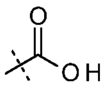, 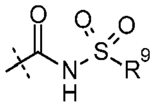, 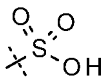, 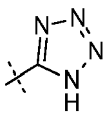, 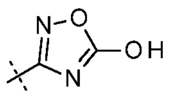, 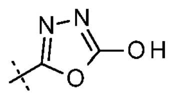,

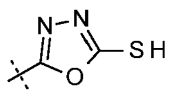, 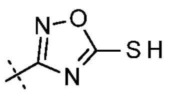, 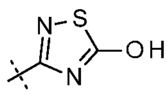, 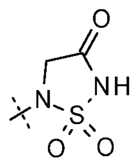, 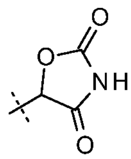;

U, V, W, and Z are each independently selected from the group consisting of CH, COH, N or $N^+$-$O^-$, and at least three of U, V, W, and Z are CH;

X and Y are each independently selected from the group consisting of CH, CF, COH or N;

$R^1$ is H, $C_{1-3}$ alkyl optionally substituted with 1-3 fluorine atoms or is joined to $R^2$ to form a $C_{3-6}$ cycloalkyl ring which is optionally substituted with 1-3 fluorine atoms;

$R^2$ is H, $C_{1-3}$ alkyl optionally substituted with 1-3 fluorine atoms or is joined to $R^1$ to form a $C_{3-6}$ cycloalkyl ring which is optionally substituted with 1-3 fluorine atoms;

$R^3$ is H, OH or F;

$R^4$ is H, OH, F or is joined to $R^5$ to form a $CH_2$ linker;

$R^5$ is H or is joined to $R^4$ to form a $CH_2$ bridge;

$R^6$ is H, OH, CN, halo, $C_{1-3}$ alkoxy optionally substituted with 1-3 fluorine atoms or $C_{13}$ alkyl optionally substituted with 1-3 fluorine atoms;

$R^7$ is OH, $CO_2H$, $CONH_2$, $SO_2NH_2$ or $OSO_2NH_2$;

$R^9$ is $C_{1-3}$ alkyl or a $C_{3-6}$ cycloalkyl ring.

In some embodiments, provided herein is compounds of Formula (1a) or (1b):

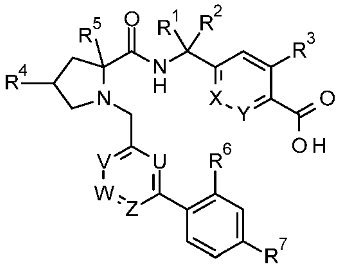
(1a)

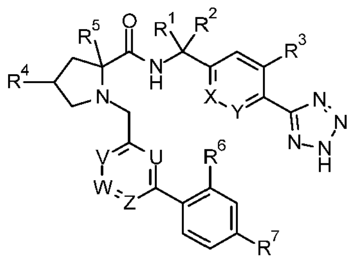
(1b)

or a pharmaceutically acceptable salt, solvate, hydrate, tautomer or optical isomer thereof, wherein U, V, W, X, Y, Z, $R^1$, $R^2$, $R^3$, $R^4$, $R^5$, $R^6$ and $R^7$ are as defined above.

In some embodiments, provided herein is compounds of Formula (2), (2a) or (2b):

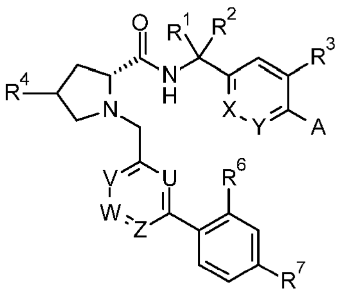
(2)

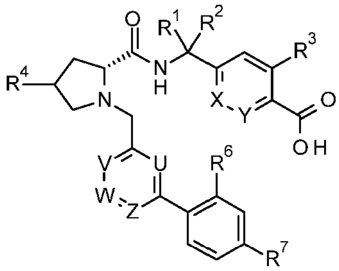
(2a)

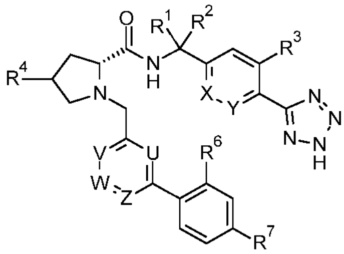
(2b)

or a pharmaceutical salt, solvate, hydrate or tautomer thereof, wherein A, U, V, W, X, Y, Z, $R^1$, $R^2$, $R^3$, $R^4$, $R^6$ and $R^7$ are as defined above.

In some embodiments, the compound is a compound of Formula (2a):

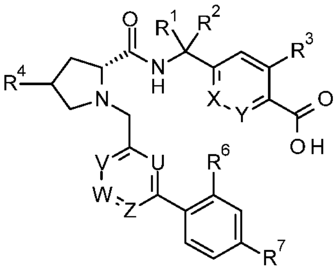
(2a),

or a pharmaceutically acceptable salt, solvate, hydrate or tautomer thereof, wherein U, V, W, X, Y, Z, $R^1$, $R^2$, $R^3$, $R^4$, $R^6$ and $R^7$ are as defined herein.

In some embodiments, the compound is a compound of Formula (2a):

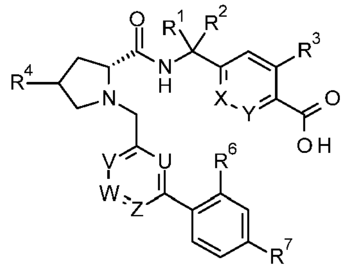

(2a),

or a pharmaceutically acceptable salt, solvate, hydrate or tautomer thereof, wherein;

U, V, W, and Z are each independently selected from the group consisting of CH, COH, N or $N^+$-$O^-$, and at least three of U, V, W, and Z are CH;

X and Y are each independently selected from the group consisting of CH, CF, COH or N;

$R^1$ is H, $C_{1-3}$ alkyl optionally substituted with 1-3 fluorine atoms or is joined to $R^2$ to form a $C_{3-6}$ cycloalkyl ring which is optionally substituted with 1-3 fluorine atoms;

$R^2$ is H, $C_{1-3}$ alkyl optionally substituted with 1-3 fluorine atoms or is joined to $R^1$ to form a $C_{3-6}$ cycloalkyl ring which is optionally substituted with 1-3 fluorine atoms;

$R^3$ is H, OH or F;

$R^4$ is H, OH, F or is joined to $R^5$ to form a $CH_2$ linker;

$R^6$ is H, OH, CN, halo, $C_{1-3}$ alkoxy optionally substituted with 1-3 fluorine atoms or $C_{13}$ alkyl optionally substituted with 1-3 fluorine atoms; and

$R^7$ is OH, $CO_2H$, $CONH_2$, $SO_2NH_2$ or $OSO_2NH_2$.

In some embodiments, $R^1$ and $R^2$ are independently H, $C_{1-3}$ alkyl optionally substituted with 1-3 fluorine atoms or $R^1$ is joined to $R^2$ to form a $C_{3-6}$ cycloalkyl ring which is optionally substituted with 1-3 fluorine atoms.

In some embodiments, $R^1$ is H or methyl or is joined to $R^2$ to form a cyclopropane-1,1-diyl ring.

In some embodiments, provided herein is compounds of Formula (3), (3a) or (3b):

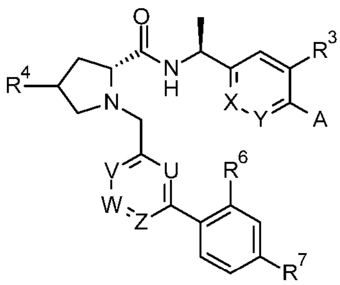

(3)

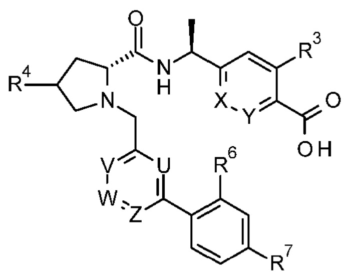

(3a)

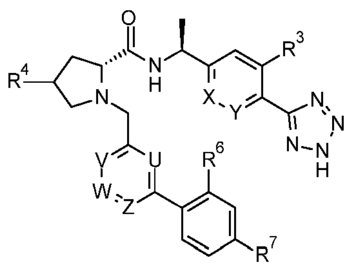

(3b)

or a pharmaceutically acceptable salt, solvate, hydrate or tautomer thereof, wherein A, U, V, W, X, Y, Z, $R^3$, $R^4$, $R^6$ and $R^7$ are as defined above.

In some embodiments, the compound is a compound of Formula (3a):

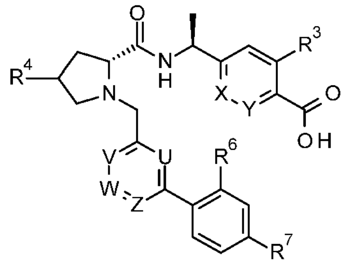

(3a),

or a pharmaceutically acceptable salt, solvate, hydrate or tautomer thereof, wherein U, V, W, X, Y, Z, $R^3$, $R^4$, $R^6$ and $R^7$ are as defined herein.

In some embodiments, the compound is a compound of Formula (3a):

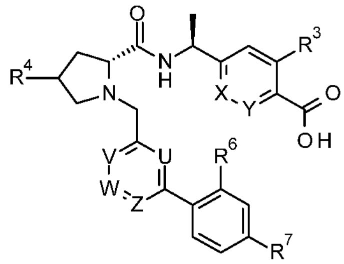

(3a),

or a pharmaceutically acceptable salt, solvate, hydrate or tautomer thereof, wherein;

U, V, W, and Z are each independently selected from the group consisting of CH, COH, N or $N^+$-$O^-$, and at least three of U, V, W, and Z are CH;

X and Y are each independently selected from the group consisting of CH, CF, COH or N;

$R^3$ is H, OH or F;

$R^4$ is H, OH, F or is joined to $R^5$ to form a $CH_2$ linker;

$R^6$ is H, OH, CN, halo, $C_{1-3}$ alkoxy optionally substituted with 1-3 fluorine atoms or $C_{1-3}$ alkyl optionally substituted with 1-3 fluorine atoms; and

$R^7$ is OH, $CO_2H$, $CONH_2$, $SO_2NH_2$ or $OSO_2NH_2$.

In some embodiments, $R^3$ is H, OH or F.

In some embodiments, $R^3$ is H or OH.

In some embodiments, $R^4$ is H, OH or F.

In some embodiments, $R^6$ is H, OH, CN, halo, $C_{1-3}$ alkoxy optionally substituted with 1-3 fluorine atoms or $C_{1-3}$ alkyl optionally substituted with 1-3 fluorine atoms.

In some embodiments, $R^6$ is H, OH, CN or methyl.

In some embodiments, $R^6$ is OH, CN or methyl.

In some embodiments, $R^6$ is methyl.

In some embodiments, $R^7$ is OH, $CO_2H$, $CONH_2$, $SO_2NH_2$ or $OSO_2NH_2$.

In some embodiments, $R^7$ is $CO_2H$, $CONH_2$, $SO_2NH_2$ or $OSO_2NH_2$.

In some embodiments, $R^7$ is $CONH_2$ or $SO_2NH_2$.

In some embodiments, X and Y are each independently selected from the group consisting of CH, CF, COH or N.

In some embodiments, provided herein is compounds of Formula (4), (4a) or (4b):

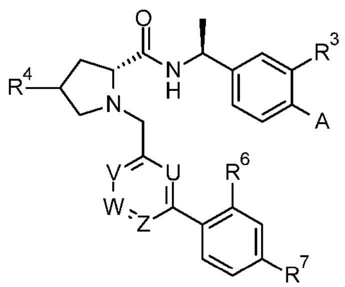

(4)

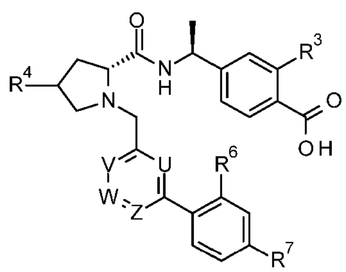

(4a)

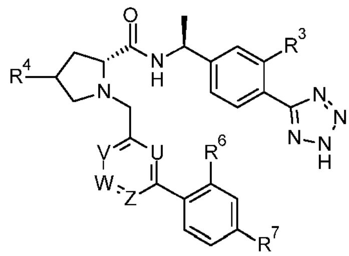

(4b)

or a pharmaceutically acceptable salt, solvate, hydrate or tautomer thereof, wherein A, U, V, W, Z, $R^3$, $R^4$, $R^6$ and $R^7$ are as defined above.

In some embodiments, provided herein is compounds of Formula (5), (5a) or (5b):

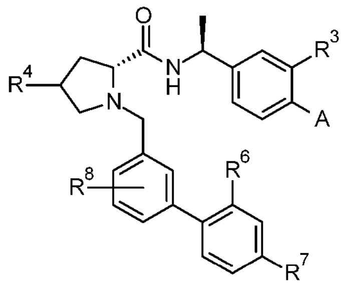

(5)

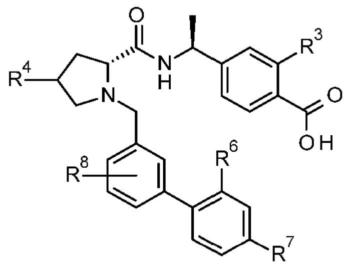

(5a)

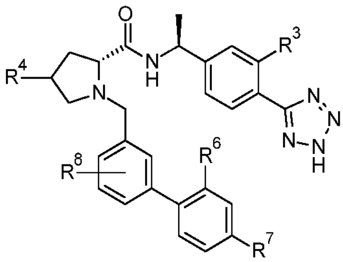

(5b)

or a pharmaceutically acceptable salt, solvate, hydrate or tautomer thereof, wherein A, $R^8$ is H or OH and $R^3$, $R^4$, $R^6$ and $R^7$ are as defined above.

In some embodiments, provided herein is compounds of Formula (6), (6a) or (6b):

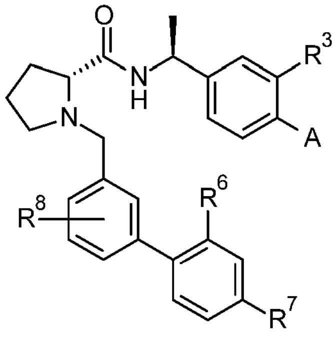

(6)

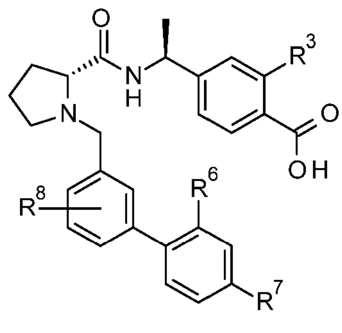

(6a)

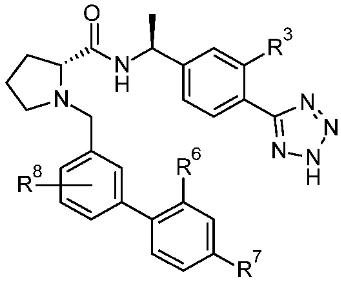

(6b)

or a pharmaceutically acceptable salt, solvate, hydrate or tautomer thereof, wherein A, $R^3$, $R^6$, $R^7$ and $R^8$ are as defined above.

In some embodiments, provided herein is compounds of Formula (7), (7a) or (7b):

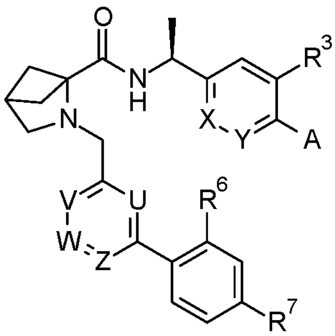

(7)

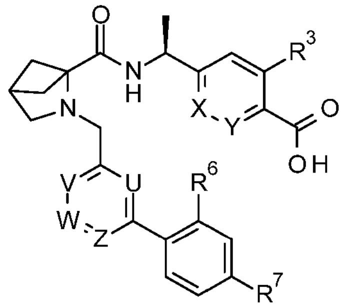

(7a)

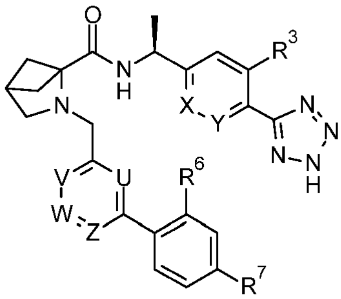

(7b)

or a pharmaceutically acceptable salt, solvate, hydrate or tautomer thereof, wherein A, U, V, W, X, Y, Z, $R^3$, $R^6$ and $R^7$ are as defined above.

In some embodiments, provided herein is compounds of Formula (8), (8a) or (8b):

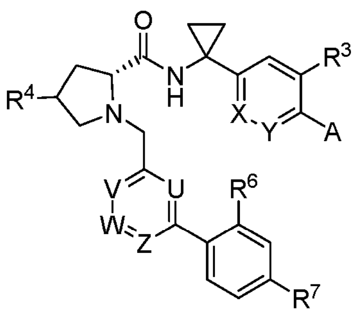

(8)

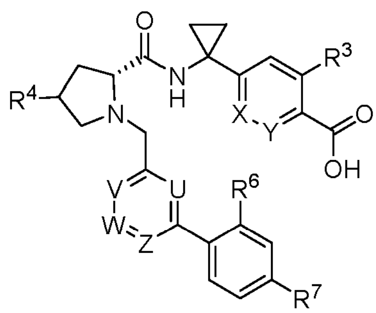

(8a)

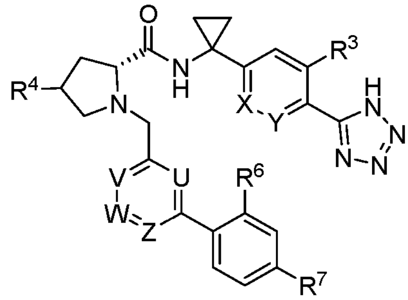

(8b)

or a pharmaceutically acceptable salt, solvate, hydrate or tautomer thereof, wherein A, U, V, W, X, Y, Z, $R^3$, $R^4$, $R^6$ and $R^7$ are as defined above.

In some embodiments, provided herein is compounds of Formula (9), (9a) or (9b):

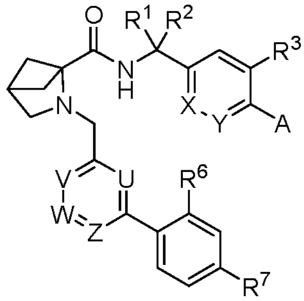

(9)

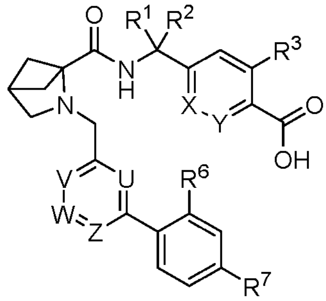

(9a)

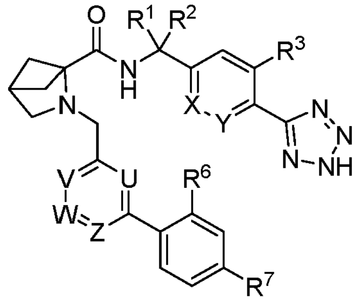

(9b)

or a pharmaceutically acceptable salt, solvate, hydrate, tautomer or optical isomer thereof, wherein A, U, V, W, X, Y, Z, $R^1$, $R^2$, $R^3$, $R^6$ and $R^7$ are as defined herein.

In some embodiments, provided herein is compounds of Formula (10), (10a) or (10b):

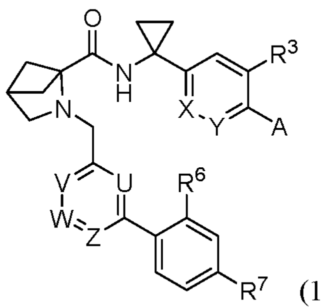

(10)

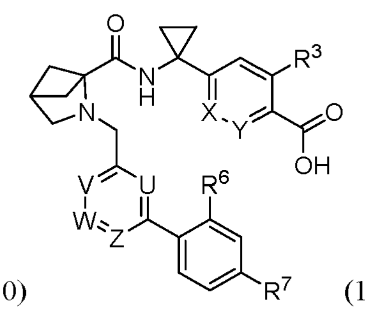

(10a)

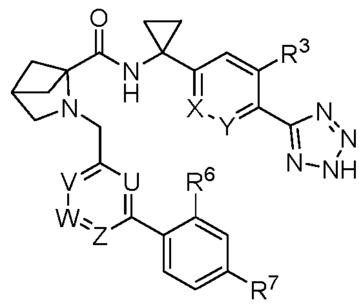

(10b)

or a pharmaceutically acceptable salt, solvate, hydrate, tautomer or optical isomer thereof, wherein A, U, V, W, X, Y, Z, $R^3$, $R^6$ and $R^7$ are as defined herein.

In some embodiments, the compound is a compound of Formula (1) or Formula (9) or a pharmaceutically acceptable salt, solvate, hydrate, tautomer or optical isomer thereof, wherein;

A is OR', C(O)R', $CO_2R'$, C(O)N(R')$_2$, C(O)N(R')S(O)$_2$R', S(O)$_2$R', S(O)$_2$OR', $SO_2N(R')_2$, $C_{1-8}$ alkyl, cycloalkyl,

heterocycloalkyl, aryl, or heteroaryl;
U, V, W, and Z are each independently selected from the group consisting of CH, COH, N or $N^+$-$O^-$ , wherein at least three of U, V, W, and Z are CH;
X and Y are each independently CR" or N, wherein at least on of X and Y is CH;
$R^1$ and $R^2$ are each independently H, $C_{1-6}$ alkyl, $C_{1-6}$ alkoxy, or $R^1$ and $R^2$, together with the carbon atom to which they are attached, form a $C_{3-6}$ cycloalkane-1,1-diyl ring;
each $R^3$ is independently selected from H, OR', COOR', C(O)R', halo, or $C_{1-6}$ alkyl;
$R^4$ is $C_{1-6}$ alkyl, halo, CN, $NO_2$, or OR';
$R^5$ is H or $C_{1-6}$ alkyl;
$R^6$ is H, $C_{1-6}$ alkyl, halo, CN, $NO_2$, OR', $CO_2R'$, or C(O)R';
$R^7$ is OR', OC(O)R', OC(O)OR', $CO_2R'$, $CON(R')_2$, $SO_2N(R')_2$, $SO_2R'$, $OSO_2R'$, or $OSO_2N(R')_2$;
each R' is independently H, $C_{1-6}$ alkyl, or $C_{3-6}$ cycloalkyl;
each R" is H, $C_{1-6}$ alkyl, halo, or OR': and
n and m are each independently 0, 1, 2, or 3;
wherein at each occurrence, alkyl, alkylene, and cycloalkyl are each optionally and independently substituted with up to 3 instances of OH, SH, CN, $NO_2$, COOH, halo, or $COOC_{1-4}$ alkyl;
wherein at each occurrence, heterocycloalkyl, aryl, and heteroaryl are each optionally and independently substituted with up to 3 instances of OR', SR', CN, $NO_2$, $CO_2R'$, halo, $C_{1-4}$ alkyl, or oxo.

In some embodiments, the compound is a compound of Formula (1) or a pharmaceutically acceptable salt, solvate, hydrate, tautomer or optical isomer thereof, wherein; A is COOH; U is CH; V is CH; W is CH, COH, N or $N^+$-$O^-$; Z is CH or COH; X is CH; Y is CH or COH; $R^1$ is H or methyl and $R^2$ is H or $R^1$ is joined to $R^2$ to form a cyclopropane-1,1-diyl ring; $R^3$ is H or OH; $R^4$ is H or OH and $R^5$ is H or $R^4$ is joined to $R^5$ to form a CH2 bridge; $R^6$ is H, OH, CN or methyl; and $R^7$ is OH, $CO_2H$, $CONH_2$, $SO_2NH_2$ or $OSO_2NH_2$.

In some embodiments, the compound is a compound of Formula (1) or Formula (9) or a pharmaceutically acceptable salt, solvate, hydrate, tautomer or optical isomer thereof, wherein; A is COOH; U is CH; V is CH; W is CH, COH, N or $N^+$-$O^-$; Z is CH or COH; X is CH; Y is CH or COH; $R^1$ is H or methyl and $R^2$ is H or $R^1$ is joined to $R^2$ to form a cyclopropane-1,1-diyl ring; $R^3$ is H or OH; $R^4$ is H or OH; $R^5$ is H; $R^6$ is H, OH, CN or methyl; and $R^7$ is OH, $CO_2H$, $CONH_2$, $SO_2NH_2$ or $OSO_2NH_2$.

In some embodiments, A is selected from $CO_2H$, tetrazole, 1,2,4-oxadiazol-5(2H)-one, 1,3,4-oxadiazol-2(3H)-one, $CONHSO_2R^6$, $CONHSO_2Me$, $SO_3H$, 1,3,4-oxadiazole-2(3H)-thione, 1,2,4-oxadiazole-5(2H)-thione, 1,2,4-thiadiazol-5(2H)-one, 1,2,5-thiadiazolidin-3-one 1,1-dioxide and 2,4-oxazolidinedione.

In some embodiments, A is selected from:

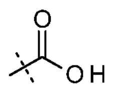
and
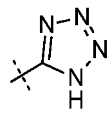
.

In some embodiments, A is selected from $CO_2H$ and a tetrazole ring. A can be $CO_2H$. A can be a tetrazole ring.

In some embodiments, U can be CH. U can be COH. U can be N. U can be $N^+$-$O^-$.

In some embodiments, U is CH.

In some embodiments, V can be CH. V can be COH. V can be N. V can be $N^+$-$O^-$.

In some embodiments, V is CH.

In some embodiments, W can be CH. W can be COH. W can be N. W can be $N^+$-$O^-$.

In some embodiments, W is CH, COH, N or $N^+$-$O^-$.

In some embodiments, W is CH or COH.

In some embodiments, Z can be CH. Z can be COH. Z can be N. Z can be $N^+$-$O^-$.

In some embodiments, Z is CH or COH.

In some embodiments, at least three of U, V, W and Z are CH. U, V and W can be CH. U, V and Z can be CH. U, W and Z can be CH. V, W and Z can be CH. U, V, W and Z can be CH. U, V and Z can be CH and W can be COH.

In some embodiments, X can be CH. X can be CF. X can be COH. X can be N.

In some embodiments, Y can be CH. Y can be CF. Y can be COH. Y can be N.

In some embodiments, Y can be CH or COH.

In the compounds herein, at least one of X and Y is CH. X and Y can both be CH.

In some embodiments, X can be CH and Y can be CH or COH.

In some embodiments, $R^1$ can be H or $C_{1-3}$ alkyl optionally substituted with 1-3 fluorine atoms. $R^1$ can be H. $R^1$ can be $C_{1-3}$ alkyl optionally substituted with 1-3 fluorine atoms. $R^1$ can be $C_{1-3}$ alkyl. $R^1$ can be joined to $R^2$ to form a $C_{3-6}$

cycloalkyl ring which is optionally substituted with 1-3 fluorine atoms. $R^1$ can be joined to $R^2$ to form a $C_{3-6}$ cycloalkyl ring. $R^1$ can be H or methyl or can be joined to $R^2$ to form a cyclopropane ring. $R^1$ can be methyl optionally substituted with 1-3 fluorine atoms. $R^1$ can be methyl. $R^1$ can be joined to $R^2$ to form a cyclopropane ring which is optionally substituted with 1-3 fluorine atoms. $R^1$ can be joined to $R^2$ to form a cyclopropane ring.

In some embodiments, $R^2$ can be H. $R^2$ can be $C_{1-3}$ alkyl optionally substituted with 1-3 fluorine atoms. $R^2$ can be $C_{1-3}$ alkyl. $R^2$ can be joined to $R^1$ to form a $C_{3-6}$ cycloalkyl ring which is optionally substituted with 1-3 fluorine atoms. $R^2$ can be joined to $R^1$ to form a $C_{3-6}$ cycloalkyl ring. $R^2$ can be joined to $R^1$ to form a cyclopropane ring which is optionally substituted with 1-3 fluorine atoms. $R^2$ can be joined to $R^1$ to form a cyclopropane ring.

In some embodiments, $R^3$ can be H. $R^3$ can be OH. $R^3$ can be F.

In some embodiments, $R^4$ can be H. $R^4$ can be OH. $R^4$ can be F. $R^4$ can be joined to $R^5$ to form a $CH_2$ bridge.

In some embodiments,$R^5$ can be H. $R^5$ can be joined to $R^4$ to form a $CH_2$ bridge.

In some embodiments, $R^6$ and $R^7$ together with the ring to which they are attached can be the group consisting of:

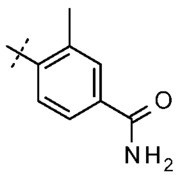
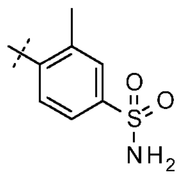
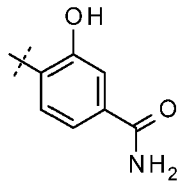
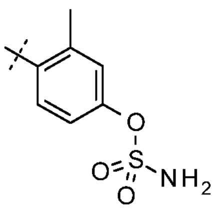
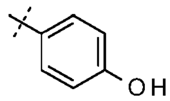
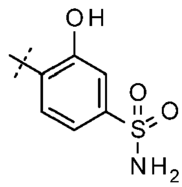
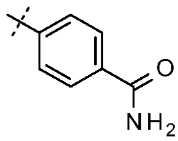
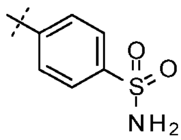
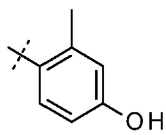
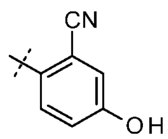
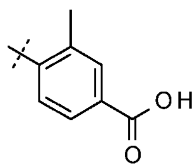

In some embodiments, $R^6$ can be H, OH, CN or methyl. $R^6$ can be H. $R^6$ can be OH. $R^6$ can be CN. $R^6$ can be halo. $R^6$ can be F. $R^6$ can be Cl. $R^6$ can be Br. $R^6$ can be $C_{1-3}$ alkoxy optionally substituted with 1-3 fluorine atoms. $R^6$ can be $C_{1-3}$ alkoxy. $R^6$ can be methoxy optionally substituted with 1-3 fluorine atoms. $R^6$ can be methoxy. $R^6$ can be $C_{1-3}$ alkyl optionally substituted with 1-3 fluorine atoms. $R^6$ can be $C_{1-3}$ alkyl. $R^6$ can be H or methyl. $R^6$ can be methyl optionally substituted with 1-3 fluorine atoms. $R^6$ can be methyl.

In some embodiments, $R^7$ can be OH. $R^7$ can be $CO_2H$. $R^7$ can be $CONH_2$. $R^7$ can be $SO_2NH_2$. $R^7$ can be $OSO_2NH_2$.

In some embodiments, $R^8$ can be H. $R^8$ can be OH.

In some embodiments, $R^9$ can be $C_{1-3}$ alkyl. $R^9$ can be a $C_{3-6}$ cycloalkyl ring. $R^9$ can be methyl.

In some embodiments, A can be $CO_2H$; U, V and Z can be CH; W can be COH; $R^1$ can be methyl; $R^2$ can be H; and $R^6$ can be methyl.

In some embodiments, the compound or pharmaceutically acceptable salt disclosed herein possesses EP4 receptor agonist activity.

In some embodiments, the invention includes a pharmaceutical composition comprising a compound, pharmaceutically acceptable salt, solvate, hydrate, tautomer or optical isomer described herein and a pharmaceutically acceptable excipient.

In some embodiments, the pharmaceutical composition further comprises at least one additional therapeutic

agent selected from the group consisting of aminosalicylates, corticosteroids, immunomodulators and combinations thereof.

In some embodiments, disclosed herein is a compound or pharmaceutically acceptable salt described herein for use in a method of treating an EP4 receptor mediated disease, the method comprising administering to a patient in need thereof a compound or pharmaceutically acceptable salt disclosed herein.

In some embodiments, the EP4 receptor mediated disease is a gastrointestinal disorder.

In some embodiments, the gastrointestinal disorder is selected from the group consisting of constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease and ischemic colitis, or pulmonary diseases and conditions such as chronic obstructive pulmonary diseases, asthma, chronic bronchitis, cystic fibrosis, emphysema, chronic idiopathic cough, hyperactive airway disorder, and idiopathic pulmonary fibrosis.

In some embodiments, the compound of Formula I or Formula (1) is the compounds listed in Table 1 or a pharmaceutically acceptable salt, solvate, hydrate or tautomer thereof.

In some embodiments, the compound of Formula I or Formula (1) is the compounds listed in Table 1, or a pharmaceutically acceptable salt thereof:

Table 1: Exemplary compounds of Formula (I)

| | | |
|---|---|---|
| 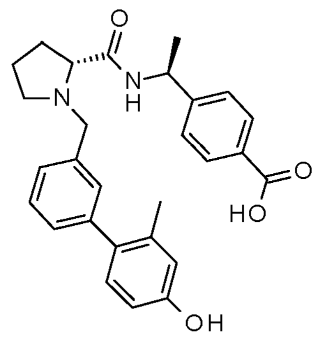<br>**1** | 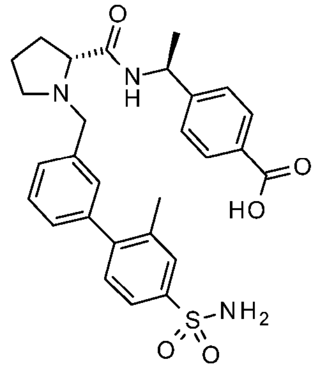<br>**2** | 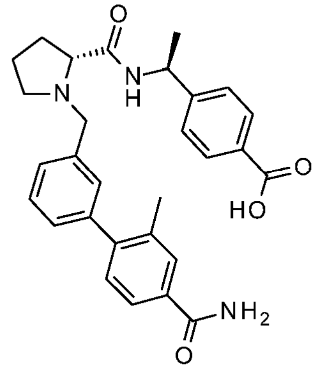<br>**3** |
| 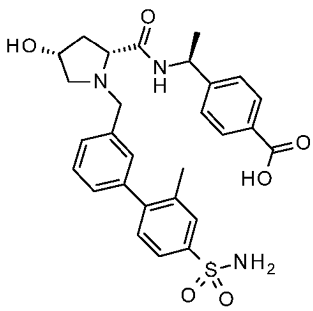<br>**4** | 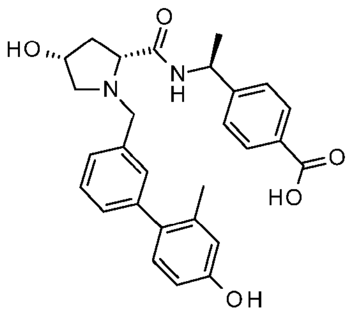<br>**5** | 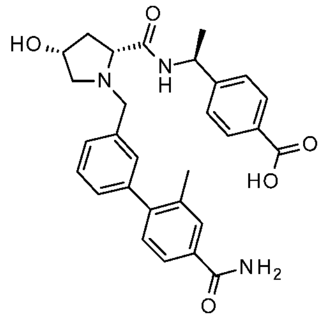<br>**6** |
| 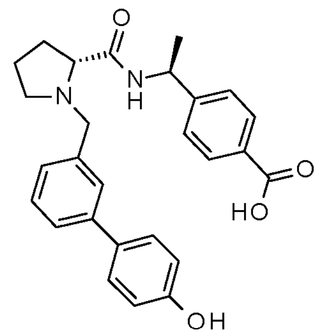<br>**7** | 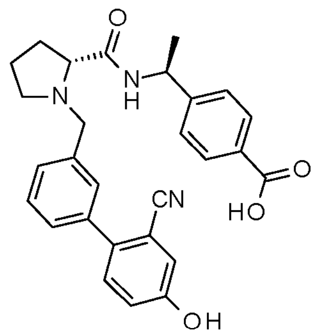<br>**8** | 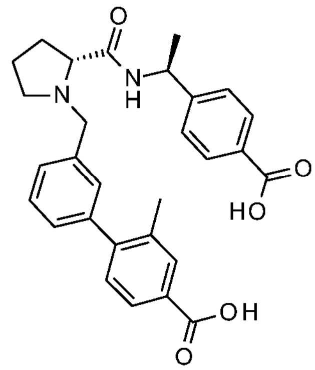<br>**9** |

| | | |
|---|---|---|
| 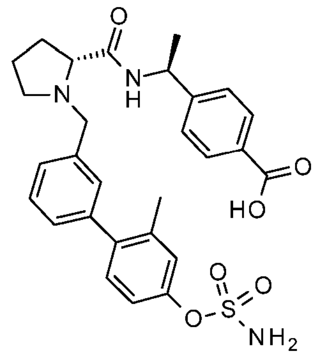<br>**10** | 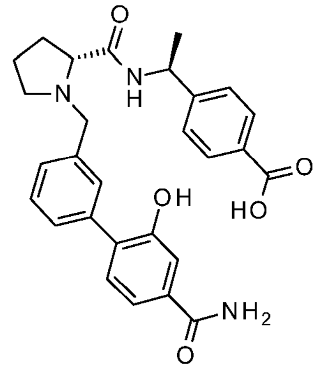<br>**11** | 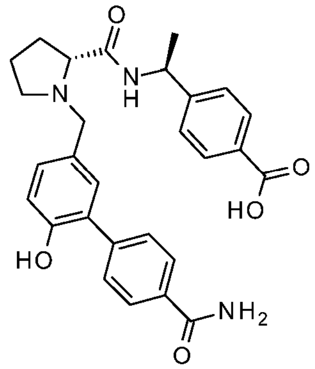<br>**12** |
| 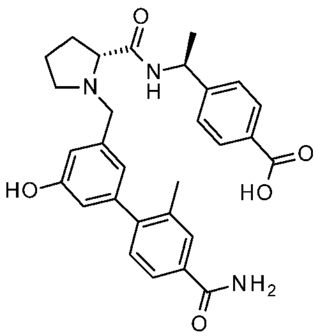<br>**13** | 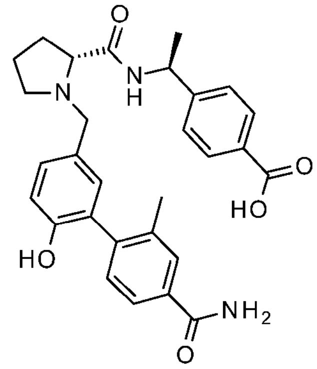<br>**14** | 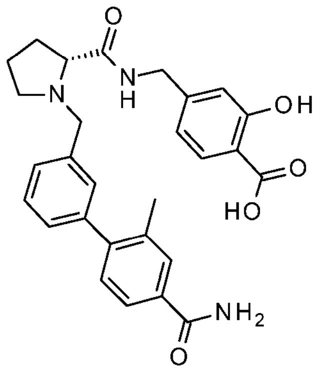<br>**15** |
| 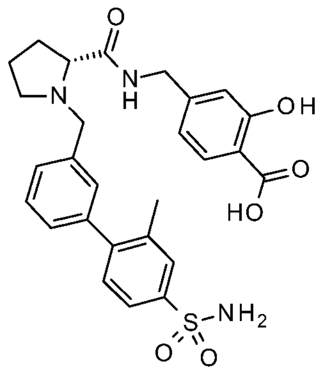<br>**16** | 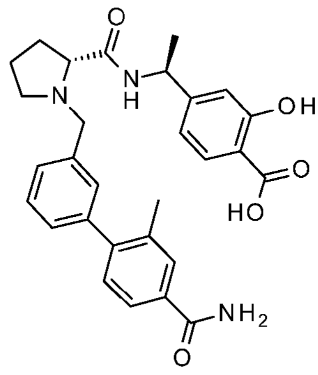<br>**17** | 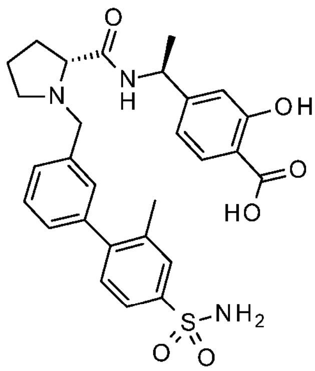<br>**18** |

| | | |
|---|---|---|
| 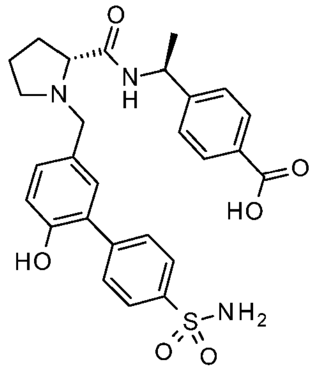 **19** | 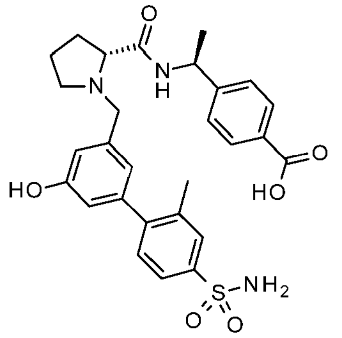 **20** | 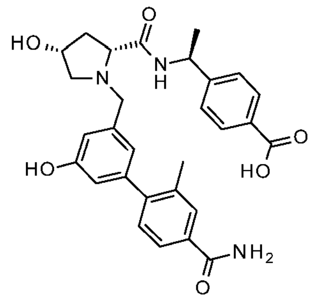 **21** |
| 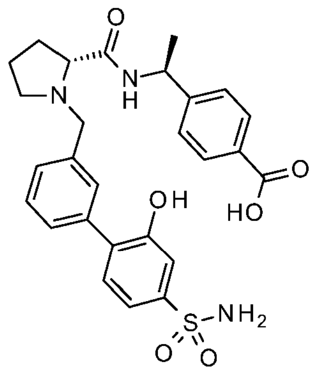 **22** | 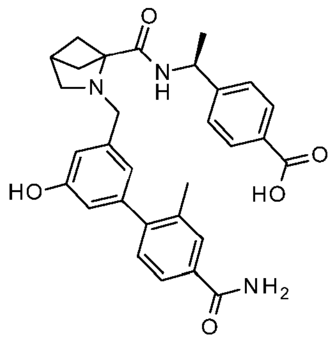 **23** | 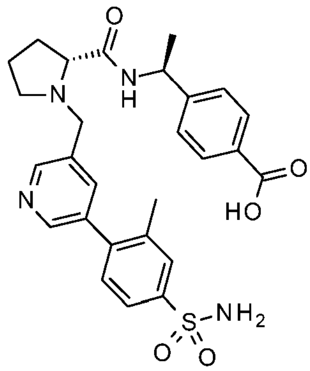 **24** |
| 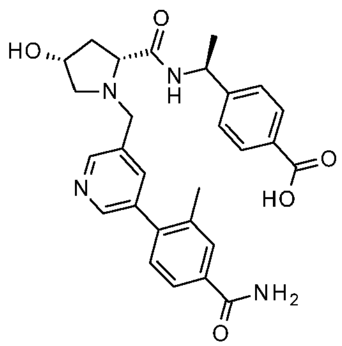 **25** | 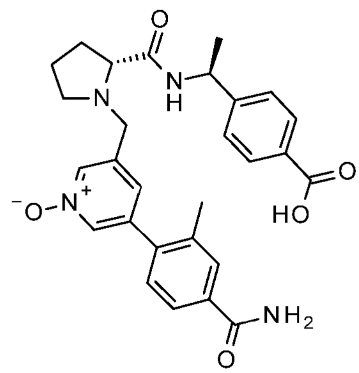 **26** | 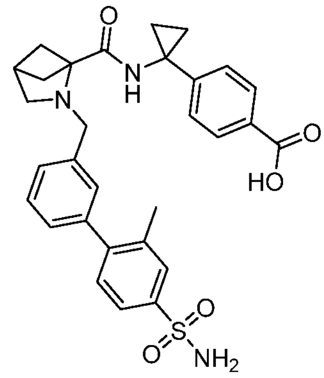 **27** |

| | | |
|---|---|---|
| 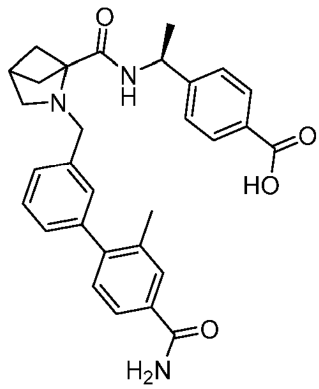<br>**28** | 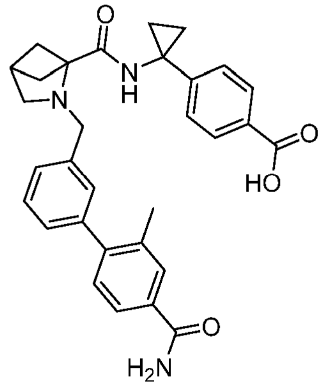<br>**29** | 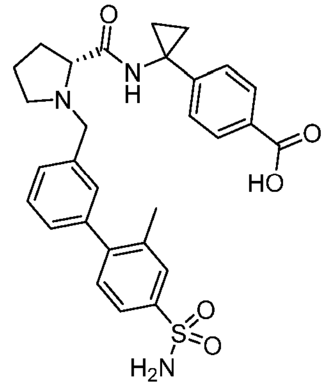<br>**30** |
| 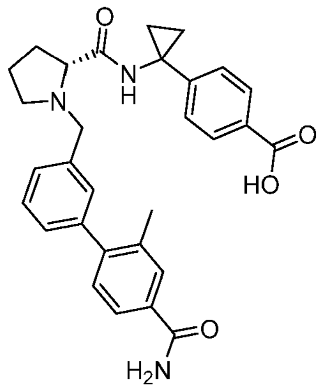<br>**31** | 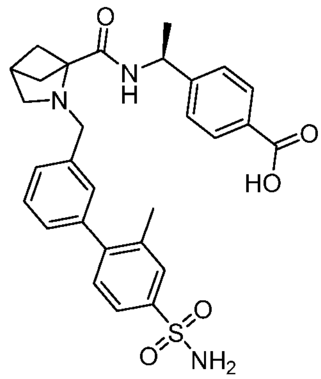<br>**32** | 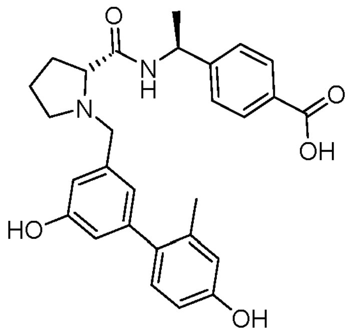<br>**33** |
| 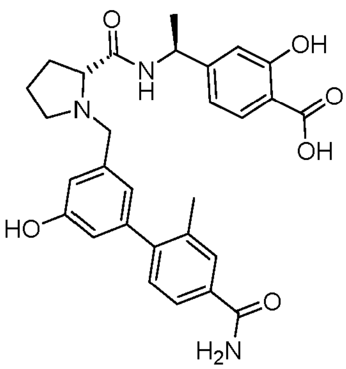<br>**34** | 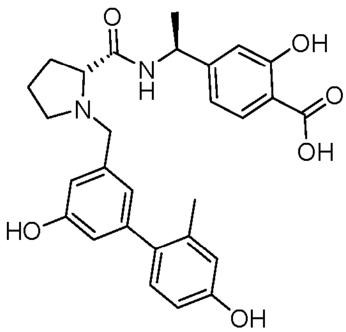<br>**35** | |

ABBREVIATIONS

| | |
|---|---|
| aq | aqueous |
| Bn | benzyl |
| DCM | dichloromethane |
| DMA | dimethylacetamide |
| DMF | dimethylformamide |
| dppf | 1,1'-bis(diphenylphosphino)ferrocene |
| EDCI | 1-ethyl-3-(3-dimethylaminopropyl)carbodiimide |

| | |
|---|---|
| FA | formic acid |
| EtOAc | ethyl acetate |
| HATU | 1-[Bis(dimethylamino)methylene]-1H-1,2,3-triazolo[4,5-b]pyridinium 3-oxide hexafluorophosphate, |
| HOBt | hydroxybenzotriazole |
| HPLC | high performance liquid chromatography |
| hr | hour |
| hrs | hours |
| LC/MS | liquid chromatography mass spectrometry |
| M | molar |
| MeCN | acetonitrile |
| MeOH | methanol |
| N | normal |
| prep HPLC | preparative high-performance liquid chromatography |
| RT | room temperature |
| sat | saturated |
| THF | tetrahydrofuran |
| UPLC | ultra performance liquid chromatography |

DEFINITIONS

In this application, the following definitions apply, unless indicated otherwise.

The term "treatment", in relation to the uses of any of the compounds described herein, including those of Formula I, IIa, IIb, IIc, IId, IIe, (1), (1a), (1b), (2), (2a), (2b), (3), (3a), (3b), (4), (4a), (4b), (5), (5a), (5b), (6), (6a), (6b), (7), (7a), (7b), (8), (8a), (8b), (9), (9a), (9b), (10), (10a) and (10b) is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" (for example in relation to the compound for use in methods of treatment of a disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

As used herein, the term "hydroxyl" or "hydroxy" refers to an -OH moiety.

As used herein, an "alkyl" group refers to a saturated aliphatic hydrocarbon group containing 1-12 (e.g., 1-8, 1-6, or 1-4) carbon atoms. An alkyl group can be straight or branched. Examples of alkyl groups include, but are not limited to, methyl, ethyl, propyl, isopropyl, butyl, isobutyl, sec-butyl, tert-butyl, n-pentyl, n-heptyl, or 2-ethylhexyl. An alkyl group can be substituted (i.e., optionally substituted) with one or more substituents such as halo, phospho, cycloaliphatic [e.g., cycloalkyl or cycloalkenyl], heterocycloaliphatic [e.g., heterocycloalkyl or heterocycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocycloalkyl)carbonylamino, (heterocycloalkylalkyl)carbonylamino, heteroarylcarbonylamino, heteroaralkylcarbonylamino alkylaminocarbonyl, cycloalkylaminocarbonyl, heterocycloalkylaminocarbonyl, arylaminocarbonyl, or heteroarylaminocarbonyl], amino [e.g., aliphaticamino, cycloaliphaticamino, or heterocycloaliphaticamino], sulfonyl [e.g., aliphatic-$SO_2$-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroarylalkoxy, alkoxycarbonyl, alkylcarbonyloxy, or hydroxy. Without limitation, some examples of substituted alkyls include carboxyalkyl (such as HOOC-alkyl, alkoxycarbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (such as (alkyl-$SO_2$-amino)alkyl), aminoalkyl, amidoalkyl, (cycloaliphatic)alkyl, or haloalkyl.

As used herein, an "alkylene" group refers to a bivalent branched or straight alkyl group that contains 2-12 (e.g. 2-8, 2-6, or 2-4) carbon atoms, and serves to connect two chemical moieties. Examples of alkylene groups include, but are not limited to methylene, ethylene, propylene, butylene, isopropylene (methylethylene), and isobutylene (2-methylpropylene). An alkylene group can be substituted (i.e., optionally substituted) with one or more substituents as defined in the alkyl group.

As used herein, an "amido" encompasses both "aminocarbonyl" and "carbonylamino." These terms when used alone or in connection with another group refer to an amido group such as -N($R^X$)-C(O)-$R^Y$ or -C(O)-N($R^X$)$_2$, when used terminally, and -C(O)-N($R^X$)- or - N($R^X$)-C(O)- when used internally, wherein $R^X$ and $R^Y$ can be aliphatic, cycloaliphatic, aryl, araliphatic, heterocycloaliphatic, heteroaryl or heteroaraliphatic. Examples of amido groups include alkylamido (such as alkylcarbonylamino or alkylaminocarbonyl), (heterocycloaliphatic)amido, (heteroaralkyl)amido, (heteroaryl)amido,

(heterocycloalkyl)alkylamido, arylamido, aralkylamido, (cycloalkyl)alkylamido, or cycloalkylamido.

As used herein, an "amino" group refers to -$NR^XR^Y$ wherein each of $R^X$ and $R^Y$ is independently hydrogen, aliphatic, cycloaliphatic, (cycloaliphatic)aliphatic, aryl, araliphatic, heterocycloaliphatic, (heterocycloaliphatic)aliphatic, heteroaryl, carboxy, sulfanyl, sulfinyl, sulfonyl, (aliphatic)carbonyl, (cycloaliphatic)carbonyl, ((cycloaliphatic)aliphatic) carbonyl, arylcarbonyl, (araliphatic)carbonyl, (heterocycloaliphatic)carbonyl, ((heterocycloaliphatic)aliphatic)carbonyl, (heteroaryl)carbonyl, or (heteroaraliphatic)carbonyl, each of which being defined herein and being optionally substituted. Examples of amino groups include alkylamino, dialkylamino, or arylamino. When the term "amino" is not the terminal group (e.g., alkylcarbonylamino), it is represented by -$NR^X$-, where $R^X$ has the same meaning as defined above.

As used herein, an "aryl" group used alone or as part of a larger moiety as in "aralkyl," "aralkoxy," or "aryloxyalkyl" refers to monocyclic (e.g., phenyl); bicyclic (e.g., indenyl, naphthalenyl, tetrahydronaphthyl, tetrahydroindenyl); and tricyclic (e.g., fluorenyl tetrahydrofluorenyl, or tetrahydroanthracenyl, anthracenyl) ring systems in which the monocyclic ring system is aromatic or at least one of the rings in a bicyclic or tricyclic ring system is aromatic. The bicyclic and tricyclic groups include benzofused 2-3 membered carbocyclic rings. For example, a benzofused group includes phenyl fused with two or more $C_{4-8}$ carbocyclic moieties. An aryl is optionally substituted with one or more substituents including aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic ring of a benzofused bicyclic or tricyclic aryl); nitro; carboxy; amido; acyl [e.g., (aliphatic)carbonyl; (cycloaliphatic)carbonyl; ((cycloaliphatic)aliphatic) carbonyl; (araliphatic)carbonyl; (heterocycloaliphatic)carbonyl; ((heterocycloaliphatic)aliphatic)carbonyl; or (heteroaraliphatic)carbonyl]; sulfonyl [e.g., aliphatic-$SO_2$- or amino-$SO_2$-]; sulfinyl [e.g., aliphatic-S(O)- or cycloaliphatic-S(O)-]; sulfanyl [e.g., aliphatic-S-]; cyano; halo; hydroxy; mercapto; sulfoxy; urea; thiourea; sulfamoyl; sulfamide; or carbamoyl. Alternatively, an aryl can be unsubstituted.

Non-limiting examples of substituted aryls include haloaryl [e.g., mono-, di (such as *p,m*-dihaloaryl), and (trihalo) aryl]; (carboxy)aryl [e.g., (alkoxycarbonyl)aryl, ((aralkyl)carbonyloxy)aryl, and (alkoxycarbonyl)aryl]; (amido)aryl [e.g., (aminocarbonyl)aryl, (((alkylamino)alkyl)aminocarbonyl)aryl, (alkylcarbonyl)aminoaryl, (arylaminocarbonyl)aryl, and (((heteroaryl)amino)carbonyl)aryl]; aminoaryl [e.g., ((alkylsulfonyl)amino)aryl or ((dialkyl)amino)aryl]; (cyanoalkyl)aryl; (alkoxy)aryl; (sulfamoyl)aryl [e.g., (aminosulfonyl)aryl]; (alkylsulfonyl)aryl; (cyano)aryl; (hydroxyalkyl)aryl; ((alkoxy)alkyl) aryl; (hydroxy)aryl, ((carboxy)alkyl)aryl; (((dialkyl)amino)alkyl)aryl; (nitroalkyl)aryl; (((alkylsulfonyl)amino)alkyl)aryl; ((heterocycloaliphatic)carbonyl)aryl; ((alkylsulfonyl)alkyl)aryl; (cyanoalkyl)aryl; (hydroxyalkyl)aryl; (alkylcarbonyl)aryl; alkylaryl; (trihaloalkyl)aryl; *p*-amino-*m*-alkoxycarbonylaryl; *p*-amino-*m*-cyanoaryl; *p*-halo-*m*-aminoaryl; or (*m*-(heterocycloaliphatic)-*o*-(alkyl))aryl.

As used herein, a "cycloalkyl" group refers to a saturated carbocyclic mono- or bicyclic (fused or bridged) ring of 3-10 (e.g., 5-10) carbon atoms. Examples of cycloalkyl groups include cyclopropyl, cyclobutyl, cyclopentyl, cyclohexyl, cycloheptyl, adamantyl, norbornyl, cubyl, octahydro-indenyl, decahydro-naphthyl, bicyclo[3.2.1]octyl, bicyclo[2.2.2]octyl, bicyclo[3.3.1]nonyl, bicyclo[3.3.2.]decyl, bicyclo[2.2.2]octyl, adamantyl, or ((aminocarbonyl)cycloalkyl)cycloalkyl.

A cycloalkyl group can be optionally substituted with one or more substituents such as phospho, aliphatic [e.g., alkyl, alkenyl, or alkynyl], cycloaliphatic, (cycloaliphatic) aliphatic, heterocycloaliphatic, (heterocycloaliphatic) aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic)aliphatic)carbonylamino, (aryl)carbonylamino, (araliphatic)carbonylamino, (heterocycloaliphatic)carbonylamino, ((heterocycloaliphatic)aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], cyano, halo, hydroxy, mercapto, sulfonyl [e.g., alkyl-$SO_2$- and aryl-$SO_2$-], sulfinyl [e.g., alkyl-S(O)-], sulfanyl [e.g., alkyl-S-], sulfoxy, urea, thiourea, sulfamoyl, sulfamide, oxo, or carbamoyl.

As used herein, a "heterocycloalkyl" group refers to a 3-10 membered mono- or bicylic (fused or bridged) (e.g., 5- to 10-membered mono- or bicyclic) saturated ring structure, in which one or more of the ring atoms is a heteroatom (e.g., N, O, S, or combinations thereof). Examples of a heterocycloalkyl group include piperidyl, piperazyl, tetrahydropyranyl, tetrahydrofuryl, 1,4-dioxolanyl, 1,4-dithianyl, 1,3-dioxolanyl, oxazolidyl, isoxazolidyl, morpholinyl, thiomorpholyl, octahydrobenzofuryl, octahydrochromenyl, octahydrothiochromenyl, octahydroindolyl, octahydropyrindinyl, decahydroquinolinyl, octahydrobenzo[b]thiopheneyl, 2-oxa-bicyclo[2.2.2]octyl, 1-aza-bicyclo[2.2.2]octyl, 3-aza-bicyclo[3.2.1]octyl, and 2,6-dioxa-tricyclo[$3.3.1.0^{3,7}$]nonyl. A monocyclic heterocycloalkyl group can be fused with a phenyl moiety to form structures, such as tetrahydroisoquinoline, that would be categorized as heteroaryls.

A heterocycloalkyl group can be optionally substituted with one or more substituents such as phospho, aliphatic [e.g., alkyl, alkenyl, or alkynyl], cycloaliphatic, (cycloaliphatic)aliphatic, heterocycloaliphatic, (heterocycloaliphatic)aliphatic, aryl, heteroaryl, alkoxy, (cycloaliphatic)oxy, (heterocycloaliphatic)oxy, aryloxy, heteroaryloxy, (araliphatic)oxy, (heteroaraliphatic)oxy, aroyl, heteroaroyl, amino, amido [e.g., (aliphatic)carbonylamino, (cycloaliphatic)carbonylamino, ((cycloaliphatic) aliphatic)carbonylamino, (aryl)carbonylamino, (araliphatic)carbonylamino, (heterocycloaliphatic)carbonylamino, ((heterocycloaliphatic) aliphatic)carbonylamino, (heteroaryl)carbonylamino, or (heteroaraliphatic)carbonylamino], nitro, carboxy [e.g., HOOC-, alkoxycarbonyl, or alkylcarbonyloxy], acyl [e.g., (cycloaliphatic)carbonyl, ((cycloaliphatic) aliphatic)carbonyl, (araliphatic)carbonyl, (heterocycloaliphatic)carbonyl, ((heterocycloaliphatic)aliphatic)carbonyl, or (heteroaraliphatic)carbonyl], nitro, cyano, halo, hydroxy, mercapto, sulfonyl [e.g., alkylsulfonyl or arylsulfonyl], sulfinyl [e.g., alkylsulfinyl], sulfanyl [e.g., alkylsulfanyl], sulfoxy, urea, thiourea, sulfamoyl, sulfamide, oxo, or carbamoyl.

A "heteroaryl" group, as used herein, refers to a monocyclic, bicyclic, or tricyclic ring system having 4 to 15 ring atoms wherein one or more of the ring atoms is a heteroatom (e.g., N, O, S, or combinations thereof) and in which the monocyclic ring system is aromatic or at least one of the rings in the bicyclic or tricyclic ring systems is aromatic.

A heteroaryl group includes a benzofused ring system having 2 to 3 rings. For example, a benzofused group includes benzo fused with one or two 4 to 8 membered heterocycloaliphatic moieties (e.g., indolizyl, indolyl, isoindolyl, 3H-indolyl, indolinyl, benzo[b]furyl, benzo[b]thiophene-yl, quinolinyl, or isoquinolinyl). Some examples of heteroaryl are azetidinyl, pyridyl, 1H-indazolyl, furyl, pyrrolyl, thienyl, thiazolyl, oxazolyl, imidazolyl, tetrazolyl, benzofuryl, isoquinolinyl, benzthiazolyl, xanthene, thioxanthene, phenothiazine, dihydroindole, benzo[1,3]dioxole, benzo[b]furyl, benzo[b]thiophenyl, indazolyl, benzimidazolyl, benzthiazolyl, puryl, cinnolyl, quinolyl, quinazolyl, cinnolyl, phthalazyl, quinazolyl, quinoxalyl, isoquinolyl, 4H-quinolizyl, benzo-1,2,5-thiadiazolyl, or 1,8-naphthyridyl.

A heteroaryl group includes N-oxide heteroaryl compounds, such as pyridine, pyrimidine, pyrazine, pyrrole, imidazole, thizaole, quinoline, or isoquinoline, which is oxidized at the nitrogen atom. Example of an N-oxide heteroaryl is pyridine-N-oxide with the formula $C_5H_5N^{-}\rightarrow O^{+}$.

Without limitation, monocyclic heteroaryls include furyl, thiophene-yl, 2H-pyrrolyl, pyrrolyl, oxazolyl, thazolyl, imidazolyl, pyrazolyl, isoxazolyl, isothiazolyl, 1,3,4-thiadiazolyl, 2H-pyranyl, 4-H-pranyl, pyridyl, pyridazyl, pyrimidyl, pyrazolyl, pyrazyl, or 1,3,5-triazyl. Monocyclic heteroaryls are numbered according to standard chemical nomenclature.

Without limitation, bicyclic heteroaryls include indolizyl, indolyl, isoindolyl, 3H-indolyl, indolinyl, benzo[b]furyl, benzo[b]thiophenyl, quinolinyl, isoquinolinyl, indolizyl, isoindolyl, indolyl, benzo[b]furyl, bexo[b]thiophenyl, indazolyl, benzimidazyl, benzthiazolyl, purinyl, 4H-quinolizyl, quinolyl, isoquinolyl, cinnolyl, phthalazyl, quinazolyl, quinoxalyl, 1,8-naphthyridyl, or pteridyl. Bicyclic heteroaryls are numbered according to standard chemical nomenclature.

A heteroaryl is optionally substituted with one or more substituents such as aliphatic [e.g., alkyl, alkenyl, or alkynyl]; cycloaliphatic; (cycloaliphatic)aliphatic; heterocycloaliphatic; (heterocycloaliphatic)aliphatic; aryl; heteroaryl; alkoxy; (cycloaliphatic)oxy; (heterocycloaliphatic)oxy; aryloxy; heteroaryloxy; (araliphatic)oxy; (heteroaraliphatic)oxy; aroyl; heteroaroyl; amino; oxo (on a non-aromatic carbocyclic or heterocyclic ring of a bicyclic or tricyclic heteroaryl); carboxy; amido; acyl [ e.g., aliphaticcarbonyl; (cycloaliphatic)carbonyl; ((cycloaliphatic)aliphatic)carbonyl; (araliphatic) carbonyl; (heterocycloaliphatic)carbonyl; ((heterocycloaliphatic)aliphatic)carbonyl; or (heteroaraliphatic)carbonyl]; sulfonyl [e.g., aliphaticsulfonyl or aminosulfonyl]; sulfinyl [e.g., aliphaticsulfinyl]; sulfanyl [e.g., aliphaticsulfanyl]; nitro; cyano; halo; hydroxy; mercapto; sulfoxy; urea; thiourea; sulfamoyl; sulfamide; or carbamoyl. Alternatively, a heteroaryl can be unsubstituted.

Non-limiting examples of substituted heteroaryls include (halo)heteroaryl [e.g., mono- and di-(halo)heteroaryl]; (carboxy)heteroaryl [e.g., (alkoxycarbonyl)heteroaryl]; cyanoheteroaryl; aminoheteroaryl [e.g., ((alkylsulfonyl)amino) heteroaryl and ((dialkyl)amino)heteroaryl]; (amido)heteroaryl [e.g., aminocarbonylheteroaryl, ((alkylcarbonyl)amino) heteroaryl, ((((alkyl)amino)alkyl)aminocarbonyl)heteroaryl, (((heteroaryl)amino)carbonyl)heteroaryl, ((heterocycloaliphatic)carbonyl)heteroaryl, and ((alkylcarbonyl)amino)heteroaryl]; (cyanoalkyl)heteroaryl; (alkoxy)heteroaryl; (sulfamoyl)heteroaryl [e.g., (aminosulfonyl)heteroaryl]; (sulfonyl)heteroaryl [e.g., (alkylsulfonyl)heteroaryl]; (hydroxyalkyl)heteroaryl; (alkoxyalkyl)heteroaryl; (hydroxy)heteroaryl; ((carboxy)alkyl)heteroaryl; (((dialkyl)amino)alkyl]heteroaryl; (heterocycloaliphatic)heteroaryl; (cycloaliphatic)heteroaryl; (nitroalkyl)heteroaryl; (((alkylsulfonyl)amino)alkyl)heteroaryl; ((alkylsulfonyl)alkyl)heteroaryl; (cyanoalkyl)heteroaryl; (acyl)heteroaryl [e.g., (alkylcarbonyl)heteroaryl]; (alkyl)heteroaryl; or (haloalkyl)heteroaryl [e.g., trihaloalkylheteroaryl].

As used herein, an "alkoxy" group refers to an alkyl-O- group where "alkyl" has been defined previously.

As used herein, a "carboxy" group refers to -COOH, -COOR$^X$, -OC(O)H, -OC(O)R$^X$, when used as a terminal group; or -OC(O)- or -C(O)O- when used as an internal group.

As used herein, a "mercapto" group refers to -SH.

As used herein, a "sulfo" group refers to -$SO_3$H or -$SO_3$R$^X$ when used terminally or -S(O)$_3$- when used internally.

As used herein, a "sulfamide" group refers to the structure -NR$^X$-S(O)$_2$-NR$^Y$R$^Z$ when used terminally and -NR$^X$-S(O)$_2$-NR$^Y$- when used internally, wherein R$^X$, R$^Y$, and R$^Z$ have been defined above.

As used herein, a "sulfamoyl" group refers to the structure -O-S(O)$_2$-NR$^Y$R$^Z$ wherein R$^Y$ and R$^Z$ have been defined above.

As used herein, a "sulfonamide" group refers to the structure -S(O)$_2$-NR$^x$R$^y$ or -NR$^x$-S(O)$_2$-R$^z$ when used terminally; or -S(O)$_2$-NR$^x$- or -NR$^x$ -S(O)$_2$- when used internally, wherein R$^x$, R$^y$, and R$^z$ are defined above.

As used herein a "sulfanyl" group refers to -S-R$^X$ when used terminally and -S- when used internally, wherein R$^X$ has been defined above. Examples of sulfanyls include aliphatic-S-, cycloaliphatic-S-, aryl-S-, or the like.

As used herein, a "halogen" or "halo" group refers to fluorine, chlorine, bromine or iodine.

As used herein, an "oxo" refers to =O.

As used herein, the term "vicinal" generally refers to the placement of substituents on a group that includes two or more carbon atoms, wherein the substituents are attached to adjacent carbon atoms.

As used herein, the term "geminal" generally refers to the placement of substituents on a group that includes two or more carbon atoms, wherein the substituents are attached to the same carbon atom.

The terms "terminally" and "internally" refer to the location of a group within a substituent. A group is terminal when the group is present at the end of the substituent not further bonded to the rest of the chemical structure. Carboxyalkyl, i.e., $R^XO(O)C$-alkyl, is an example of a carboxy group used terminally. A group is internal when the group is present in the middle of a substituent of the chemical structure. Alkylcarboxy (e.g., alkyl-C(O)O- or alkyl-OC(O)-) and alkylcarboxyaryl (e.g., alkyl-C(O)O-aryl- or alkyl-O(CO)-aryl-) are examples of carboxy groups used internally.

As used herein, an "aliphatic chain" refers to a branched or straight aliphatic group (e.g., alkyl groups, alkenyl groups, or alkynyl groups). A straight aliphatic chain has the structure $-[CH_2]_v-$, where v is 1-12. A branched aliphatic chain is a straight aliphatic chain that is substituted with one or more aliphatic groups. A branched aliphatic chain has the structure $-[CQQ]_v-$ where Q is independently a hydrogen or an aliphatic group; however, Q shall be an aliphatic group in at least one instance. The term aliphatic chain includes alkyl chains, alkenyl chains, and alkynyl chains, where alkyl, alkenyl, and alkynyl are defined above.

The phrase "optionally substituted" is used herein interchangeably with the phrase "substituted or unsubstituted." As described herein, compounds of the invention can optionally be substituted with one or more substituents, such as are illustrated generally above, or as exemplified by particular classes, subclasses, and species of the invention. Unless otherwise noted, each of the specific groups for the variables recited herein can be optionally substituted with one or more substituents described herein. Each substituent of a specific group is further optionally substituted with one to three of halo, cyano, oxo, alkoxy, hydroxy, amino, nitro, aryl, cycloaliphatic, heterocycloaliphatic, heteroaryl, haloalkyl, and alkyl. For instance, an alkyl group can be substituted with alkylsulfanyl and the alkylsulfanyl can be optionally substituted with one to three of halo, cyano, oxo, alkoxy, hydroxy, amino, nitro, aryl, haloalkyl, and alkyl. As an additional example, the cycloalkyl portion of a (cycloalkyl)carbonylamino can be optionally substituted with one to three of halo, cyano, alkoxy, hydroxy, nitro, haloalkyl, and alkyl. When two alkoxy groups are bound to the same atom or adjacent atoms, the two alkxoy groups can form a ring together with the atom(s) to which they are bound.

As used herein, the term "substituted," whether preceded by the term "optionally" or not, refers generally to the replacement of hydrogen atoms in a given structure with the radical of a specified substituent. Specific substituents are described above in the definitions and below in the description of compounds and examples thereof. Unless otherwise indicated, an optionally substituted group can have a substituent at each substitutable position of the group, and when more than one position in any given structure can be substituted with more than one substituent selected from a specified group, the substituent can be either the same or different at every position. A ring substituent, such as a heterocycloalkyl, can be bound to another ring, such as a cycloalkyl, to form a spiro-bicyclic ring system, e.g., both rings share one common atom. As one of ordinary skill in the art will recognize, combinations of substituents envisioned by this invention are those combinations that result in the formation of stable or chemically feasible compounds.

As used herein, the phrase "stable or chemically feasible" refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40 °C or less, in the absence of moisture or other chemically reactive conditions, for at least a week.

To the extent that any of the compounds described have chiral centers, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centers such as carboxylates or amino groups, then all salt forms of said compounds are included herein. In the case of pharmaceutical uses, the salt should be seen as being a pharmaceutically acceptable salt.

Salts or pharmaceutically acceptable salts that may be mentioned include acid addition salts and base addition salts. Such salts may be formed by conventional means, for example by reaction of a free acid or a free base form of a compound with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound in the form of a salt with another counter-ion, for example using a suitable ion exchange resin.

Examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic,

(+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), α-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), malonic, (±)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The term "pharmaceutical composition" in the context of this invention means a composition comprising an active agent and comprising additionally one or more pharmaceutically acceptable carriers or pharmaceutically acceptable excipients. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may take the form, for example, of tablets, dragees, powders, elixirs, syrups, liquid preparations including suspensions, sprays, inhalants, tablets, lozenges, emulsions, solutions, cachets, granules, capsules and suppositories, as well as liquid preparations for injections, including liposome preparations.

The compounds of the invention may contain one or more isotopic substitutions, and a reference to a particular element includes within its scope all isotopes of the element. For example, a reference to hydrogen includes within its scope $^{1}H$, $^{2}H$ (D), and $^{3}H$ (T). Similarly, references to carbon and oxygen include within their scope respectively $^{12}C$, $^{13}C$ and $^{14}C$ and $^{16}O$ and $^{18}O$. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

Therapeutic dosages may be varied depending upon the requirements of the patient, the severity of the condition being treated, and the compound being employed. Determination of the proper dosage for a particular situation is within the skill of the art. Generally, treatment is initiated with the smaller dosages which are less than the optimum dose of the compound. Thereafter the dosage is increased by small increments until the optimum effect under the circumstances is reached. For convenience, the total daily dosage may be divided and administered in portions during the day if desired.

The magnitude of an effective dose of a compound will, of course, vary with the nature of the severity of the condition to be treated and with the particular compound and its route of administration. The selection of appropriate dosages is within the ability of one of ordinary skill in this art, without undue burden. In general, the daily dose range may be from about 10 μg to about 30 mg per kg body weight of a human and non-human animal, preferably from about 50 μg to about 30 mg per kg of body weight of a human and non-human animal, for example from about 50 μg to about 10 mg per kg of body weight of a human and non-human animal, for example from about 100 μg to about 30 mg per kg of body weight of a human and non-human animal, for example from about 100 μg to about 10 mg per kg of body weight of a human and non-human animal and most preferably from about 100 μg to about 1 mg per kg of body weight of a human and non-human animal.

COMBINATION THERAPY

An effective amount can be achieved in the method or pharmaceutical composition of the invention employing a compound of the invention (including a pharmaceutically acceptable salt or solvate (e.g., hydrate)) alone or in combination with an additional suitable therapeutic agent, for example, an antiviral agent or a vaccine. When "combination therapy" is employed, an effective amount can be achieved using a first amount of a compound of the invention and a second amount

of an additional suitable therapeutic agent.

In another embodiment of this invention, a compound of the invention and the additional therapeutic agent, are each administered in an effective amount (i.e., each in an amount which would be therapeutically effective if administered alone). In another embodiment, a compound of the invention and the additional therapeutic agent, are each administered in an amount which alone does not provide a therapeutic effect (a sub-therapeutic dose). In yet another embodiment, a compound of the invention can be administered in an effective amount, while the additional therapeutic agent is administered in a sub-therapeutic dose. In still another embodiment, a compound of the invention can be administered in a sub-therapeutic dose, while the additional therapeutic agent, for example, a suitable cancer-therapeutic agent is administered in an effective amount.

As used herein, the terms "in combination" or "co-administration" can be used interchangeably to refer to the use of more than one therapy (e.g., one or more prophylactic and/or therapeutic agents). The use of the terms does not restrict the order in which therapies (e.g., prophylactic and/or therapeutic agents) are administered to a subject.

Co-administration encompasses administration of the first and second amounts of the compounds of the co-administration in an essentially simultaneous manner, such as in a single pharmaceutical composition, for example, capsule or tablet having a fixed ratio of first and second amounts, or in multiple, separate capsules or tablets for each. In addition, such co-administration also encompasses use of each compound in a sequential manner in either order.

In one embodiment, a compound of the invention and an additional therapeutic agent are administered separately, sequentially or simultaneously to the subject.

When co-administration involves the separate administration of the first amount of a compound of the invention and a second amount of an additional therapeutic agent, the compounds are administered sufficiently close in time to have the desired therapeutic effect. For example, the period of time between each administration which can result in the desired therapeutic effect, can range from minutes to hours and can be determined taking into account the properties of each compound such as potency, solubility, bioavailability, plasma half-life and kinetic profile. For example, a compound of the invention and the second therapeutic agent can be administered in any order within 24 hours of each other, within 16 hours of each other, within 8 hours of each other, within 4 hours of each other, within 1 hour of each other or within 30 minutes of each other.

More, specifically, a first therapy (e.g., a prophylactic or therapeutic agent such as a compound of the invention) can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy (e.g., a prophylactic or therapeutic agent such as an anticancer agent) to a subject.

It is understood that the method of co-administration of a first amount of a compound of the invention and a second amount of an additional therapeutic agent can result in an enhanced or synergistic therapeutic effect, wherein the combined effect is greater than the additive effect that would result from separate administration of the first amount of a compound of the invention and the second amount of an additional therapeutic agent.

As used herein, the term "synergistic" refers to a combination of a compound of the invention and another therapy (e.g., a prophylactic or therapeutic agent), which is more effective than the additive effects of the therapies. A synergistic effect of a combination of therapies (e.g., a combination of prophylactic or therapeutic agents) can permit the use of lower dosages of one or more of the therapies and/or less frequent administration of said therapies to a subject. The ability to utilize lower dosages of a therapy (e.g., a prophylactic or therapeutic agent) and/or to administer said therapy less frequently can reduce the toxicity associated with the administration of said therapy to a subject without reducing the efficacy of said therapy in the prevention, management or treatment of a disorder. In addition, a synergistic effect can result in improved efficacy of agents in the prevention, management or treatment of a disorder. Finally, a synergistic effect of a combination of therapies (e.g., a combination of prophylactic or therapeutic agents) may avoid or reduce adverse or unwanted side effects associated with the use of either therapy alone.

The presence of a synergistic effect can be determined using suitable methods for assessing drug interaction. Suitable methods include, for example, the Sigmoid-Emax equation (Holford, N.H.G. and Scheiner, L.B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T.C. and Talalay, P., Adv. Enzyme Regul. 22: 27-55 (1984)). Each equation referred to above can be applied with experimental data to generate a corresponding graph to aid in assessing the effects of the drug combination. The corresponding graphs associated with the equations referred to above are the concentration-effect curve, isobologram curve and combination index curve, respectively.

In one aspect the invention provides a pharmaceutical composition comprising a compound, pharmaceutically acceptable salt, solvate, hydrate, tautomer or optical isomer of the invention, a pharmaceutically acceptable excipient and at least one additional therapeutic agent. In some embodiments the compound, pharmaceutically acceptable salt, solvate, hydrate, tautomer or optical isomer of the invention and the at least one additional therapeutic agent are co-formulated. In

some embodiments the compound, pharmaceutically acceptable salt, solvate, hydrate, tautomer or optical isomer of the invention and the at least one additional therapeutic agent are formulated separately.

In another aspect the invention provides a kit comprising a compound, pharmaceutically acceptable salt, solvate, hydrate, tautomer or optical isomer of the invention and at least one additional therapeutic agent. The kit may comprise instructions to administer the compound, pharmaceutically acceptable salt, solvate, hydrate, tautomer or optical isomer of the invention and the at least one additional therapeutic agent to a subject in need thereof.

In another aspect the invention provides a combination therapy for use as a medicament, wherein the combination therapy comprises administering a compound, pharmaceutically acceptable salt, solvate, hydrate, tautomer or optical isomer of the invention to a subject in need thereof and administering an additional therapeutic agent to the subject in need thereof.

The compound, pharmaceutically acceptable salt, solvate, hydrate, tautomer or optical isomer of the invention and the additional therapeutic agent may be administered to the subject separately, sequentially or simultaneously.

The pharmaceutical composition, kit and/or combination therapy may be for use in the treatment of a gastrointestinal disorder or a pulmonary disease or condition. The gastrointestinal disorder may be selected from the group consisting of constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease, and ischemic colitis. The pulmonary disease or condition may be selected from the group consisting of chronic obstructive pulmonary diseases, asthma, chronic bronchitis, cystic fibrosis, emphysema, chronic idiopathic cough, hyperactive airway disorder, and idiopathic pulmonary fibrosis.

The at least one additional therapeutic agent may be selected from the group consisting of aminosalicylates, corticosteroids, immunomodulators and combinations thereof. Aminosalicylates are also known as 5-aminosalicylates (5-ASAs). The at least one additional therapeutic agent may be an aminosalicylate. The aminosalicylate may be mesalamine. The aminosalicylate may be sulfasalazine. The at least one additional therapeutic agent may be a corticosteroid. The corticosteroid may be budesonide. The at least one additional therapeutic agent may be an immunomodulator. The immunomodulator may be thiopurine. The immunomodulator may be methotrexate.

## PHARMACEUTICAL COMPOSITIONS

While it is possible for the active compound to be administered alone, it is preferable to present it as a pharmaceutical composition (e.g. formulation).

Accordingly, in another embodiment of the invention, there is provided a pharmaceutical composition comprising at least one compound of Formula (1) as defined above together with at least one pharmaceutically acceptable excipient.

When the pharmaceutical composition comprises at least one additional therapeutic agent, the compound of the invention and the at least one additional therapeutic agent may be co-formulated or the compound of the invention and the at least one additional therapeutic agent may be formulated separately.

The composition may be a tablet composition.

The composition may be a capsule composition.

The pharmaceutically acceptable excipient(s) can be selected from, for example, carriers (e.g. a solid, liquid or semi-solid carrier), adjuvants, diluents (e.g solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, antifungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds of the Formula (1) can be formulated in accordance with known techniques, see for example, Remington's Pharmaceutical Sciences, Mack Publishing Company, Easton, PA, USA.

The pharmaceutical compositions can be in any form suitable for oral, parenteral, topical, intranasal, intrabronchial, sublingual, ophthalmic, otic, rectal, intra-vaginal, or transdermal administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

The pharmaceutical compositions typically comprise from approximately 1% (w/w) to approximately 95%, preferably% (w/w) active ingredient and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredient and from 80% (w/w) to 10% of a pharmaceutically excipient or combination of excipients. The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, pre-filled syringes, dragées, powders, tablets or capsules.

Tablets and capsules may contain, for example, 0-20% disintegrants, 0-5% lubricants, 0-5% flow aids and/or 0-99% (w/w) fillers/ or bulking agents (depending on drug dose). They may also contain 0-10% (w/w) polymer binders, 0-5% (w/w) antioxidants, 0-5% (w/w) pigments. Slow release tablets would in addition typically contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

The pharmaceutical formulations may be presented to a patient in "patient packs" containing an entire course of treatment in a single package, usually a blister pack.

The compounds of the Formula (1) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 milligrams to 1 gram, of active compound.

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

METHODS FOR THE PREPARATION OF COMPOUNDS OF FORMULA (I)

**General Synthetic Schemes**

Scheme 1

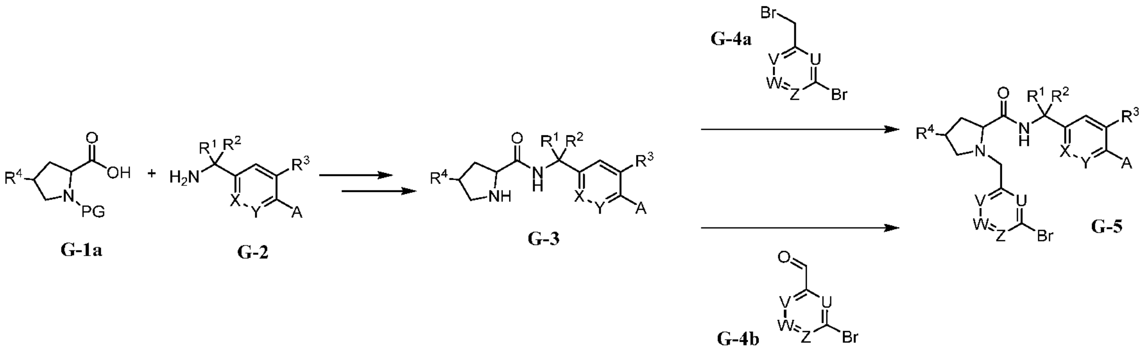

G-1a G-2 G-3 G-4a G-4b G-5

Referring to Scheme 1, compounds of the general formula G-5 can be synthesized from proline derivatives of formula G-1a, where PG is a protecting group, such as BOC. Amine compounds of the general formula G-2 are coupled with G-1a, followed by deprotection of the amine protecting group, PG, to provide amides of the general formula G-3. Common coupling reagents to achieve this transformation are well known to those having skill in the art, e.g. EDC, DCC, BOP, etc. Compounds of formula G-3 can be reacted with benzyl compounds of the general formula G-4a to produce compounds of the general formula G-5 via SN-2 reaction conditions, where bromide is the leaving group, or with aldehyde compounds of the general formula G-4b to produce compounds of the general formula G-5 via reductive amination conditions.

Scheme 2

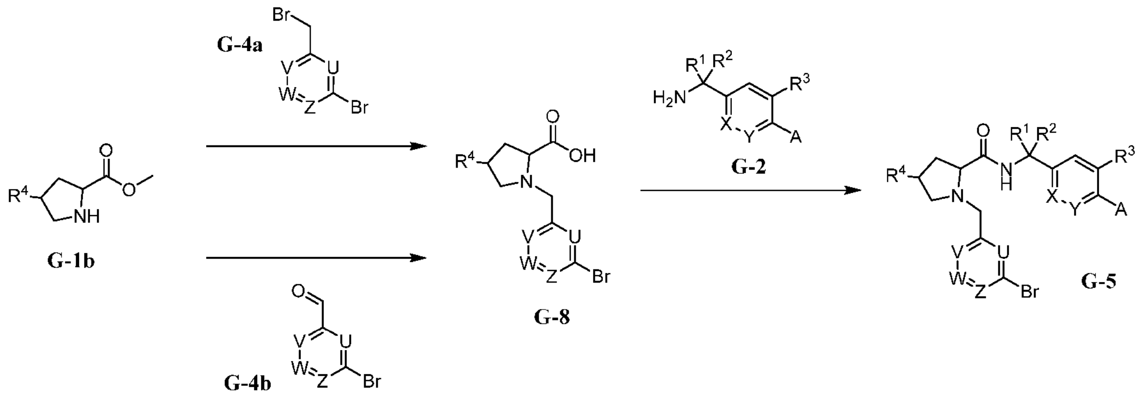

G-1b G-4a G-4b G-8 G-2 G-5

Referring to Scheme 2, compounds of the general formula G-5 can also be prepared from proline derivatives of the general formula G-1b, where the carboxylic acid of G-1b is protected by a simple ester, such as a methyl ester. Compounds of the general formula G-8 can be synthesized by the reaction of G-1b with a benzyl compound of the general formula G-4a via SN-2 conditions, where bromide is the leaving group, or with an aldehyde compound of the general formula G-4b via reductive amination conditions. Compounds of the general formula G-8 can then be coupled with an amine compound of the general formula G-2 using coupling conditions discuss above to provide compounds of the general formula G-5.

Scheme 3

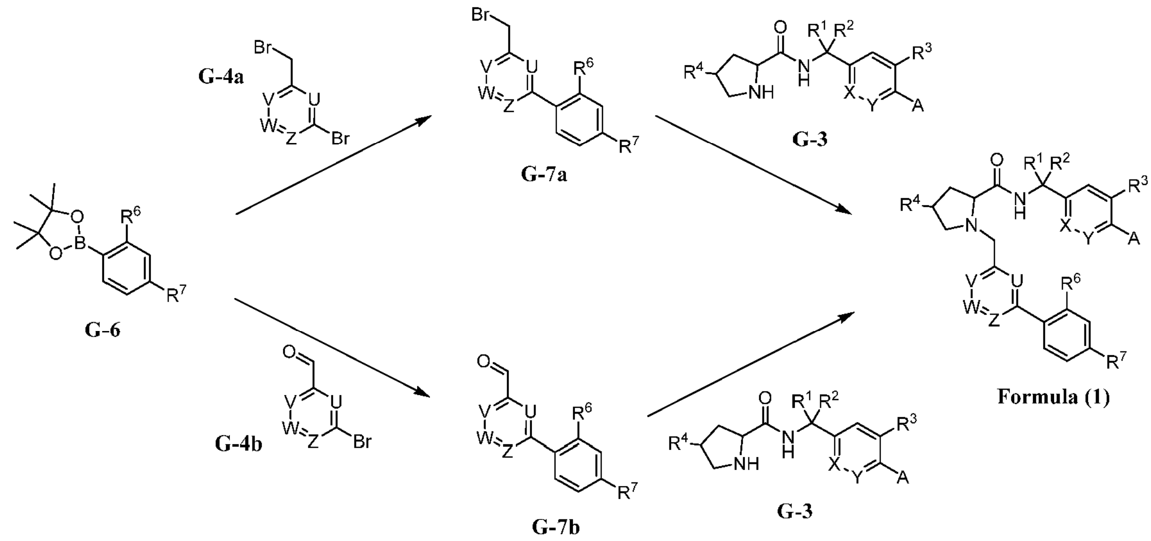

G-4a G-7a G-3 G-6 Formula (1) G-4b G-7b G-3

Referring to Scheme 3, compounds of formula (1) can be synthesized from organoboronate compounds of the general formula G-6 via palladium catalyzed coupling conditions, using a palladium catalyst selected from those known to the skilled artisan, e.g. $PdCl_2$(dppf)DCM. Compounds of the general formula G-6 can be coupled with aryl bromide compounds of the general formula G-4a or benzaldehyde compounds of the general formula G-4b to produce compounds of the general formula G-7a or G-7b, respectively. Benzyl compounds of the general formula G-7a, where bromide is the leaving group, react with amine compounds of the general formula G-3 via SN-2 reaction conditions to produce compounds of formula (1). Benzaldehyde compounds of the general formula G-7b react with amine compounds of the general formula G-3 via reductive amination conditions to produce compounds of formula (1).

Scheme 4

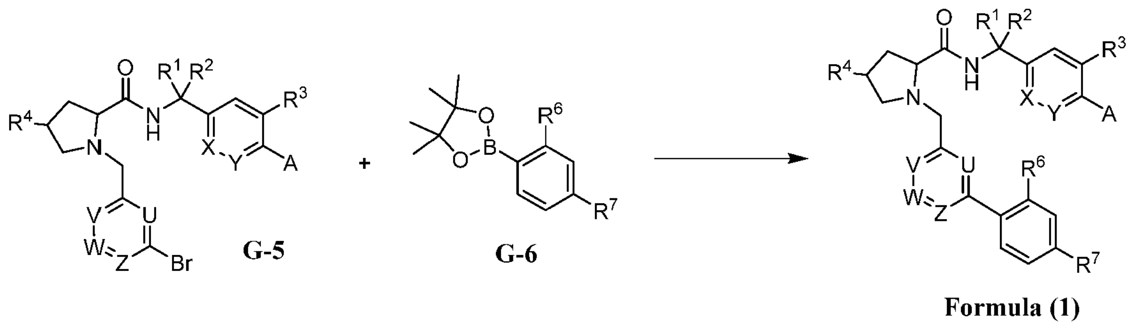

G-5 + G-6 Formula (1)

Referring to Scheme 4, compounds of the general formula G-5 can be coupled with organoboronate compounds of the general formula G-6 using palladium catalyzed coupling chemistry discussed above to provide compounds of formula (1). It is also well known to the skilled artisan that some compounds of formula (1) will have the ability to undergo further derivatization. For example, compounds of formula (1), wherein $R^7$ is OH, can be further derivatized by reaction with an appropriate electrophile, such as sulfamyl chloride to produce a sulfamoyloxy derivative, carbamic chloride to produce a carbamate derive, and the like.

## Chromatographic methods

### LC/MS Method A

Instruments: Acquity UPLC with Photodiode Array Detector and QDA mass detector; Column: Acquity C-18, 1.6 micron, 50 x 1.6 mm; Gradient [time (min)/solvent B in A (%)]: 0.00/3, 0.20/3, 2.70/98. 3.00/100, 3.50/100, 3.51/3, 4.00/3; Solvents: solvent A = 0.1% formic acid in water; solvent B = 0.1% formic acid in water / acetonitrile (10:90); column temperature 35 °C; Flow rate 0.9 mL/min.

**LC/MS Methods B & C**

Instruments: HP 1100 with G1315A DAD, Waters Micromass ZQ; Column: Phenomenex Gemini-NX C-18, 3 micron, 2.0 x 30 mm; **Method B** Gradient [time (min)/solvent B in A (%)]: 0.00/2, 0.10/2, 2.50/95, 3.50/95; **Method C** Gradient [time (min)/solvent B in A (%)]: 0.00/2. 0.01/2. 8.40/95, 10.00/95; Solvents: solvent A = 2.5 L $H_2O$ + 2.5 mL 28% ammonia in $H_2O$ solution; solvent B = 2.5 L MeCN + 135 mL $H_2O$ + 2.5 mL 28% ammonia in $H_2O$ solution. Injection volume 1 μL; UV detection 230 to 400 nm; Mass detection 130 to 800 AMU; column temperature 45 °C; Flow rate 1.5 mL/min.

**LC/MS Method D**

Instruments: Agilent 1260 Infinity LC with Diode Array Detector, Agilent 6120B Single Quadrupole MS with API-ES Source; Column: Restek Penta Fluoro Phenyl Propyl, 3 micron, 2.1 x 30 mm. Gradient [time (min)/solvent B in A (%)]: 0.00/2, 0.1/2, 8.4/95, 10/95, 10.1/2, 12/2; Solvents: solvent A = water (2.5 L) with 2.5 mL Formic acid; Solvent B = MeCN (2.5 L) with 125 mL water and 2.5 mL Formic acid. Injection volume 0.5 μL; UV detection 190 to 400 nm; Mass detection 130 to 800 AMU; column temperature 40 °C; Flow rate 1.5 mL/min.

**LC/MS Method E**

Instruments: Agilent Technologies 1290 series with Binary Pump, Diode Array Detector and G6120A, Quadrupole MS. Column: Agilent Poroshell 120 EC- C18, 2.7 μm, 4.6×50 mm; Gradient [time (min)/solvent B in A (%)]: 0.00/10, 0.50/10, 4.00/90, 4.50/100, 4.51/10, 5.00/10;Solvents: solvent A = 0.05% formic acid in water; solvent B =0.05% formic acid in acetonitrile; Column temperature 35 °C; Flow rate 1.0 mL/min.

**LC/MS Method F**

Instruments: Water 2690 with Photodiode Array Detector and QDA mass detector; Column: X-Bridge C-18, 5 micron, 100 x 1.6 mm; Gradient [time (min)/solvent B in A (%)]: 0.01/10, 1.00/10, 5.00/100, 7.00/100, 7.50/10, 8.00/10; Solvents: solvent A = 0.1% formic acid and 10mM ammonium carbonate in water; solvent B = acetonitrile; Column temperature 35 °C; Flow rate 0.9 mL/min.

**LC/MS Method G**

Instruments: Water 2690 with 996 Photodiode Array Detector and Micromass ZQ mass detector; Column: C-18, 3.5 micron, 50 x 4.6 mm; Gradient [time (min)/solvent B in A (%)]: 0.00/10, 1.00/10, 4.00/100, 6.00/100, 6.50/10, 7.00/10; Solvents: solvent A = 10mM ammonium bicarbonate in water; solvent B = methanol; Flow rate 1.2 mL/min.

**LC/MS Method H**

Instruments: Agilent 1290 RRLC with Agilent 6120 Mass detector; Column: X-Bridge C18 50*4.6mm, 3.5μm; Gradient: 95:5 at 0.01 min, 15:85 at 2.8 min, 5:95 at 3.5 min till 5.0min, 95:5 at 5.01min up to 6.0 min; Solvents: solvent A = 5 mM Ammonium bicarbonate in Water; solvent B = Acetonitrile; Flow rate 1 mL/min.

**LC/MS Method I**

Instruments: Agilent 1290 RRLC with Agilent 6120 Mass detector; Column: BEH C18 2.1 X 50 mm, 1.7 μm; Gradient: 98:2 at 0.01 min up to 0.5 min, 30:70 at 3.0 min(Flow rate: 0.45 ml/min), 5:95 at 4.0 min to till 5.5 min(Flow rate: 0.50 ml/min), 98:2 at 5.51 min up to 6.0 min(Flow rate: 0.45 ml/min); Solvents: solvent A = 5mM Ammonium acetate &0.1% FA in water; solvent B = 0.1% FA in Acetonitrile; Flow rate 0.45 mL/min.

**LC/MS Method J**

Instruments: Waters Acquity H-Class LCMS, Mass Lynx software, PDA Detector and QDa Mass Detector; Column: Gemini-NX C18, 3 μm, 30 x 2mm; Gradient: [Time (min)/ % A: % B] 3 min run: [0.00/ 100:0], [1.30/ 0:100], [1.551 0:100], [1.601 100:0], [3.00/ 100:0]; Solvents: (A): 50 mM ammonium acetate aqueous solution at pH 7.40 (B): acetonitrile; Column temperature 40°C; Flow rate 0.5 mL/min; Mass spec solvent: solution of 0.1% formic acid in 90% v/v methanol: water; Injection 1.0 μL; λ Range 200 nm to 500 nm.

**LC/MS Method K**

Instruments: Waters Acquity H Class and SQ Mass Detector; Column: BEH C18 2.1 X 50 mm, 1.7 μm; Gradient: 95:5 at 0.01 min up to 0.6 min (Flow rate: 0.55 ml/min), 30:70 at 0.6 min (Flow rate: 0.60 ml/min), 10:90 at 0.80 min (Flow rate: 0.65 ml/min), 0:100 at 1.1 min up to 1.70 min (Flow rate: 0.65 ml/min), 95:5 at 1.71 min up to 2.0 min(Flow rate: 0.55 ml/min); Solvents: solvent A = 2mM Ammonium acetate &0.1% FA in water; solvent B = 0.1% FAin Acetonitrile; Flow rate 0.55 mL/min.

**LC/MS Method L**

Instruments: Waters Acquity H Class with SQ Mass Detector; Column: BEH C18 2.1 X 50 mm, 1.7 μm; Gradient: 98:2 at 0.01 min up to 0.5 min, 30:70 at 3.0 min(Flow rate: 0.45 ml/min), 5:95 at 4.0 min to till 5.5 min(Flow rate: 0.50 ml/min), 98:2 at 5.51 min up to 6.0 min(Flow rate: 0.45 ml/min); Solvents: solvent A = 5mM Ammonium acetate &0.1% FA in water; solvent B = 0.1% FA in Acetonitrile; Flow rate 0.45 mL/min.

**Prep HPLC Purification**

Where stated, intermediates or final compounds were purified by reversed phase preparative HPLC conditions using the instruments and methods given below:

**HPLC method A**

Gilson Semi Preparative HPLC System- including a 321 Pump, a 171 Diode Array Detector and a GX-271 Liquid Handler with Gilson Trilution software. Column Phenomenex Kinetix C18, 100 × 30 mm, 5 μm. Flow 30 mL/min, gradient of Solvent B in Solvent A: 5 % to 35 % Solvent B (over 10 min), 100 % Solvent B (for 2 min); Solvent A: Water with 0.1% of TFA. Solvent B: MeCN].

**HPLC method B**

Gilson Semi Preparative HPLC System- including a 321 Pump, a 171 Diode Array Detector and a GX-271 Liquid Handler with Gilson Trilution software. Column Gemini-NX C18, 100 × 30 mm, 5 μm. Flowrate 30 mL/min. Gradient of Solvent B in Solvent A: 5 % to 35 % Solvent B (over 10 min), 100 % Solvent B (for 2 min); Solvent A: Water with 0.2% of 28% aq. ammonia. Solvent B: MeCN].

**HPLC method C**

Gilson Semi Preparative HPLC system- including Dual Piston Pumps 331 and 332, a 171 Diode Array Detector and a GX-271 Liquid Handler with Gilson Trilution software. Column Gemini-NX C18, 100 × 30 mm, 5 μm. Flowrate 30 mL/min, Gradient of Solvent B in Solvent A: 5 % to 35 % Solvent B (over 10 min), 100 % Solvent B (for 2 min); Solvent A: Water with 0.2% of 28% aq. ammonia. Solvent B: MeCN].

**HPLC method D**

Agilent Preparative 1200 infinity series

Column Sunfire C18 250 x 19mm 5 μm. Flowrate 17mL/min, Gradient of Solvent B in Solvent A 0.00/10. 17.00/16, 17.01/98, 19.00/98, 19.01/10, 21/10. Solvent A: Water with 0.1%FA, Solvent B: MeCN.

**HPLC method E**

SHIMADZU Preparative HPLC System- including a LC-20AP Pump, a SPD-20A Detector and Labsolutions (version 5.90) software. Column: Agilent 10, Prep-C18, 250x21.2 mm. Solvent/Gradient: 10-80% Acetonitrile in water containing 0.1% TFA. Flow Rate: 20 mL/min.

**HPLC method F**

Shimadzu LC-20AP and UV detector. The column used sunfire c18 (250*19) mm, 5μ. Column flow was 12.0 ml/min. Mobile phase were used (A) 0.1% formic acid in Water and (B) 100% Acetonitrile. The gradient solvent B was

0-20% over 30 min, 100% over 2 min then 100-0% over 5 min.

**HPLC method G**

Waters Xbridge $C_{18}$ 150 * 50mm * 10um; mobile phase: [water (10mM $NH_4HCO_3$) - ACN]; B%: 33%-63%,min.

**HPLC method H**

Phenomenex luna $C_{18}$ 150 * 25mm * 10um; mobile phase: [water (0.225% FA) - ACN]; B%: 3% - 33%, 10min.

**HPLC method I**

Shimadzu LC-20AP and UV detector. The column used was sunfire c18 (250*19) mm, 5 microns, Column flow was 14.0 ml/min. Mobile phase were used (A) 0.1% Formic acid in water and (B) 100% acetonitrile. The gradient solvent B was 0-20% over 23 min, then 20-20% over 2 min, 100% over 2 min, then 100-0% over 6 min.

EXAMPLES

Compounds of Formula (1) can be prepared in accordance with synthetic methods known to the skilled practitioner. The following examples are provided so that the invention may be more fully understood.

Where no preparative routes are included, the relevant intermediate is commercially available. Commercial reagents were utilized without further purification. Final compounds and intermediates are named using ChemDraw Professional, Version 17.0.0.206 (121). Room temperature (RT) refers to approximately 20-27 °C. $^1$H NMR spectra were recorded at 400 or 500 MHz on either a Bruker, Varian or Jeol instrument. Chemical shift values are expressed in parts per million (ppm), i.e. ($\delta$), relative to a deuterated solvent, such as chloroform-*d* (7.26 ppm), DMSO-$d_6$ (2.50 ppm), or methanol-$d_4$ (3.31 ppm). The following abbreviations are used for the multiplicity of the NMR signals: s=singlet, br=broad, d=doublet, t=triplet, q=quartet, m=multiplet. Coupling constants are listed as *J* values, measured in Hz. NMR and mass spectroscopy results were corrected to account for background peaks. Chromatography refers to column chromatography performed using 60 - 120 mesh or 40 - 633 $\mu$m, 60 Å silica gel and executed under nitrogen pressure (flash chromatography) conditions or automated flash chromatography using Biotage Isolera equipment. Microwave-mediated reactions were performed in Biotage Initiator or CEM Discover microwave reactors.

**Synthetic Preparation of the Intermediates**

**Intermediate 1: methyl 4-((S)-1-((R)-pyrrolidine-2-carboxamido)ethyl)benzoate hydrochloride (Intermediate 1-HCl)**

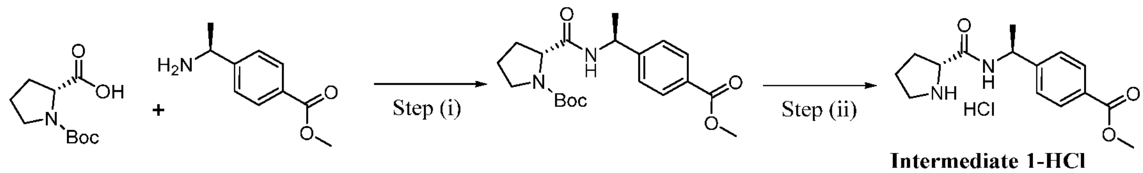


Intermediate 1-HCl

**Step (i):** To a mixture of N-Boc-D-proline (2.0 g, 9.29 mmol) in DMF (43.85 mL), were added diisopropylethylamine (4.82 mL, 27.87 mmol), HATU (4.25 g, 11.15 mmol) and Methyl 4-[(1*S*)-1-aminoethyl]benzoate (2.0 g, 11.15 mmol). The mixture was stirred at RT overnight, after which the mixture was diluted with EtOAc and water (1:1) and the organic layers were separated. The organic phase was washed with brine (2 x 50 mL) and dried (frit) before being concentrated in vacuo. The residue was purified by flash column chromatography (normal phase, 25 g Biotage® SNAP KP-Sil, 70 mL per min, gradient 0% to 100% Ethyl Acetate in Isohexane], to afford *tert*-butyl (2R)-2-[[(1S)-1-(4-methoxycarbonylphenyl) ethyl]carbamoyl]pyrrolidine-1-carboxylate (3.383 g, 9.0 mmol, 97% yield) as a pale yellow solid. LC/MS (Method B) 2.05 min $[M+H]^+$ 377

**Step (ii):** To a solution of *tert*-butyl (2R)-2-[[(1S)-1-(4-methoxycarbonylphenyl)ethyl]carbamoyl]pyrrolidine-1-carboxylate (4.03 g, 10.71 mmol) in 1,4-Dioxane (20.2 mL), was added 4 M HCl in 1,4-Dioxane (20.2 mL, 10.71 mmol), which was left to stir for 3 hours at RT. 1,4-Dioxane (20.2 mL) was added to the reaction mixture once completed, monitored by LC/MS. The reaction mixture was then concentrated in vacuo to give Intermediate 1-HCl (3.39 g, 101%) as a white solid.

LC/MS (Method B) 1.73 min $[M+H]^+$ 277.

**Intermediate 2: methyl 4-((S)-1-((R)-1-(3-bromobenzyl)pyrrolidine-2-carboxamido)ethyl)benzoate (Intermediate 2)**

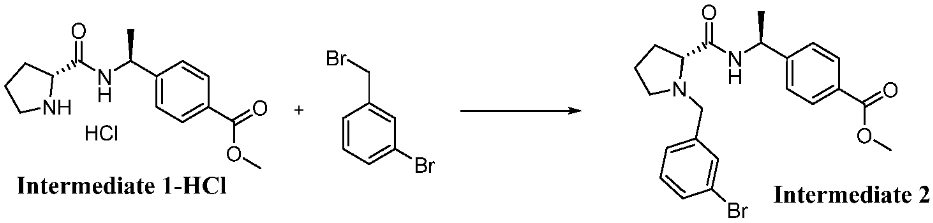

Intermediate 1-HCl

Intermediate 2

To a solution of Intermediate 1-HCl (1.0 g, 3.2 mmol) in MeCN (96 mL), were added Potassium carbonate (1.1 g, 7.99 mmol) and 3-Bromobenzyl bromide (1.6 g, 6.39 mmol). The resulting mixture was heated to 70 °C overnight and then filtered through a phase separator and washed with EtOAc before being concentrated in vacuo. The residue was taken up in EtOAc and the organic phase was washed with water and brine, dried through a hydrophobic frit and concentrated in vacuo. The residue was purified by flash column chromatography (10 g Biotage® SNAP KP-Sil, 30 mL per min, gradient 30 % to 100 % ethyl acetate in isohexane) to give Intermediate 2 (1.18 g, 83%) as a white solid. LC/MS (Method B) 1.73 min $[M+H]^+$ 445

**Intermediate 3: methyl 4-((S)-1-((2R,4R)-4-hydroxypyrrolidine-2-carboxamido)ethyl)benzoate hydrochloride (Intermediate 3)**

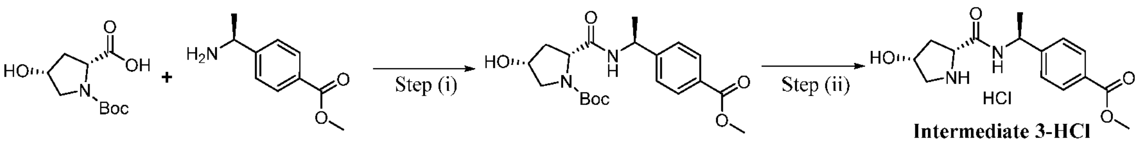


Intermediate 3-HCl

Intermediate 3-HCl was synthesized in a process analogous to Intermediate 1, using (2R,4R)-1-tert-butoxycarbonyl-4-hydroxy-pyrrolidine-2-carboxylic acid. LC/MS (Method B) 1.47 min $[M+H]^+$ 293

**Intermediate** 4: **methyl 4-((S)-1-((2R,4R)-1-(3-bromobenzyl)-4-hydroxypyrrolidine-2-carboxamido)ethyl)benzoate (Intermediate 4)**

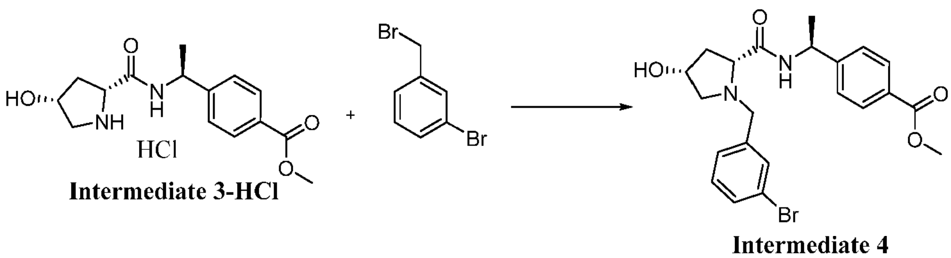

Intermediate 3-HCl

Intermediate 4

Intermediate 4 was synthesized in a process analogous to Intermediate 2, using Intermediate 3-HCl. LC/MS (Method B) 2.17 min $[M+H]^+$ 463

**Intermediate 5: methyl 4-((S)-1-((R)-1-(3-bromo-4-methoxybenzyl)pyrrolidine-2-carboxamido)ethyl)benzoate (Intermediate 5)**

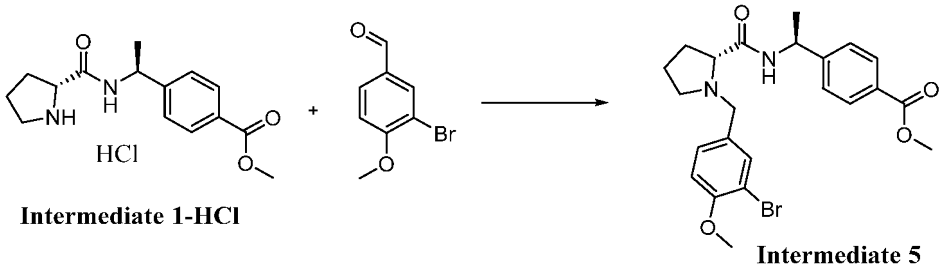

Intermediate 1-HCl

Intermediate 5

To a mixture of Intermediate 1-HCl (300 mg, 0.96 mmol) and 3-Bromo-4-methoxybenzaldehyde (412.49 mg, 1.92 mmol) in DCM (5 mL) was added sodium triacetoxyborohydride (426.87 mg, 2.01 mmol). The mixture was stirred at RT for 16 hours, after which it was diluted with sat $NaHCO_3$. The organic layers were separated, washed with brine, dried (by passing through a hydrophobic frit), and concentrated. The residue was purified by flash column chromatography (normal phase, 10 g Biotage® SNAP KP-Sil, 30 mL per min, gradient 30% to 100% ethyl acetate in isohexane) to afford Intermediate 5 (380 mg,0.80 mmol, 83% yield) as transparent gum, which was sonicated in $Et_2O$ to afford a fluffy white solid. LC/MS (Method B) 2.36 min $[M+H]^+$ 475.

**Intermediate 6: methyl 4-((S)-1-((R)-1-(3-bromo-5-methoxybenzyl)pyrrolidine-2-carboxamido)ethyl)benzoate (Intermediate 6)**

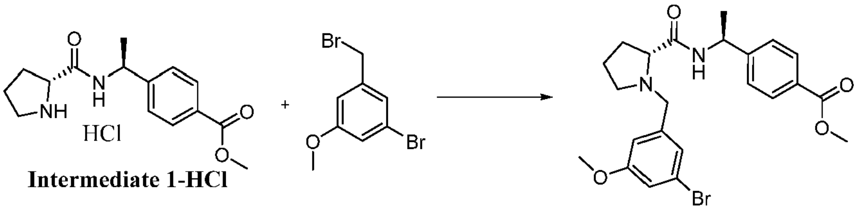

Intermediate 1-HCl

Intermediate 6

To a solution of Intermediate 1-HCl (300 mg, 0.96 mmol) in MeCN (10.8 mL) were added 1-bromo-3-(bromomethyl)-5-methoxybenzene (0.15 mL, 1.92 mmol) and potassium carbonate (331.4 mg, 2.4 mmol). The resulting mixture was heated to reflux for 16 hours, after which it partitioned between EtoAc and water. The organic layers were separated, washed with brine, dried (frit), and concentrated. The residue was purified by flash column chromatography (25 g Biotage® SNAP KP-Sil 60 mL per min, gradient 0% to 90% EtOAc in isohexane) to afford Intermediate 6 (300 mg, 0.63 mmol, 66% yield) as a white solid. LC/MS (Method B) 2.44 min $[M+H]^+$ 475

**Intermediate 7: (3-bromobenzyl)-D-proline (Intermediate 7)**

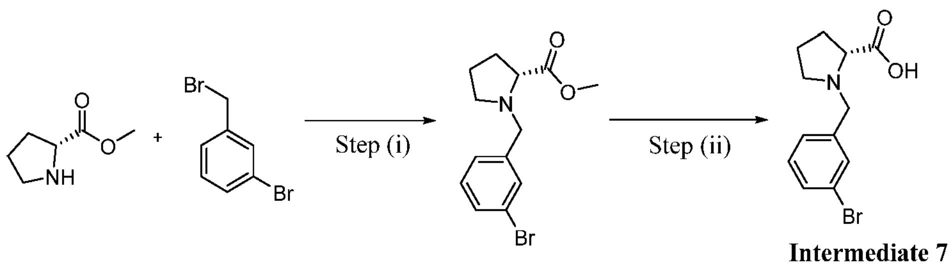


Intermediate 7

**Step (i):** Methyl D-prolinate hydrochloride (10.00 g, 60.38 mmol) was dissolved in ACN (120 mL) and $Na_2CO_3$ (12.80 g, 120.77 mmol) was added and the reaction mixture was allowed stir at room temperature for 15 min. 1-Bromo-3-(bromomethyl) benzene (18.11 g, 72.46 mmol) was then added and the reaction mixture was stirred at 800 °C for 16 hours. The reaction mixture was then partitioned between water (300 mL) and EtOAc (200 mL), and the aqueous layer was again extracted with EtOAc (2x100 mL). The organic layers were combined and dried ($Na_2SO_4$), the solvent was removed *in vacuo,* and the crude product was purified by gradient column chromatography. The product eluted at 0% to

20% EtOAc in hexane to afford pure methyl (3-bromobenzyl)-D-prolinate (11.00 g, 61.34%) as a yellow sticky material. LC/MS: (Method A): m/z 298 $[M+H]^+$ at 1.35 min.

**Step (ii):** Methyl (3-bromobenzyl)-D-prolinate (10.60 g, 35.68 mmol) was dissolved in dioxane (50 mL) and water (30 mL), LiOH monohydrate (7.48 g, 178.42 mmol) was added at room temperature, and the reaction mixture was allowed to stir at room temperature for 6 hours. Glacial Acetic Acid (20 mL) was then added to bring the solution to pH ~6, then the reaction mixture was concentrated *in vacuo* to obtain the crude product which was purified by reverse phase gradient flash column chromatography (reverse phase, C18 silica), product eluted at 0% to 13% MeCN in water (0.1% FA as a modifier) to afford pure ((3-bromobenzyl)-D-proline) (7.8 g, 77.%) as white solid. LC/MS: (Method A): m/z 284 $[M+H]^+$ at 1.28 min.

**Intermediate 8: methyl (R)-4-((1-(3-bromobenzyl)pyrrolidine-2-carboxamido)methyl)-2-hydroxybenzoate (Intermediate 8)**

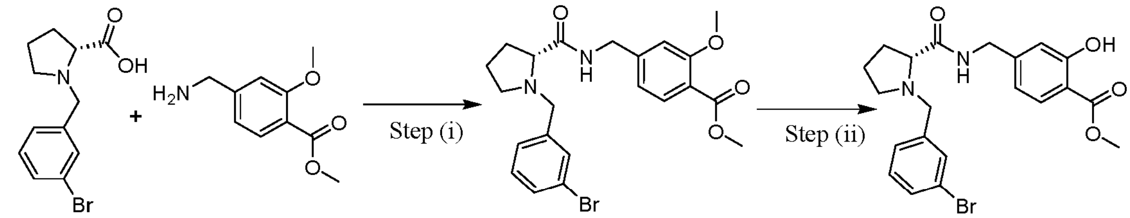


Intermediate 7

Intermediate 8

**Step (i):** Intermediate 7 (2.50 g, 8.83 mmol) was dissolved in MeCN (15 mL) and methyl 4-(aminomethyl)-2-methoxybenzoate (1.90 g, 9.71 mmol) was added to the mixture at room temperature. Propylphosphonic anhydride (50% solution in EtOAC) (11.42 mL, 17.66 mmol) was then added and the reaction mixture was allowed to stir at room temperature for 30 min. TEA (3.88 mL, 26.54 mmol) was then added at 0 °C and reaction mixture was allowed to stir at room temperature for 16 hours. The reaction mixture was partition between saturated aqueous $NaHCO_3$ (100 mL) and EtOAc (100 mL). The aqueous layer was further extracted with EtOAc (2 x 70 mL). The organic layers were combined and dried over $Na_2SO_4$, the solvent was removed *in vacuo,* and the crude product was purified by gradient flash column chromatography (normal phase, silica), product eluted at 0% to 64% EtOAc in hexane to afford pure methyl (R)-4-((1-(3-bromobenzyl) pyrrolidine-2-carboxamido)methyl)-2-methoxybenzoate (2.50 g, 60%) as a yellow sticky material. LC/MS: (Method A): m/z 461 $[M+H]^+$ , at 1.56 min.

**Step (ii):** Methyl (R)-4-((1-(3-bromobenzyl)pyrrolidine-2-carboxamido)methyl)-2-methoxybenzoate (0.30 g, 0.65 mmol) was dissolved in DCM (30 mL) and reaction mixture was cooled to -78 °C. $BBr_3$ was added as a 1 M solution in DCM (3.3 mL, 3.26 mmol), and the reaction mixture was stirred at -78 °C for 1 hour. Reaction mixture was then partitioned between saturated aqueous solution of $NaHCO_3$ (70 mL) and DCM (70 mL), and the aqueous layer was again extracted with DCM (2x40 mL). The organic layers were combined and dried ($Na_2SO_4$), and the solvent was removed *in vacuo* to afford pure Intermediate 8 (0.25 g, 86%) as an off white solid. LC/MS: (Method A): m/z 447 $[M+H]^+$ at 1.67 min.

**Intermediate 9: methyl (S)-4-(1-aminoethyl)-2-methoxybenzoate (Intermediate 9)**

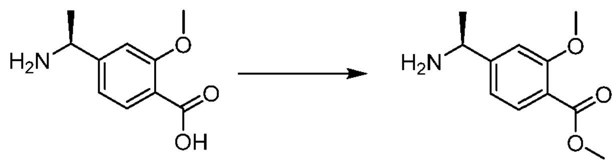

Intermediate 9

(S)-4-(1-aminoethyl)-2-methoxybenzoic acid (2.00 g, 10.25 mmol) was dissolved in methanol (20 mL) at room temperature. HCl, as a 4 N solution in dioxane (10 mL, 40 mmol), was added and the reaction mixture was allowed to stir at 70 °C for 8 hours. The reaction mixture was concentrated *in vacuo* and the resulting residue was purified by triturating with diethyl ether to afford Intermediate 9 (2.1 g, quantitative) as a white solid. LC/MS: (Method A) 0.93 min no mass ion.

**Intermediate 10: methyl 4-((S)-1-((R)-1-(3-bromobenzyl)pyrrolidine-2-carboxamido)ethyl)-2-hydroxybenzoate (Intermediate 10)**

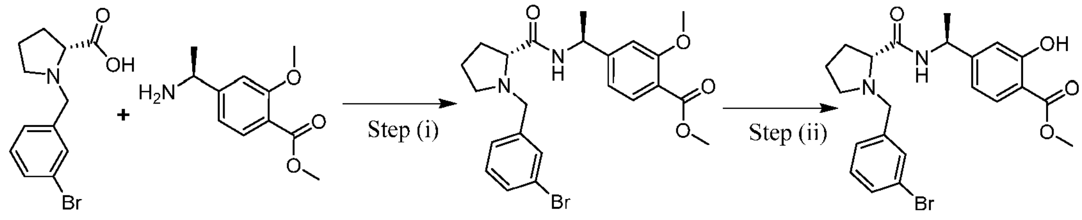


Intermediate 7 Intermediate 9 Intermediate 10

**Step (i):** Intermediate 9 (1.5 g, 7.71 mmol) was dissolved in MeCN (15 mL) and Intermediate 7 (2.23 g, 7.89 mmol) was added to the reaction mixture at room temperature. HATU (4.09 g, 10.75 mmol) was then added and allowed to stir for 30 min. N, N-diisopropylethylamine (2.50 mL, 14.34 mmol) was then added at 0 °C and allowed to stir at room temperature for 2 hours. The reaction mixture was then partition between saturated aqueous $NaHCO_3$ (250 mL) and EtOAc (250 mL). The aqueous layer was further extracted with EtOAc (2 x 150 mL) and the organic layers were combined and dried over $Na_2SO_4$, the solvent was removed *in vacuo,* and the crude product was purified by gradient flash column chromatography (reverse phase followed by normal phase). First, the reverse phase flash column chromatography product eluted at 0% to 68% using MeCN in water as the mobile phase. Second, the normal phase chromatography product eluted using a gradient of 0% to 78% EtOAc in hexane to afford methyl 4-((S)-1-((R)-1-(3-bromobenzyl) pyrrolidine-2-carboxamido)ethyl)-2-methoxybenzoate (1.50 g, 44.11%) as a white solid. LC/MS (Method G): Product was confirmed m/z 475.1 (ES+, M+2), at 1.438 min.

**Step (ii):** Methyl 4-((S)-1-((R)-1-(3-bromobenzyl)pyrrolidine-2-carboxamido)ethyl)-2-methoxy benzoate (1.50 g, 3.16 mmol) was dissolved in DCM (30 mL) and the mixture was cooled to -78°C. $BBr_3$ was added as a 1 M solution in DCM (3.79 mL, 3.79 mmol) and the reaction mixture was stirred at -78°C for 1 hour. The reaction mixture was then partitioned between saturated aqueous solution of $NaHCO_3$ (200 mL) and DCM (250 mL). The aqueous layer was again extracted with DCM (2x150 mL), and the organic layers were combined and dried ($Na_2SO_4$). The solvent was removed *in vacuo* and the crude product was purified by reverse phase gradient flash column chromatography (reverse phase, C18 silica), product eluted at 0% to 65% MeCN in water to afford pure Intermediate 10 (0.99 g, 68%) as a white solid. LC/MS: (Method G): m/z 461 $[M+H]^+$ at 5.43 min.

**Intermediate 11: 3'-(bromomethyl)-5'-hydroxy-2-methyl-[1,1'-biphenyl]-4-carboxamide (Intermediate 11)**

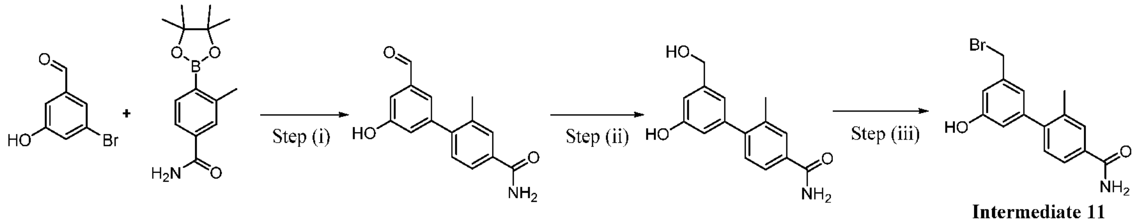


Intermediate 11

**Step (i):** 3-bromo-5-hydroxybenzaldehyde (4.00 g, 20.00 mmol), 3-methyl-4-(4,4,5,5-tetramethyl-1,3,2-dioxaborolan-2-yl)benzamide (5.74 g, 22.00 mmol), and $K_2CO_3$ (3.20 g, 60.01 mmol) were dissolved in 1,4-dioxane (15 mL) and water (15 mL). Nitrogen gas was then purged through the mixture for 20 minutes at RT, follow by the addition of $PdCl_2$(dppf) DCM (1.63 g, 2.00 mmol). The reaction mixture was then allowed to stir at 80 °C for 2 hours. The reaction mixture was then partitioned between water (400 mL) and EtOAc (250 mL), and the aqueous layer was further extracted with EtOAc (2 x 100 mL). the organic layers were combined and dried ($Na_2SO_4$), the solvent was removed *in vacuo,* and the crude product was purified by reverse phase gradient flash column chromatography (reverse phase, C18 Silica), and the product was eluted at 0% to 35% MeCN in water to afford 3'-formyl-5'-hydroxy-2-methyl-[1,1'-biphenyl]-4-carboxamide (3.60 g, 71%) as a brown solid. LC/MS: (Method A): m/z 256 $[M+H]^+$, at 1.09 min.

**Step (ii):** 3'-formyl-5'-hydroxy-2-methyl-[1,1'-biphenyl]-4-carboxamide (1.50 g, 5.88 mmol) was dissolved in methanol (15 mL) at room temperature. The reaction mixture was cooled to 0 °C, sodium borohydride (0.44 g, 11.76 mmol)

was added at 0 °C, and then allowed to stir at room temperature for 2 hours. The reaction mixture was partitioned between saturated aqueous $NaHCO_3$ (100 mL) and EtOAc (80 mL), and the aqueous layer was further extracted with EtOAc (3 x 50 mL). The organic layers were combined and dried ($Na_2SO_4$), and the solvent was removed *in vacuo* to afford 3'-hydroxy-5'-(hydroxymethyl)-2-methyl-[1,1'-biphenyl]-4-carboxamide (1.36 g, 90%) as an off-white solid. LC/MS: (Method A): m/z 258 $[M+H]^+$, at 0.94 min.

**Step (iii):** 3'-hydroxy-5'-(hydroxymethyl)-2-methyl-[1,1'-biphenyl]-4-carboxamide (1.36 g, 5.29 mmol) was dissolved in 33% HBr in $CH_3COOH$ (12 mL) at room temperature, and the resulting reaction mixture was allowed to stir at room temperature for 16 hours. The reaction mixture was then partitioned between saturated aqueous $NaHCO_3$ solution (200 mL) and EtOAc (150 mL), and the aqueous layer was further extracted with EtOAc (2 x 50 mL). The organic layers were combined and dried ($Na_2SO_4$), and the solvent was removed *in vacuo* to afford 3'-(bromomethyl)-5'-hydroxy-2-methyl-[1,1'-biphenyl]-4-carboxamide (1.59 g, 94%) as a reddish-brown solid. LC/MS: (Method A): m/z 320 $[M+H]^+$, at 1.23 min.

**Intermediate 12: 3'-(bromomethyl)-2-methyl-[1,1'-biphenyl]-4-sulfonamide (Intermediate 12)**

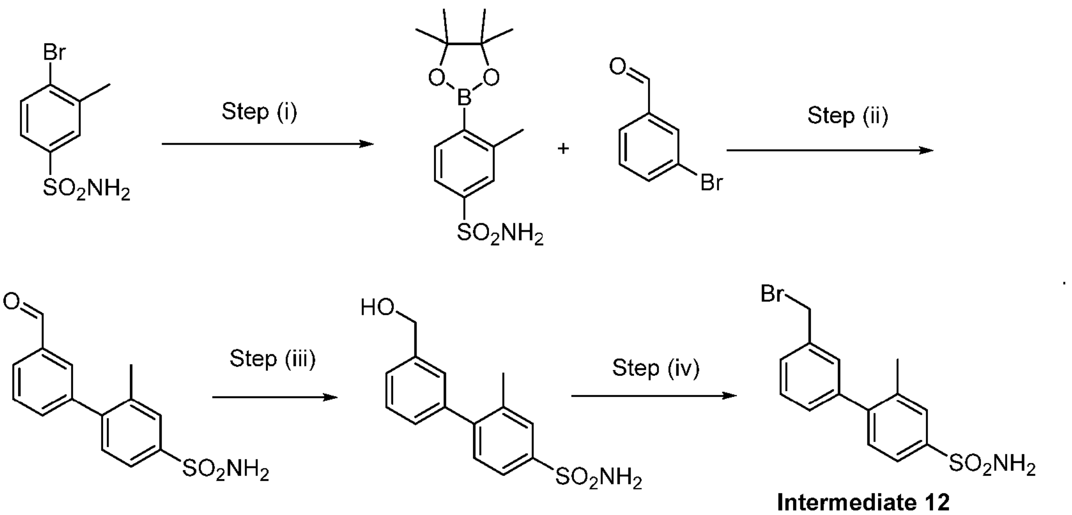


**Intermediate 12**

**Step (i)** : 4-bromo-3-methyl-benzenesulphonamide (1.00g, 3.99mmole), Bis(pincaolato)diborone (2.02g, 7.98mmol) and KOAc (1.17g, 11.97mmol) were dissolved in Dioxane (10mL) at room temperature. To it, nitrogen gas was purged for 30min at room temperature. After this, $PdCl_2$(dppf)DCM (0.325g, 0.399 mmole) was added and reaction mixture was allowed to stir at 80°C for 2h. The reaction mixture was then partitioned between water (100mL) and EtOAc (100mL), aqueous layer was further extracted with EtOAc (2 x 100mL). Organic layers were combined and dried ($Na_2SO_4$). Solvent was removed in vacuo and the crude product was purified by gradient flash column chromatography (normal phase, Silica) product eluted at 0% to 30% EtOAc in hexane to afford pure 3-methyl-4-(4,4,5,5-tetramethyl-1,3,2-dioxaborolan-2-yl)benzenesulfonamide (1.0g, 85%) as yellow solid. LC/MS: (Method F): Product's m/z was not supported in the major peak (ES+), at 0.46 min.

**Step (ii):** 3-bromobenzaldehyde (4.50g, 24.46mmole), 3-methyl-4-(4,4,5,5-tetramethyl-1,3,2-dioxaborolan-2-yl)benzenesulfonamide (8.72g, 29.35mmole) and $K_2CO_3$ (10.20g, 73.38mmole) were dissolved in 1,4-dioxane (18mL) and water (12mL). Nitrogen gas was purged for 30min at room temperature. Then, $PdCl_2$(dppf)DCM (1.99g, 2.44mmole) was added and the reaction mixture was allowed to stir at 80°C for 2h. The reaction mixture was partitioned between water (500mL) and EtOAc (150mL). Aqueous layer was further extracted with EtOAc (2 x 150mL). Organic layers were combined and dried ($Na_2SO_4$). Solvent was removed in vacuo and the crude product was purified by gradient column chromatography (Normal phase, silica), product eluted at 0% to 40% EtOAc in Hexane to afford pure 3'-formyl-2-methyl-[1,1'-biphenyl]-4-sulfonamide (4.5g, 67%) as an off-white solid. LC/MS: (Method A): Desired mass was not supported in major peak at 1.74 min.

**Step (iii):** 3'-formyl-2-methyl-[1,1'-biphenyl]-4-sulfonamide (4.00g, 14.54mmole) was dissolved in methanol (30mL) at room temperature. The reaction mixture was cooled to 0 °C and sodium borohydride (1.65g, 43.62mmole) was added in portions. Reaction mixture was allowed to stir at room temperature for 1h. The reaction mixture was diluted with cold water (50mL). Solid was precipitated out which was filtered and dried in vacuo to afford 3'-(hydroxymethyl)-2-methyl-[1,1'-biphenyl]-4-sulfonamide (3.6g, 79%) as off white solid. LC/MS: (Method A): 260. $[M+H-H_2O]^+$, at 1.51min.

**Step (iv):** 3'-(hydroxymethyl)-2-methyl-[1,1'-biphenyl]-4-sulfonamide (3.60g, 12.99mmole) was dissolved in

HBr (64% in water) (35mL) and the reaction mixture was allowed to stir at room temperature for 2h. The reaction mixture was diluted with cold water (50mL). Solid was precipitated out which was filtered and dried in vacuo to afford 3'-(bromomethyl)-2-methyl-[1,1'-biphenyl]-4-sulfonamide (3.7g, 84%) as yellow solid. LC/MS: (Method A): Mass was not supported at 1.51min.

**Intermediate 13: 3'-(bromomethyl)-2-methyl-[1,1'-biphenyl]-4-carboxamide (Intermediate 13)**

Intermediate 13 was synthesized by a process analogous to Intermediate 12 using 4-bromo-3-methylbenzamide to give Intermediate 13 LC/MS: (Method A): m/z 305 $[M+H]^+$, at 2.02min.

## Intermediate 14

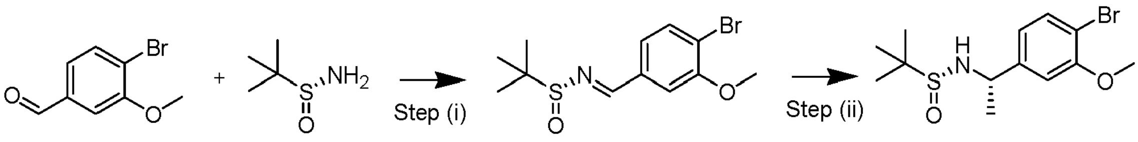


a1 a2 a3 a4

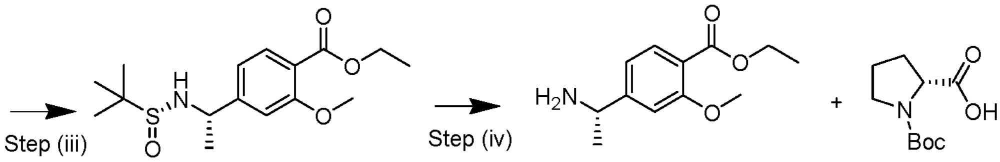


a5 a6 a7

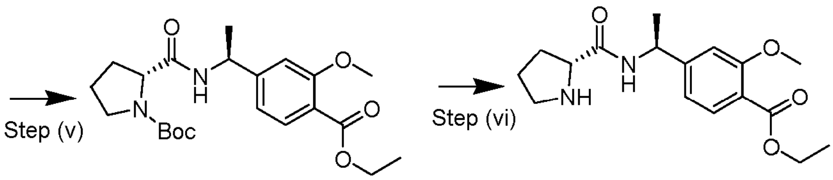


a8 Intermediate 14

**Step** (i): To a solution of compound a1 (2.4 g, 11.16 mmol, 1 eq), compound a2 (1.62 g, 13.39 mmol, 1.2 eq) in THF (50 mL) was added $Ti(OEt)_4$ (5.09 g, 22.32 mmol, 4.63 mL, 2 eq). The mixture was stirred at 30 °C for 5 hr. TLC (PE: EA = 5: 1) indicated compound a1 was consumed completely and one new spot formed. The reaction mixture was diluted with $H_2O$ (50 mL) and extracted with EA (100 mL * 2). The combined organic layers were dried over anhydrous sodium sulfate, filtered and concentrated under reduced pressure to give residue. The residue was purified by flash silica gel chromatography (ISCO®; 20 g SepaFlash® Silica Flash Column, Eluent of 0~30% Ethylacetate/ Petroleum ether gradient @ 50 mL/min) to give the compound a3 (3.5 g, 11.00 mmol, 98.55% yield) as a yellow oil, which was confirmed by 1H NMR.

$^1$H NMR ($CDCl_3$, 400 MHz) δ 8.54 (s, 1 H), 7.67 (d, J = 8.0 Hz, 1 H), 7.40 (d, J = 1.6 Hz, 1 H), 7.30 (dd, J = 1.6, 8.0 Hz, 1 H), 3.98 (s, 3 H), 1.28 (s, 9 H).

**Step (ii):** DIMETHYLZINC (1 M, 13.36 mL, 1.7 eq) was dissolved with THF (50 mL), and MeMgBr (3 M, 3.93 mL, 1.5 eq) was added dropwise at 10°C, and then the mixture was stirred at 10°C for 30 min. Then the reaction solution was cooled to 0 °C, and a solution of compound a3 (2.50 g, 7.86 mmol, 1 eq) in THF (12.5 mL) was added dropwise, and then the reaction mixture was stirred at 10°C for 1hr. TLC (PE: EA = 1: 1) indicated compound a3 was consumed completely. The reaction mixture was diluted with $H_2O$ (30 mL) and extracted with EA (50 mL * 2). The combined organic layers were dried over anhydrous sodium sulfate, filtered and concentrated under reduced pressure to give residue. The residue was purified by flash silica gel chromatography (ISCO®; 20 g SepaFlash® Silica Flash Column, Eluent of 30~60% Ethylacetate/ Petroleum ether gradient @ 50 mL/min) to give the compound a4 (1.2 g, 3.52 mmol, 44.83% yield, 98.1% purity) as a white solid, which was confirmed by 1HNMR.

SFC: Rt = 1.220 min, ee: 97.1%

$^1$H NMR ($CDCl_3$, 400 MHz) δ 7.49 (d, J = 8.4 Hz, 1 H), 6.90 (s, 1 H), 6.82 (d, J = 8.0 Hz, 1 H), 4.55 (dd, J = 2.0, 6.4 Hz, 1 H), 3.90 (s, 3 H), 3.32 (s, 1 H), 1.52 (d, J = 6.8 Hz, 3 H), 1.22 (s, 9 H).

**Step (iii):** To a solution of compound a4 (1.4 g, 4.19 mmol, 1 eq) in EtOH (40 mL) was added TEA (1.27 g, 12.56

mmol, 1.75 mL, 3 eq) and Pd(dppf)$Cl_2$.$CH_2Cl_2$ (342.03 mg, 418.82 umol, 0.1 eq). The mixture was stirred at 80 °C for 16 hr under CO (50PSI). LC-MS showed compound a4 was consumed completely and one main peak with desired mass was detected. The reaction mixture was filtered and the filtrate was concentrated under reduced pressure to give residue. The residue was purified by column chromatography ($SiO_2$, Petroleum ether/ Ethyl acetate = 3/ 1 to 1/ 1) to give the compound a5 (1.35 g, 4.09 mmol, 97.65% yield, 99.2% purity) as a yellow oil, which was confirmed by HNMR.

LCMS: $[M+1]^+$ = 328.4.

SFC: Rt = 0.901 min, ee = 97.9%.

$^1$H NMR ($CDCl_3$, 400 MHz) δ 7.77 (d, J = 7.6 Hz, 1 H), 7.02 - 6.90 (m, 2 H), 4.63 - 4.57 (m, 1 H), 4.36 (q, J = 7.2 Hz, 2 H), 3.91 (s, 3 H), 3.33 (d, J = 1.6 Hz, 1 H), 1.54 (d, J = 6.8 Hz, 3 H), 1.38 (t, J = 7.2 Hz, 3 H), 1.23 (s, 9 H).

**Step (iv):** To a solution of compound a5 (1.35 g, 4.12 mmol, 1 eq) in DCM (15 mL) was added HCl/dioxane (4 M, 20 mL) at 0°C. The mixture was stirred at 15°C for 12hr. LC-MS showed compound a5 was consumed completely. The reaction mixture was concentrated under reduced pressure to give residue. The residue was triturated with PE: EA = 5: 1 (10 mL) at 15 °C for 10 min to give the compound a6 (920 mg, 3.54 mmol, 85.91% yield, HCl) as yellow solid, which was confirmed by HNMR.

$^1$H NMR (DMSO-$d_6$, 400 MHz) δ 8.67 (s, 3 H), 7.64 (d, J = 8.0 Hz, 1 H), 7.44 (s, 1 H), 7.13 (d, J = 8.0 Hz, 1 H), 4.50 - 4.36 (m, 1 H), 4.24 (q, J = 6.8 Hz, 2 H), 3.85 (s, 3 H), 1.52 (d, J = 6.4 Hz, 3 H), 1.27 (t, J = 7.2 Hz, 3 H).

**Step (v):** To a solution of compound a7 (795.58 mg, 3.70 mmol, 1.5 eq) in DMF (8 mL) was added HATU (1.87 g, 4.93 mmol, 2 eq) and DIEA (1.59 g, 12.32 mmol, 2.15 mL, 5 eq), the mixture was stirred at 25°C for 0.5hr. Compound a6 (640 mg, 2.46 mmol, 1 eq, HCl) was added and the resulting mixture was stirred at 25°C for 1hr. LC-MS showed compound a6 was consumed completely and one main peak with desired mass was detected. The reaction mixture was diluted with $H_2O$ (30 mL) and extracted with EA (50 mL * 2). The combined organic layers were dried over anhydrous sodium sulfate, filtered and concentrated under reduced pressure to give residue. The residue was purified by flash silica gel chromatography (ISCO®; 20 g SepaFlash® Silica Flash Column, Eluent of 0~50% Ethyl acetate/ Petroleum ether gradient @ 40 mL/min) to give the compound a8 (1.26 g, crude) as yellow oil, which was confirmed by HNMR.

LCMS: $[M-Boc]^+$ = 321.5

SFC: Rt = 0.926 min, ee: 100%

$^1$H NMR (DMSO-$d_6$, 400 MHz) δ 8.39 - 8.23 (m, 1 H), 7.56 (d, J = 7.6 Hz, 1 H), 7.12 (s, 1 H), 6.97 (d, J = 7.6 Hz, 1 H), 5.04 - 4.81 (m, 1 H), 4.23 (q, J = 7.2 Hz, 2 H), 4.18 - 4.05 (m, 1 H), 3.88 - 3.78 (m, 3 H), 3.49 - 3.35 (m, 1 H), 3.29 - 3.22 (m, 1 H), 2.22 - 2.03 (m, 1 H), 1.95 - 1.71 (m, 3 H), 1.47 - 1.29 (m, 8 H), 1.24 - 1.11 (m, 7 H).

**Step (vi):** To a solution of compound a8 (1.2 g, 2.85 mmol, 1 eq) in DCM (10 mL) was added HCl/dioxane (4 M, 10 mL). The mixture was stirred at 15 °C for 1 hr. LC-MS showed compound a8 was consumed completely and one main peak with desired mass was detected. The reaction mixture was concentrated under reduced pressure to give Intermediate 14 (830 mg, crude, HCl) as a yellow oil.

LCMS: $[M+1]^+$ = 321.2

## Intermediate 15

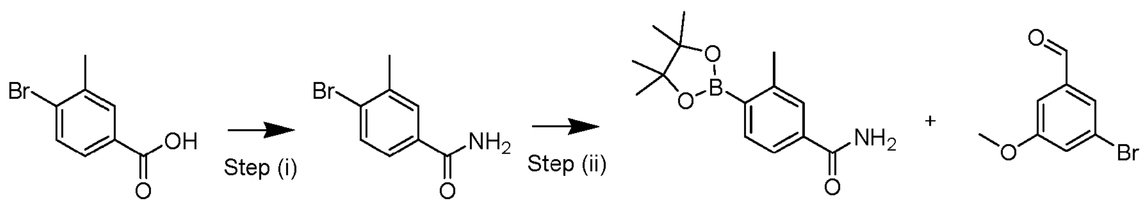


**b1** **b2** **b3** **b4**

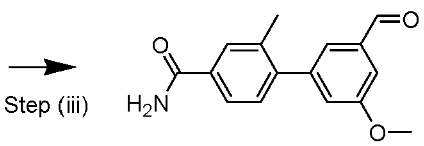


**Intermediate 15**

**Step (i):** A mixture of compound b1 (1 g, 4.65 mmol, 1 *eq)*, $NH_4Cl$ (373.12 mg, 6.98 mmol, 1.5 *eq)*, HATU (2.65 g, 6.98 mmol, 1.5 *eq)* and DIEA (1.20 g, 9.30 mmol, 1.62 mL, 2 *eq)* in DMF (25 mL) was degassed and purged with $N_2$ for 3 times, and then the mixture was stirred at 30 °C for 12 h under $N_2$ atmosphere. LCMS indicated the compound b1 was consumed completely. The mixture was slowly dropwise into the water (35 mL). The precipitate was filtered and the filter cake was concentrated to give the compound b2 (600 mg, 2.80 mmol, 60.28% yield) as a brown solid, which was confirmed

by HNMR.

LCMS: m/z= 213.7 [M+1].

$^1$H NMR (DMSO-$d_6$, 400 MHz) δ 8.00 (s, 1 H), 7.85 (d, *J* = 1.6 Hz, 1 H), 7.67 - 7.65 (m, 1 H), 7.62 - 7.59 (m, 1 H), 7.42 (s, 1 H), 2.38 (s, 3 H).

**Step (ii):** A mixture of compound b2 (450 mg, 2.10 mmol, 1 eq), 4,4,5,5-tetramethyl-2-(4,4,5,5- tetramethyl-1,3,2-dioxaborolan-2-yl)-1,3,2-dioxaborolane (640.60 mg, 2.52 mmol, *1.2 eq*), AcOK (412.63 mg, 4.20 mmol, 2 *eq*), Pd(dppf)$Cl_2$ (153.82 mg, 210.22 umol, 0.1 *eq)* in dioxane (12 mL) was degassed and purged with $N_2$ for 3 times, and then the mixture was stirred at 85 °C for 12 h under $N_2$ atmosphere. LCMS indicated the compound b2 was consumed completely, and one main peak with desired mass was detected. TLC (Petroleum ether: Ethyl acetate = 1: 1) indicated the compound b2 was consumed completely. The reaction mixture was concentrated under reduced pressure to give a residue. The residue was purified by flash silica gel chromatography (ISCO®; 20 g SepaFlash® Silica Flash Column, Eluent of 0~65% Ethyl acetate/Petroleum ether gradient @ 45 mL/min) to give the compound b3 (440 mg, 1.69 mmol, 80.15% yield) as a white solid, which was confirmed by HNMR.

LCMS: m/z= 261.9[M+1].

$^1$H NMR (CDCl$_3$, 400 MHz) δ 7.82 (d, *J* = 7.6 Hz, 1H), 7.65 - 7.54 (m, 2H), 6.22 - 5.84(m, 2 H), 2.58 (s, 3H), 1.36 (s, 12 H).

**Step (iii):** To a solution of compound b3 (1 g, 3.83 mmol, 1 eq), compound b4 (1.07 g, 4.98 mmol, 1.3 *eq*) in dioxane (30 mL) and $H_2O$ (3 mL) was added $K_2CO_3$ (1.06 g, 7.66 mmol, 2 *eq*) and Pd(dppf)$Cl_2$ (280.21 mg, 382.96 umol, 0.1 *eq).* The mixture was stirred at 80 °C for 12 hr under $N_2$. TLC (PE: EA = 1: 1) indicated compound b3 was consumed completely and one new spot formed. The reaction mixture was diluted with $H_2O$ (30 mL) and extracted with EA (50 mL * 2). The combined organic layers were dried over anhydrous sodium sulfate, filtered and concentrated under reduced pressure to give residue. The residue was purified by column chromatography ($SiO_2$, Petroleum ether/Ethyl acetate = 3/ 1 to 1/ 2) to give Intermediate 15 (550 mg, 2.04 mmol, 53.33% yield) as a yellow solid, which was confirmed by HNMR.

$^1$H NMR (CDCl$_3$, 400 MHz) δ 10.03 (s, 1 H), 7.80 (s, 1 H), 7.69 (d, *J* = 4.4 Hz, 1 H), 7.42 (s, 2 H), 7.33 (d, *J* = 4.8 Hz, 1 H), 7.13 (s, 1 H), 6.55 - 5.32 (m, 2 H), 3.92 (s, 3 H), 2.34 (s, 3 H).

**Intermediate 16: (S)-1-(4-bromo-3-methoxyphenyl) ethan-1-amine (Intermediate 16)**

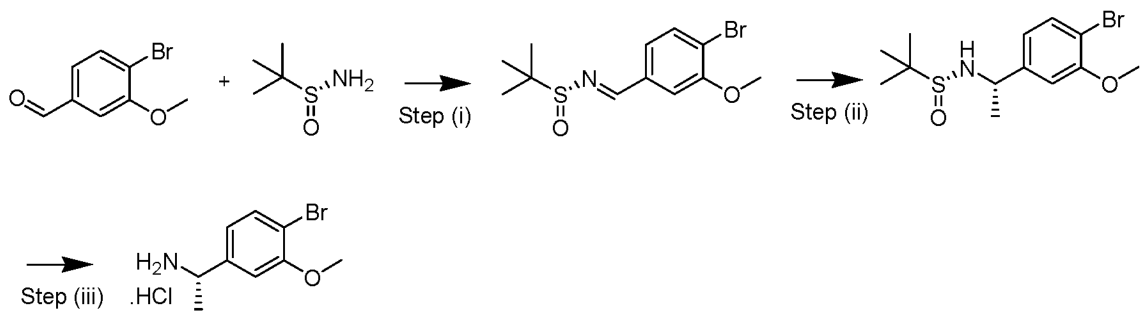


**Intermediate 16**

**Step (i):** To a solution of 4-bromo-3-methoxybenzaldehyde (31.16 g, 0.14 mol, 1 eq) were dissolved in THF (240 mL) at RT, then Ti(OEt)$_4$ (66.10 g, 0.28 mol, 61.20 mL, 2 eq) was added at RT to a reaction mixture and the reaction mixture was stirred at RT for 15 minutes. (R)-2-methylpropane-2-sulfinamide (28.09 g, 0.23 mol, 1.6 eq) pre-dissolved in THF (60 mL) was added to a reaction mixture and the mixture was stirred at 30 °C for overnight. TLC (EA: Hexane = 2:8) indicated 4-bromo-3-methoxybenzaldehyde was consumed completely and one new spot formed. The reaction mixture was diluted with $H_2O$ (300 mL), but salt was followed and filter through vacuum pump and filtrate was extracted with EA (3×500 mL). The combined organic layers were dried over anhydrous sodium sulphate, filtered and concentrated under reduced pressure to give residue. The residue was purified by flash silica gel chromatography (Normal-Phase silica at 0 to 15.0% EA/Hexane) to give the (R, E)-N-(4-bromo-3-methoxybenzylidene)-2-methylpropane-2-sulfinamide (35.9 g, 77.85% yield) as a white solid.

TLC: (2.0:8.0, EtOAc/Hexane, RF: 0.40).

Mass (ESI +ve): 318.0 [M].

LCMS: 100.0 % (LCMS Method H), RT: 3.545 mins, 280.0 nm.

$^1$H NMR (400 MHz, CDCl3) δ: 8.57 (s, 1 H), 7.69-7.71 (d, 1 H), 7.44 (d, 1 H), 7.30-7.33 (dd, 1 H), 4.01 (s, 3 H), 1.31 (s, 9 H).

**Step (ii):** Dimethyl zinc (1.5 M in toluene, 269.66 mL, 3.7 *eq)* was dissolved with THF (150 mL), and MeMgBr (3.0

M in Diethyl ether, 116.6 mL, 3.2 *eq*) was added dropwise at 15-20°C, and then the mixture was stirred at 15-20°C for 45 min. Then the reaction solution was cooled to 0 °C, and a solution of (R, E)-N-(4-bromo-3-methoxybenzylidene)-2-methylpropane-2-sulfinamide (34.79 g, 0.034 mol, 1 *eq*) pre-dissolved in THF (150 mL) was added dropwise, and then the reaction mixture was stirred at 0°C for 30min. TLC (EA: Hexane = 5:5) indicated (R, E)-N-(4-bromo-3-methoxybenzylidene)-2-methylpropane-2-sulfinamide was consumed completely. The reaction mixture was diluted with cold $H_2O$ (1000 mL) and solid was followed, then acidify with 1N HCl (Ph=2 to3) and extracted with EA (800 mL × 3). The combined organic layers were dried over anhydrous sodium sulfate, filtered and concentrated under reduced pressure to give residue. The residue was purified by flash silica gel chromatography (Normal-Phase silica at 0 to 45.0% EA/Hexane) to give (R)-N-((S)-1-(4-bromo-3-methoxyphenyl)ethyl)-2-methylpropane-2-sulfinamide (25.57 g, 69.97% yield, 97.20% purity) as an off-white solid.

TLC: (5.0:5.0, EtOAc/Hexane, RF: 0.25).

Mass (ESI +ve): 335.8 [M+1].

LCMS: 100.0 % (LCMS Method H), RT: 3.067 mins, 254.0 nm.

$^1H$ NMR (400 MHz, CDCl3) δ: 7.50-7.52 (s, 1 H), 7.01 (s, 1 H), 6.82-6.84 (dd, 1 H), 4.77-4.78 (s, 1H), 4.42 (s, 1H), 3.93 (s, 3H), 1.58-1.62 (t, 6 H), 1.29 (s, 9 H).

**Step (iii):** To a solution of (R)-N-((S)-1-(4-bromo-3-methoxyphenyl)ethyl)-2-methylpropane-2-sulfinamide (25.57 g, 0.07 mol, 1 *eq)* in Dioxane (20 mL) was added HCl in dioxane (4.0 M, 125 mL) at 0°C. The mixture was stirred at RT for 3hr. TLC (EA: Hexane = 5:5) indicated (R)-N-((S)-1-(4-bromo-3-methoxyphenyl)ethyl)-2-methylpropane-2-sulfinamide was consumed completely. The reaction mixture was concentrated under reduced pressure to give residue. The residue was wash with dioxane (40 mL) and concentrated under reduced pressure to give residue to give (S)-1-(4-bromo-3-methoxyphenyl) ethan-1-amine (Intermediate 16) (18.73 g, HCl salt) as a white solid.

TLC: (5.0:5.0, EtOAc/Hexane, RF: 0.25).

LCMS: 100.0 % (LCMS Method H), RT: 2.371 mins, 230.0 nm.

$^1H$ NMR (400 MHz, DMSO-D2O) δ: 8.75 (s, 3H), 7.59-7.68 (d, 1 H), 7.49 (s, 1 H), 7.02-7.04 (d, 1 H), 4.36 - 4.39 (s, 1 H), 3.88 (s, 3 H), 3.56 (s, 1 H), 1.51-152 (t, 3 H).

**Intermediate 17: 4'-hydroxy-5-methoxy-2'-methyl-[1,1'-biphenyl]-3-carbaldehyde (Intermediate 17)**

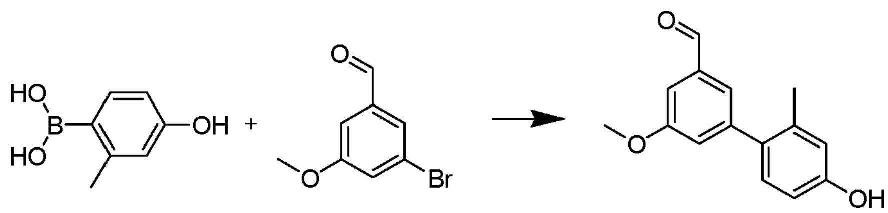

**Intermediate 17**

To a solution of (4-hydroxy-2-methylphenyl) boronic acid (3.5 g, 0.02 mol, 1 *eq*), 3-bromo-5-methoxybenzaldehyde (4.95 g, 0.020 mol, 1.0 *eq*) in dioxane (35 mL) and $H_2O$ (11 mL) was added $K_2CO_3$ (6.35 g, 0.046 mol, 2 eq) mixture was d-gassed for 10 min., after d-gassed added Pd(dppf)$Cl_2$ (1.6 g, 0.002 mol, 0.1 *eq*) to a reaction mixture. Then reaction mixture was stirred at 80°C for overnight. TLC (EA: Hexane = 5:5) indicated (4-hydroxy-2-methylphenyl) boronic acid was consumed completely and one new spot formed. The reaction mixture was diluted with $H_2O$ (300 mL) and extracted with EA (3×150 mL). The combined organic layers were dried over anhydrous sodium sulphate, filtered and concentrated under reduced pressure to give residue. The residue was purified by column chromatography (Normal-Phase silica at 0 to 46.0% EA/Hexane) to give 4'-hydroxy-5-methoxy-2'-methyl-[1,1'-biphenyl]-3-carbaldehyde (Intermediate 17) (4.0 g, 71.68% yield) as a brown solid.

TLC: (5.0:5.0, EtOAc/Hexane, RF: 0.25).

Mass (ESI +ve): 243.0 [M+1].

LCMS: 95.66 % (LCMS Method H), RT: 3.029 mins, 254.0 nm.

$^1H$ NMR (400 MHz, DMSO-D2O) δ: 10.01 (s, 1H), 9.48 (s, 1H), 7.43-7.36 (d, 2H), 7.16 (s, 1H), 7.08-7.06 (d, 1H), 6.67-6.71 (m, 2H), 3.86 (s, 3 H), 2.18 (s, 3 H).

<u>**Synthetic Preparation of Compounds of Formula I**</u>

**Example 1: 4-((1S)-1-((2R)-1-((4'-hydroxy-2'-methyl-1',2',3',4',5',6'-hexahydro-[1,1'-biphenyl]-3-yl)methyl)pyrrolidine-2-carboxamido)ethyl)benzoic acid (Compound 1)**

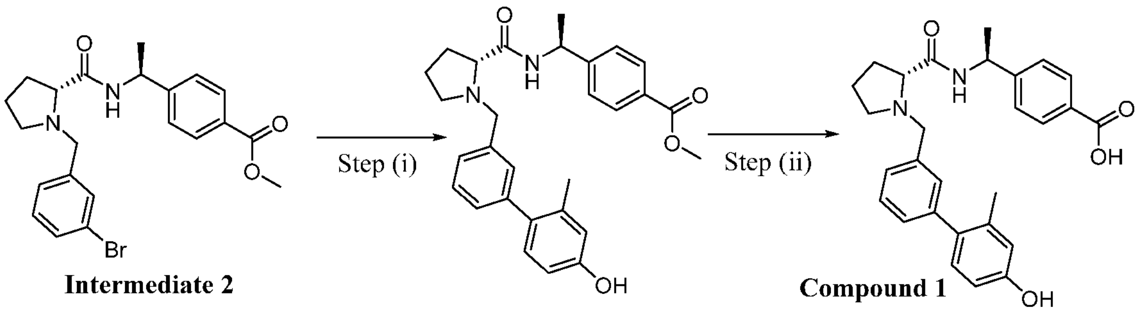


Intermediate 2

Compound 1

**Step (i):** A mixture of Intermediate 2 (150. Mg, 0.3400 mmol), (4-hydroxy-2-methylphenyl)boronic acid (56.3 mg, 0.3700 mmol), potassium carbonate (0.09 g, 0.67 mmol) and tetrakis(triphenylphosphine) palladium (0) (11.68 mg, 0.01 mmol), in 1,4-dioxane (1.4 mL) and water (0.3 mL) was heated to 100 °C for 1 hour in a microwave reactor. A further addition of (4-hydroxy-2-methylphenyl)boronic acid (56.3 mg, 0.3700 mmol) was made, and the mixture was placed in the microwave reactor for a further 1 hour. The crude product was diluted with water and EtOAc and the organic layers were separated, washed with water, brine and dried by passing through a hydrophobic frit before being concentrated in vacuo. The residue was purified by flash column chromatography (normal phase, 10 g, Biotage® SNAP KP-Sil, 30 mL per min, gradient 30% to 100% ethyl acetate in isohexane) to give methyl 4-[(1S)-1-[[(2R)-1-[[3-(4-hydroxy-2-methyl-phenyl) phenyl]methyl] pyrrolidine-2-carbonyl]amino]ethyl]benzoate (148 mg, 93%), as a pale yellow gum. LC/MS (Method B) 2.36 min $[M+H]^+$ 473.

**Step (ii):** To a solution of methyl 4-[(1S)-1-[[(2R)-1-[[3-(4-hydroxy-2-methylphenyl)phenyl]methyl]pyrrolidine-2-carbonyl]amino]ethyl]benzoate (148. Mg, 0.31 mmol) in 1,4-dioxane (2.62 mL) and water (2.62 mL), was added lithium hydroxide monohydrate (59.13 mg, 1.41 mmol). The reaction was left to stir at RT for 3 hours before being checked for completion by LC/MS and then concentrated in vacuo. The crude residue was purified by reversed phase HPLC (HPLC method A), to give Compound 1 (52.5 mg, 36%), as a white solid. LC/MS (Method C): m/z 459 $[M+H]^+$ 2.30 min. $^1$H NMR (400 MHz, DMSO-d6) δ 7.91 (m, 1H), 7.74 (m, 2H), 7.33 (dd, J = 7.5 Hz, 1H), 7.27 - 7.21 (m, 2H), 7.18 - 7.08 (m, 3H), 6.98 (m, 1H), 6.68 - 6.61 (m, 2H), 4.85 - 4.76 (m, 1H), 3.80 (d, J = 12.9 Hz, 1H), 3.52 (d, J = 12.9 Hz, 1H), 3.08 (dd, J = 9.5, 4.1 Hz, 1H), 2.99 - 2.93 (m, 1H), 2.39 - 2.29 (m, 1H), 2.13 (s, 3H), 2.09 - 1.97 (m, 2H), 1.72 - 1.61 (m, 3H), 1.24 (d, *J* = 7.0 Hz, 3H). Exchangeable acid proton not observed in spectra.

**Example 2: 4-((S)-1-((R)-1-((2'-methyl-4'-sulfamoyl-[1,1'-biphenyl]-3-yl)methyl)pyrrolidine-2-carboxamido) ethyl)benzoic acid (Compound 2)**

Compound 2 was synthesized by a process analogous to Example 1, using Intermediate 2 and (2-Methyl-4-sulfamoylphenyl)boronic acid, wherein only one boronic acid addition is required in Step (i) of the process. The crude product was purified by prep. HPLC (HPLC method A) to provide Compound 2 (48.5 mg, 0.09 mmol, 34%) as a white foam. LC/MS (Method C): *m/z* 522 $[M+H]^+$ 2.28 min. $^1$H NMR (400 MHz, DMSO-$d_6$) δ 7.94 (d, *J* = 8.0 Hz, 1H), 7.71 (s, 1H), 7.67 (m, 3H), 7.47 (br s, 2H), 7.39 (dd, *J* = 7.6 Hz, 1H), 7.36 - 7.27 (m, 3H), 7.23 (m, 1H), 7.17 (m, 2H), 4.83 - 4.74 (m, 1H), 3.84 (d, *J* = 13.2 Hz, 1H), 3.52 (d, *J* = *13.1* Hz, 1H), 3.08 (dd, *J* = 7.8 Hz, 1H), 2.95 - 2.88 (m, 1H), 2.35 - 2.26 (m, 1H), 2.23 (s, 3H), 2.07 - 1.96 (m, 1H), 1.71 - 1.57 (m, 3H), 1.24 (d, *J* = 6.9 Hz, 3H). Exchangeable acid proton not observed in spectra.

**Example 3: 4-((S)-1-((R)-1-((4'-carbamoyl-2'-methyl-[1,1'-biphenyl]-3-yl)methyl)pyrrolidine-2-carboxamido) ethyl)benzoic acid (Compound 3)**

Compound 3 was synthesized by a process analogous to Example 1, using Intermediate 2 and 3-Methyl-4-(4,4,5,5-tetramethyl-1,3,2-dioxaborolan-2-yl)benzamide, wherein only one boronic ester addition is required in Step (i) of the process. The crude product was purified by HPLC (HPLC method C) to give Compound 3 (71 mg, 0.15 mmol, 43%) as a white foam. LC/MS (Method C): *m/z* 486 $[M+H]^+$ 2.36 min. $^1$H NMR (400 MHz, DMSO-$d_6$) δ 8.06 - 7.96 (m, 2H), 7.80 - 7.74 (m, 3H), 7.72 (m, 1H), 7.38 (m, 1H), 7.33 - 7.18 (m, 7H), 4.85 - 4.76 (m, 1H), 3.82 (d, *J* = 13.0 Hz, 1H), 3.52 (d, *J* = 13.0 Hz, 1H), 3.07 (dd, *J* = 9.8, 4.1 Hz, 1H), 2.98 - 2.90 (m, 1H), 2.35 - 2.27 (m, 1H), 2.21 (s, 3H), 2.06 - 1.96 (m, 1H), 1.73 - 1.59 (m, 3H), 1.25 (d, *J* = 7.0 Hz, 3H). Exchangeable acid proton not observed in spectra

**Example 4: 4-((S)-1-((2R,4R)-4-hydroxy-1-((2'-methyl-4'-sulfamoyl-[1,1'-biphenyl]-3-yl)methyl)pyrrolidine-2-carboxamido)ethyl)benzoic acid (Compound 4)**

Compound 4 was synthesized by a process analogous to Example 1, using Intermediate 4 and (2-Methyl-4-sulfamoylphenyl)boronic acid, wherein only one boronic ester addition is required in Step (i) of the process. The crude residue was purified by HPLC (HPLC method C) to give Compound 4 (73.1 mg, 0.14 mmol, 60 %) as a white foam. LC/MS (Method C): *m/z* 538 $[M+H]^+$ 2.12 min. $^1H$ NMR (400 MHz, DMSO-$d_6$) δ 7.86 (d, *J* = 8.0 Hz, 2H), 7.81 (s, 1H), 7.75 (m, 1H), 7.46 - 7.34 (m, 4H), 7.32 (d, *J* = 8.0 Hz, 2H), 7.25 (m, 1H), 4.75-5.00 (m, 1H), 4.32 - 4.25 (m, 1H), 3.87 (d, *J* = 12.9 Hz, 1H), 3.69 (d, *J* = 12.9 Hz, 1H), 3.23 (dd, *J* = 10.6, 4.6 Hz, 1H), 3.10 (d, *J* = 10.3 Hz, 1H), 2.65 - 2.58 (m, 1H), 2.53 - 2.43 (m, 1H), 2.31 (s, 3H), 1.82 - 1.74 (m, 1H), 1.31 (d, *J* = 7.0 Hz, 3H). Exchangeable protons not observed in spectra.

**Example 5: 4-((S)-1-((2R,4R)-4-hydroxy-1-((4'-hydroxy-2'-methyl-[1,1'-biphenyl]-3-yl)methyl)pyrrolidine-2-carboxamido)ethyl)benzoic acid (Compound 5)**

Compound 5 was synthesized by a process analogous to Example 1, using Intermediate 4 and (4-hydroxy-2-methylphenyl)boronic acid, wherein only one boronic ester addition is required in Step (i) of the process. The crude residue was purified by HPLC (HPLC method B) to give Compound 5 (63.6 mg, 0.13 mmol, 65 %) as a white solid. LC/MS (Method C): *m/z* 475 $[M+H]^+$ 2.11 min. $^1H$ NMR (400 MHz, DMSO-$d_6$) δ 9.72 (br s, 1H), 8.73 (br s, 1H), 7.78 (m, 2H), 7.43 (s, 1H), 7.38 - 7.28 (m, 2H), 7.21 - 7.16 (m, 1H), 7.14 - 7.02 (m, 2H), 6.84 (d, *J* = 8.3 Hz, 1H), 6.65 - 6.62 (m, 1H), 6.58 (dd, *J* = 8.2, 2.5 Hz, 1H), 5.36 (br s, 1H), 4.83 - 4.74 (m, 1H), 4.51 - 4.37 (m, 2H), 4.32 - 4.15 (m, 2H), 3.63 - 3.52 (m, 1H), 3.50 - 3.16 (m, 2H, under water peak),), 2.75 - 2.63 (m, 1H), 2.02 (s, 3H), 1.96 - 1.85 (m, 1H), 1.30 (d, *J* = 7.0 Hz, 3H).

**Example 6: 4-((S)-1-((2R,4R)-1-((4'-carbamoyl-2'-methyl-[1,1'-biphenyl]-3-yl)methyl)-4-hydroxypyrrolidine-2-carboxamido)ethyl)benzoic acid (Compound 6)**

Compound 6 was synthesized by a process analogous to Example 1, using Intermediate 4 and 3-Methyl-4-(4,4,5,5-tetramethyl-1,3,2-dioxaborolan-2-yl)benzamide, wherein only one boronic ester addition is required in Step (i) of the process. The crude residue was purified by HPLC (HPLC method B) to give Compound 6 (106.5 mg, 0.21 mmol, 86 %) as a white foam. LC/MS (Method C): *m/z* 502 $[M+H]^+$ 2.12 min. $^1H$ NMR (400 MHz, DMSO-$d_6$) δ 9.74 (br s, 1H), 8.74 (d, *J* = 7.7 Hz, 1H), 7.92 (s, 1H), 7.79 - 7.74 (m, 3H), 7.68 (dd, *J* = 7.8, 1.8 Hz, 1H), 7.52 (s, 1H), 7.47 - 7.43 (m, 1H), 7.38 (m, 1H), 7.33 (s, 1H), 7.25 (dt, *J* = 7.6, 1.5 Hz, 1H), 7.07 (m, 3H), 5.36 (br s, 1H), 4.82 - 4.74 (m, 1H), 4.52 - 4.41 (m, 2H), 4.33 - 4.25 (m, 1H), 4.25 - 4.18 (m, 1H), 3.65 - 3.54 (m, 1H), 3.43 - 3.30 (m, 1H), 2.76 - 2.64 (m, 1H), 2.11 (s, 3H), 1.97 - 1.89 (m, 1H), 1.30 (d, *J* = 7.0 Hz, 3H).

**Example 7: 4-((S)-1-((R)-1-((4'-hydroxy-[1,1'-biphenyl]-3-yl)methyl)pyrrolidine-2-carboxamido)ethyl)benzoic acid (Compound 7)**

Compound 7 was synthesized by a process analogous to Example 1, using Intermediate 2 and 4-hydroxybenzeneboronic acid. The crude residue was purified by HPLC (HPLC method C) to give Compound 7 (49.2 mg, 0.11 mmol, 56 %) as a white foam. LC/MS (Method D): *m/z* 445 $[M+H]^+$ 1.82min. $^1H$ NMR (400 MHz, DMSO-$d_6$) δ 8.07 (d, *J* = 8.2 Hz, 1H), 7.84 - 7.79 (m, 2H), 7.51 - 7.49 (m, 1H), 7.48 - 7.42 (m, 3H), 7.36 - 7.29 (m, 3H), 7.25 - 7.20 (m, 1H), 6.84 - 6.79 (m, 2H), 4.87 - 4.79 (m, 1H), 3.79 (d, *J* = 12.8 Hz, 1H), 3.54 (d, *J* = 12.8 Hz, 1H), 3.09 (dd, *J* = 9.7, 4.4 Hz, 1H), 3.01 - 2.95 (m, 1H), 2.36 (dd, *J* = 8.2 Hz, 1H), 2.08 - 1.98 (m, 1H), 1.74 - 1.62 (m, 3H), 1.27 (d, *J*= 7.0 Hz, 3H). Exchangeable acid and hydroxy protons not observed in spectra.

**Example 8: 4-((S)-1-((R)-1-((2'-cyano-4'-hydroxy-[1,1'-biphenyl]-3-yl)methyl)pyrrolidine-2-carboxamido)ethyl) benzoic acid (Compound 8)**

Compound 8 was synthesized by a process analogous to Example 1, using Intermediate 2 and 5-hydroxy-2-(4,4,5,5-tetramethyl-1,3,2-dioxaborolan-2-yl)benzonitrile, wherein only one boronic ester addition is required in Step (i) of the process. The crude residue was purified by HPLC (HPLC method C) to give Compound 8 (22 mg, 0.05 mmol, 32 %) as a white foam. LC/MS (Method C): *m/z* 470 $[M+H]^+$ 1.60 min. $^1H$ NMR (400 MHz, DMSO-$d_6$) δ 8.04 (d, *J* = 8.2 Hz, 1H), 7.82 - 7.76 (m, 2H), 7.48 (br s, 1H), 7.45 - 7.33 (m, 4H), 7.28 - 7.23 (m, 2H), 7.22 (d, *J* = 2.6 Hz, 1H), 7.17 (dd, *J* = 8.6, 2.6 Hz, 1H), 4.89 - 4.80 (m, 1H), 3.85 (d, *J* = 13.0 Hz, 1H), 3.53 (d, *J*= 13.0 Hz, 1H), 3.10 (dd, *J* = 9.7, 4.3 Hz, 1H), 3.02 - 2.94 (m, 1H), 2.37 - 2.29 (m, 1H), 2.09 - 1.98 (m, 1H), 1.72 - 1.61 (m, 3H), 1.28 (d, *J* = 6.9 Hz, 3H). Exchangeable acid and hydroxy protons not observed in spectra.

**Example 9: 3'-(((R)-2-(((S)-1-(4-carboxyphenyl)ethyl)carbamoyl)pyrrolidin-1-yl)methyl)-2-methyl-[1,1'-biphenyl]-4-carboxylic acid (Compound 9)**

Compound 9 was synthesized by a process analogous to Example 1, using Intermediate 2 and 3-Methyl-4-(4,4,5,5-tetramethyl-1,3,2-dioxaborolan-2-yl)benzoic acid, wherein only one boronic ester addition is required in Step (i) of the process. The crude residue was purified by HPLC (HPLC method B) to give Compound 9 (34.7 mg, 0.07 mmol, 34 %), as a white foam. LC/MS (Method C): *m/z* 487 $[M+H]^+$, 1.29 min. $^1H$ NMR (400 MHz, DMSO-$d_6$) δ 12.88 (br s, 1H), 9.67 (br s, 1H), 8.93 (d, *J* = 7.7 Hz, 1H), 7.84 - 7.82 (m, 1H), 7.81 - 7.77 (m, 2H), 7.77 - 7.73 (m, 1H), 7.48 - 7.41 (m, 3H), 7.36 - 7.30 (m, 1H), 7.17 - 7.10 (m, 3H), 4.88 - 4.79 (m, 1H), 4.45 - 4.31 (m, 2H), 4.21 - 4.13 (m, 1H), 3.64 - 3.56 (m, 1H), 3.35 - 3.23 (m, 1H), 2.55 - 2.51 (m, 1H), 2.16 (s, 3H), 2.13 - 2.04 (m, 1H), 1.96 - 1.81 (m, 2H), 1.31 (d, *J* = 6.9 Hz, 3H).

**Example 10: 4-((S)-1-((R)-1-((2'-methyl-4'-(sulfamoyloxy)-1',2',3',4',5',6'-hexahydro-[1,1'-biphenyl]-3-yl)methyl) pyrrolidine-2-carboxamido)ethyl)benzoic acid (Compound 10)**

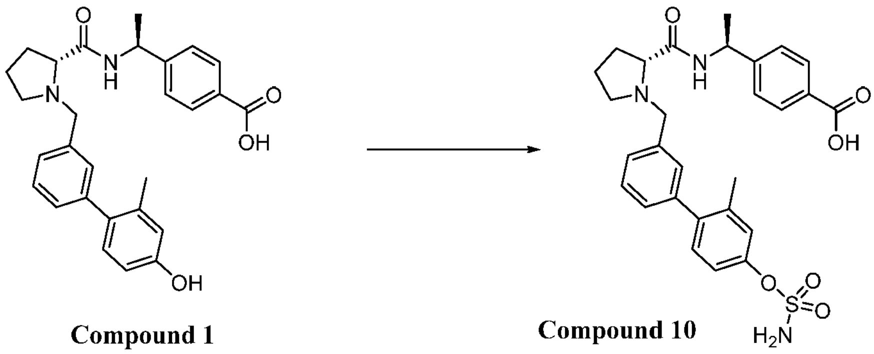

Compound 1

Compound 10

To a solution of Compound 1 (74.0 mg, 0.16 mmol) in DMA (1.4 mL), was added sulfamyl chloride (46.61 mg, 0.40 mmol) and the mixture was left to stir at RT for 18 hours, and checked by LC/MS for completion. A further addition of sulfamyl chloride (46.61 mg, 0.40 mmol) was made and the reaction mixture left to stir for a further 18 hours, and then 0.5 mL of water was added to quench the reaction. The mixture was purified by reversed phase HPLC (HPLC method B) to afford Compound 10 (68.4 mg, 0.127 mmol, 79% yield), as a colourless glass. LC/MS (Method C): *m/z* 538 $[M+H]^+$, 1.88 min. $^1H$ NMR (400 MHz, MeOD-$d_4$) δ 7.87 - 7.83 (m, 2H), 7.42 - 7.37 (m, 3H), 7.30 - 7.26 (m, 1H), 7.20 - 7.17 (m, 1H), 7.17 - 7.09 (m, 3H), 7.00 - 6.96 (m, 1H), 4.91 (q, *J* = 7.0 Hz, 1H), 4.50 (d, *J* = 12.8 Hz, 1H), 4.29 (d, *J* = 12.8 Hz, 1H), 4.22 (dd, *J* = 9.4, 6.1 Hz, 1H), 3.82 - 3.74 (m, 1H), 3.41 - 3.35 (m, 1H), 2.69 - 2.57 (m, 1H), 2.28 - 2.18 (m, 1H), 2.14 (s, 3H), 2.12 - 1.99 (m, 2H), 1.40 (d, *J* = 7.0 Hz, 3H). Exchangeable protons not observed.

**Example 11: 4-((S)-1-((R)-1-((4'-carbamoyl-2'-hydroxy-[1,1'-biphenyl]-3-yl)methyl)pyrrolidine-2-carboxamido) ethyl)benzoic acid (Compound 11)**

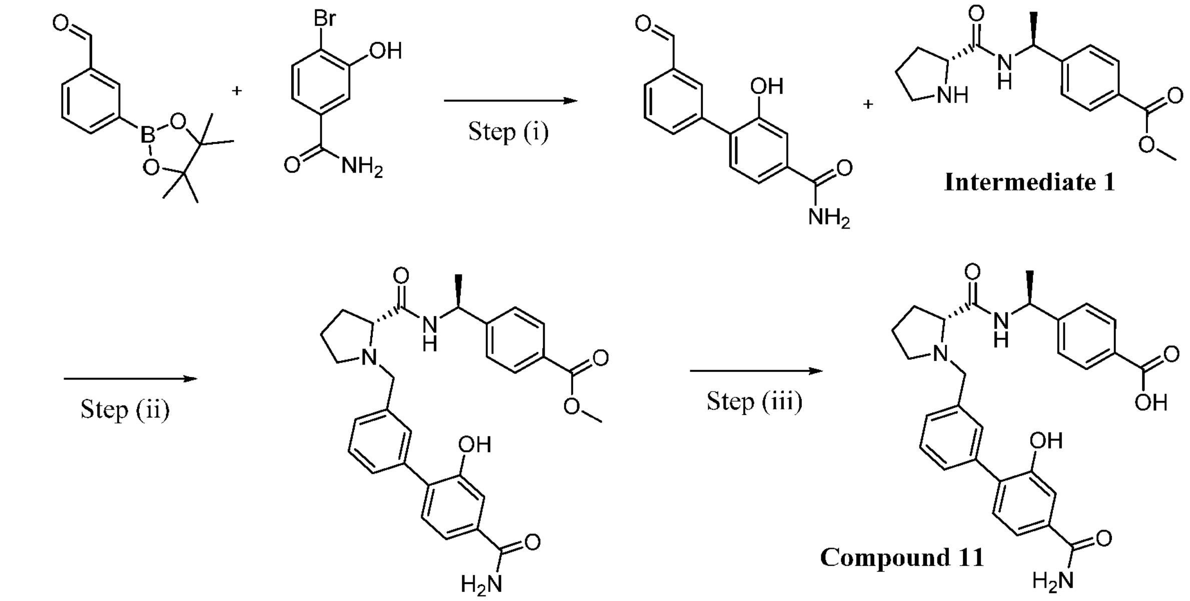


Intermediate 1

Compound 11

**Step (i):** A mixture of 4-bromo-3-hydroxybenzamide (204.79 mg, 0.95 mmol), 3-(4,4,5,5-tetramethyl-1,3,2-dioxaborolan-2-yl)benzaldehyde (200.00 mg, 0.86 mmol), potassium carbonate (238.21 mg, 1.72 mmol) and tetrakis(triphenylphosphine)palladium (0) (99.58 mg, 0.09 mmol) in 1,4-dioxane (4.5 mL) and water (1 mL) was heated to 100 °C in a microwave reactor for 40 minutes. The mixture was diluted with EtOAc and 1 M HCl (aq). The organic layers were separated, dried over $MgSO_4$, filtered and concentrated. The gummy residue was suspended in $Et_2O$ and sonicated. The liquid was pipetted off to afford 4-(3-formylphenyl)-3-hydroxy-benzamide (150 mg, 0.6218 mmol, 72% yield) as a yellow solid that was used without further purification.

**Step (ii):** A mixture of Intermediate 1 (150.0 mg, 0.480 mmol) and 4-(3-formylphenyl)-3-hydroxybenzamide (138.83 mg, 0.58 mmol) in DMF (3 mL) was stirred at RT for 30 minutes, and then sodium triacetoxyborohydride (203.27 mg, 0.96 mmol) was added. The mixture was stirred at RT overnight, after which it was purified directly by HPLC (HPLC method A) to afford methyl 4-[(1S)-1-[[(2R)-1-[[3-(4-carbamoyl-2-hydroxyphenyl)phenyl]methyl]pyrrolidine-2-carbonyl]amino]ethyl]benzoate (56.8 mg, 0.11 mmol, 23% yield) as a transparent gum. LC/MS (Method C): *m/z* 502 $[M+H]^+$ 3.69 min.

**Step (iii):** Performed analogously to Step (ii) of Example 1 to provide Compound 11. (40.2mg 73%) as a white foam. LC/MS (Method C): *m/z* 488 $[M+H]^+$ 1.72 min 1H NMR (400 MHz, DMSO-d6) $\delta$ 12.88 br(s, 1H), 9.87 (s, 1H), 9.73 (brs, 1H), 8.94 (d, J = 7.8 Hz, 1H), 7.88 (s, 1H), 7.84 (d, J = 8.0 Hz, 2H), 7.68 (s, 1H), 7.65-7.59 (m, 1H), 7.46 (s, 1H), 7.40 - 7.35(m, 3H), 7.31 (brs, 1H), 7.25 (d, J = 7.9 Hz, 1H), 7.18 (d, J = 8.0 Hz, 2H), 4.87 (q, J = 7.1 Hz, 1H), 4.43-4.32 (m, 2H), 4.22-4.14 (m, 1H), 3.36-3.26 (m, 1H), 3.16 (s, 1H), 2.16-2.05 (m, 1H), 1.96-1.82 (m, 2H), 1.34 (d, J = 6.9 Hz, 3H).LC/MS (Method C): *m/z* 488 $[M+H]^+$ 1.72 min

**Example 12: 4-((S)-1-((R)-1-((4'-carbamoyl-6-hydroxy-[1,1'-biphenyl]-3-yl)methyl)pyrrolidine-2-carboxamido)ethyl)benzoic acid (Compound 12)**

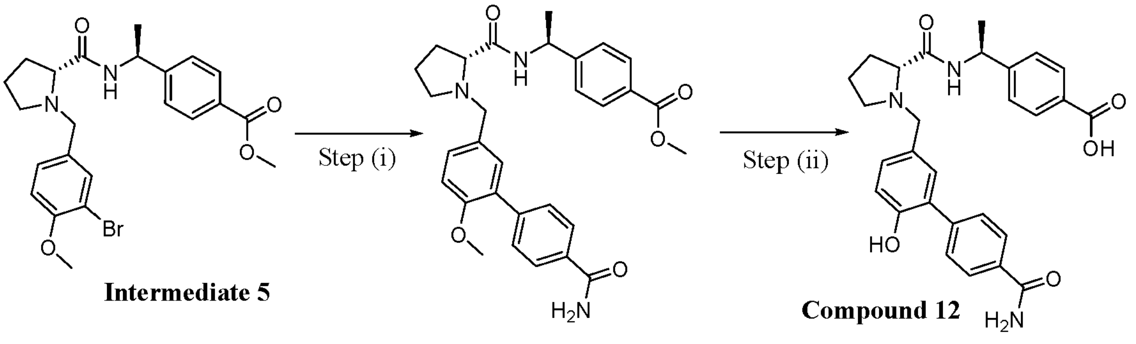


Intermediate 5

Compound 12

**Step (i):** A mixture of Intermediate 5 (100.0 mg, 0.21 mmol), (4-carbamoylphenyl)boronic acid (38. Mg, 0.23

mmol), potassium carbonate (58.15 mg, 0.42 mmol) and tetrakis(triphenylphosphine) palladium (0) (24.31 mg, 0.02 mmol) in 1,4-dioxane (1.6 mL) and water (0.50 mL) was heated to 100 °C in a microwave reactor for 40 minutes, after which time the mixture was diluted with EtOAc and water. The organic layers were separated and the aqueous layer was washed with brine, dried over $MgSO_4$, filtered and concentrated. The residue was purified by flash column chromatography (normal phase, 10 g Biotage® SNAP KP-Sil, 30 mL per min, gradient 0% to 8% MeOH in DCM), to afford methyl 4-[(1S)-1-[[(2R)-1-[[3-(4-carbamoylphenyl)-4-methoxy-phenyl]methyl]pyrrolidine-2-carbonyl]amino]ethyl]benzoate (48 mg, 0.093 mmol, 44% yield) as a transparent glass. LC/MS (Method B): *m/z* 516 $[M+H]^+$ 2.11 min.

**Step (ii):** To a solution of methyl 4-[(1S)-1-[[(2R)-1-[[3-(4-carbamoylphenyl)-4-methoxy-phenyl]methyl]pyrrolidine-2-carbonyl]amino]ethyl]benzoate (45.0 mg, 0.09 mmol) in DCM (1.5 mL) at -78 °C was added dropwise a 1 M solution of boron tribromide in DCM (0.44 mL, 0.44 mmol). The mixture was allowed to warm to RT gradually and was stirred at RT overnight. The mixture was cooled to 0 °C and $MeOH/H_2O$ 1:1 (2 mL) was added dropwise. The mixture was stirred for 1 hour and then was concentrated, and the crude material was purified by reversed phase HPLC (HPLC method C) to afford Compound 12 (25 mg, 0.05 mmol, 59% yield) as a white foam. LC/MS (Method C): *m/z* 488 $[M+H]^+$ 1.64 min. $^1H$ NMR (400 MHz, Methanol-$d_4$) δ 7.97 - 7.87 (m, 2H), 7.87 - 7.79 (m, 2H), 7.62 - 7.49 (m, 2H), 7.41 (d, J = 2.3 Hz, 1H), 7.26 (dd, J = 8.5, 2.3 Hz, 1H), 7.13 (d, J = 8.2 Hz, 2H), 6.91 (d, J = 8.3 Hz, 1H), 4.90 (q, J = 8.5, 7.1 Hz, 1H), 4.43 (d, J = 12.9 Hz, 1H), 4.23 - 4.11 (m, 2H), 3.76 (dt, J = 11.6, 6.0 Hz, 1H), 3.41 - 3.33 (m, 1H), 2.64 - 2.50 (m, 1H), 2.31 - 2.14 (m, 1H), 2.12 - 1.94 (m, 2H), 1.40 (d, J = 7.0 Hz, 3H). Exchangeable protons are absent

**Example 13: 4-((S)-1-((R)-1-((4'-carbamoyl-5-hydroxy-2'-methyl-1,2,3,4,5,6-hexahydro-[1,1'-biphenyl]-3-yl) methyl)pyrrolidine-2-carboxamido)ethyl)benzoic acid (Compound 13)**

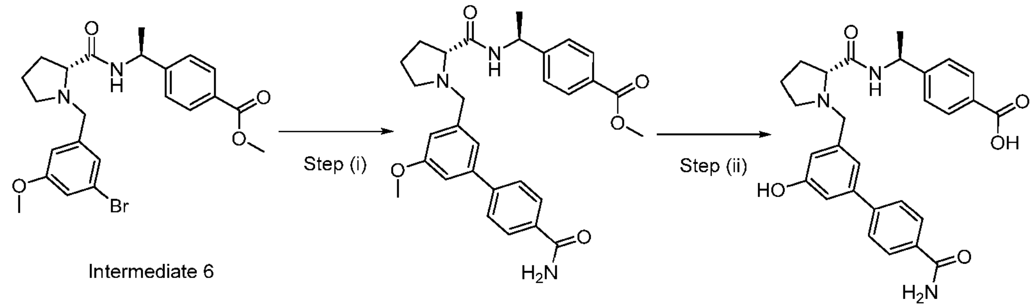


Intermediate 6

Compound 13

**Step (i):** A mixture of methyl 4-[(1S)-1-[[(2R)-1-[(3-bromo-5-methoxyphenyl)methyl]pyrrolidine-2-carbonyl]amino]ethyl]benzoate Intermediate 6 (250.mg, 0.53mmol), 3-Methyl-4-(4,4,5,5-tetramethyl-1,3,2-dioxaborolan-2-yl)benzamide (151.0mg, 0.58mmol), potassium carbonate (145.4mg, 1.05mmol) and tetrakis(triphenylphosphine)palladium (0) (60.8mg, 0.050mmol) in 1,4-Dioxane (3.5 mL) and water (0.8 mL) was heated to 100 °C in a Biotage microwave reactor for 1 hour, after which time the mixture was diluted with EtOAc and water. The organics were separated, and the aq. Layer was washed with brine, dried over $MgSO_4$ filtered and concentrated. The residue was purified by flash column chromatography (normal phase, 10g, Biotage® SNAP KP-Sil - 50μm irregular silica, 30 mL per min, [gradient 0% to 8% MeOH in DCM], The resulting impure gum was sonicated in ether and the solids were filtered off to afford methyl 4-[(1S)-1-[[(2R)-1-[[3-(4-carbamoyl-2-methyl-phenyl)-5-methoxy-phenyl]methyl]pyrrolidine-2-carbonyl]amino]ethyl]benzoate (174 mg,0.33mmol, 62% yield) as a cream coloured solid. LC/MS (Method B): *m/z* 530 $[M+H]^+$ 2.16 min.

**Step (ii):** To a solution of methyl 4-[(1S)-1-[[(2R)-1-[[3-(4-carbamoyl-2-methylphenyl)-5-methoxy-phenyl] methyl]pyrrolidine-2-carbonyl]amino]ethyl]benzoate (170.mg, 0.32mmol) in DCM (5 mL) at -78 °C was added boron tribromide, 1M solution in DCM (1.6mL, 1.6mmol) dropwise. The mixture was allowed to warm to RT gradually and was stirred at RTovernight. The mixture was cooled to 0 °C and $MeOH/H_2O$ 1:1 (2 mL) was added dropwise. The mixture was stirred for 1 hour after which time it was concentrated. The crude material was purified by reversed phase HPLC (HPLC method C) to afford Compound 13 (89 mg,0.18mmol, 55% yield) as a white foam. LC/MS (Method C): *m/z* 502 $[M+H]^+$ 1.66 min. $^1H$ NMR (400 MHz, Methanol-$d_4$) δ 8.00 - 7.85 (m, 2H), 7.73 (s, 1H), 7.63 (d, J = 8.0 Hz, 1H), 7.19 (d, J = 8.1 Hz, 2H), 7.00 (d, J = 7.9 Hz, 1H), 6.95 - 6.69 (m, 3H), 4.96 (q, J = 6.7 Hz, 1H), 4.43 (d, J = 12.8 Hz, 1H), 4.28 - 4.12 (m, 2H), 3.88 - 3.71 (m, 1H), 3.40 - 3.35 (m, 1H), 2.67 - 2.55 (m, 1H), 2.31 - 2.18 (m, 1H), 2.16 (s, 3H), 2.13 - 1.97 (m, 2H), 1.42 (d, J = 7.0 Hz, 3H). Exchangeable protons are absent

**Example 14: 4-((1S)-1-((2R)-1-((4'-carbamoyl-6-hydroxy-2'-methyl-1,2,3,4,5,6-hexahydro-[1,1'-biphenyl]-3-yl)methyl)pyrrolidine-2-carboxamido)ethyl)benzoic acid (Compound 14)**

Compound 14 was synthesized by a process analogous to Example 12, using Intermediate 5 and 3-Methyl-4-(4,4,5,5-tetramethyl-1,3,2-dioxaborolan-2-yl)benzamide to give Compound 14. LC/MS (Method C): *m/z* 502 $[M+H]^+$ 3.69 min. $^1H$ NMR (400 MHz, Methanol-$d_4$) δ 7.94 (d, J = 8.2 Hz, 2H), 7.73 (d, J = 1.8 Hz, 1H), 7.68 - 7.54 (m, 1H), 7.35 - 7.26 (m, 1H), 7.21 (d, J = 8.1 Hz, 2H), 7.16 (d, J = 2.3 Hz, 1H), 7.09 - 6.96 (m, 1H), 6.90 (d, J = 8.3 Hz, 1H), 4.96 (q, J = 7.0, 6.5 Hz, 1H), 4.38 (d, J = 12.8 Hz, 1H), 4.23 - 4.11 (m, 2H), 3.78 - 3.66 (m, 1H), 3.34 - 3.31 (m, 1H), 2.64 - 2.52 (m, 1H), 2.29 - 2.15 (m, 1H), 2.11 (s, 3H), 2.09 - 1.98 (m, 2H), 1.44 (d, J = 7.0 Hz, 3H). Exchangeable protons are absent.

**Example 15: (R)-4-((1-((4'-carbamoyl-2'-methyl-[1,1'-biphenyl]-3-yl)methyl)pyrrolidine-2-carboxamido)methyl)-2-hydroxybenzoic acid (Compound 15)**

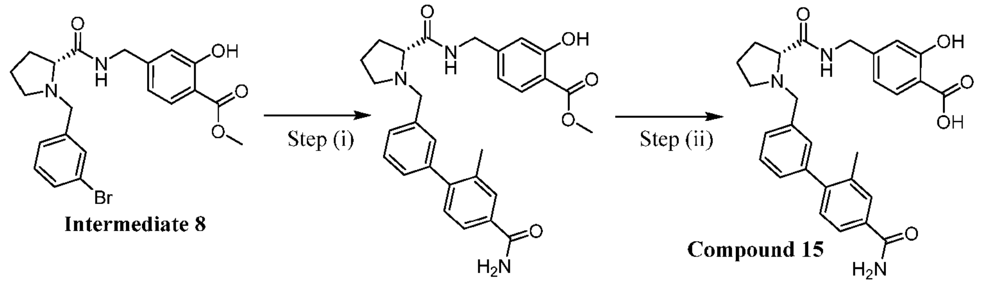

Intermediate 8
Compound 15

**Step (i):** Intermediate 8 (0.20 g, 0.45 mmol), 3-methyl-4-(4,4,5,5-tetramethyl-1,3,2-dioxaborolan-2-yl)benzamide (0.15 g, 0.58 mmol), and $K_2CO_3$ (0.123 g, 0.89 mmol) were dissolved in 6 mL of a mixture of dioxane:water (3:3), and nitrogen gas was purged through the mixture for 30 minutes at room temperature. $PdCl_2$(dppf)DCM (0.036 g, 0.045 mmol) was then added and the reaction mixture was allowed to stir at 80 °C for 4 hours. The reaction mixture was then partitioned between water (70 mL) and EtOAc (70 mL). Aqueous layer was further extracted with EtOAc (2 x 50 mL), and the organic layers were combined and dried ($Na_2SO_4$). The solvent was removed *in vacuo* and the crude product was purified by reverse phase gradient flash column chromatography (reverse phase, C18 silica). The product eluted at 0% to 55% MeCN in water to afford pure methyl (R)-4-((1-((4'-carbamoyl-2'-methyl-[1,1'-biphenyl]-3-yl)methyl)pyrrolidine-2-carboxamido)methyl)-2-hydroxybenzoate (0.147 g, 65%) as a light brown solid. LC/MS: (Method A): m/z 502 $[M+H]^+$ at 1.46 min.

**Step (ii):** methyl 4-((S)-1-((3R,6R)-6-methyl-4-(4-(trifluoromethyl)benzyl) morpholine-3-carboxamido)ethyl) benzoate (0.147 g, 0.29 mmol) was dissolved in dioxane (2 mL) and water (1 mL). LiOH monohydrate (0.036 g, 0.88 mmol) was added at room temperature and allowed to stir at room temperature for 16 hours. The reaction mixture was then acidified with 4 N aqueous HCl (2 mL) to adjust the pH ~1, and extracted with EtOAc (40 mL). The aqueous layer was further extracted with EtOAc (3 x 30 mL), and the organic layers were combined and dried ($Na_2SO_4$). The solvent was removed *in vacuo* and the crude product was purified by reverse phase gradient flash column chromatography (reverse phase, C18 silica), product eluted at 0% to 26% MeCN in water to afford Compound 15 (0.10 g, 70%) as an off white solid. LC/MS: (Method A): m/z 488 $[M+H]^+$ at 1.34 min. $^1H$ NMR: (400 MHz, DMSO) 1.79 (s, 3H), 2.24 (s, 3H), 3.17 (s, 3H), 3.81 (s, 1H), 4.08 (s, 1H), 4.26-4.14 (m, 2H), 6.62-6.60 (d, 1H, J= 8.0Hz), 6.66 (s, 1H), 7.34-7.24 (m, 3H), 7.44-7.401 (m, 3H), 7.64-7.62 ((d, 1H, J= 8.0Hz), 7.75-7.73 (d, 1H, J= 7.6Hz), 7.80 (s, 1H), 7.97 (s, 1H), 8.53 (s, 1H), 12.97 (s, 2H).

**Example 16: (R)-2-hydroxy-4-((1-((2'-methyl-4'-sulfamoyl-[1,1'-biphenyl]-3-yl)methyl)pyrrolidine-2-carboxamido)methyl)benzoic acid (Compound 16)**

Compound 16 was synthesized by a process analogous to Example 15, using Intermediate 8 and 3-methyl-4-(4,4,5,5-tetramethyl-1,3,2-dioxaborolan-2-yl)benzenesulfonamide to give Compound 16. LC/MS: (Method A) m/z 524 $[M+H]^+$ at 1.40 min. $^1H$ NMR: (400 MHz, DMSO): 1.77-1.74 (t, 3H, J=6.6Hz), 2.17 (s, 1H), 2.26 (s, 3H), 3.05 (s, 1H), 3.42-3.40 (m, 1H), 3.50-3.47 (m, 1H), 3.68 (s, 1H), 4.00-3.97 (d, 1H, J=12.4Hz), 4.25-4.13 (m, 2H), 6.57-6.56 (d, 1H, J=8.0Hz), 6.61 (s, 1H), 7.28-7.268 (d, 1H, J=6.8Hz), 7.44-7.35 (m, 6H), 7.60-7.58 (d, 1H, J=8.0Hz), 7.70-7.67 (dd, 1H, J=1.6Hz & J=4.0Hz), 7.74-7.74 (d, 1H, 1.2Hz), 8.39 (s, 1H), 13.62 (s, 2H).

**Example 17: 4-((S)-1-((R)-1-((4'-carbamoyl-2'-methyl-[1,1'-biphenyl]-3-yl)methyl)pyrrolidine-2-carboxamido) ethyl)-2-hydroxybenzoic acid (Compound 17)**

Compound 17 was synthesized by a process analogous to Example 15, using Intermediate 10 and 3-methyl-4-(4,4,5,5-tetramethyl-1,3,2-dioxaborolan-2-yl)benzamide to give Compound 17. LC/MS: (Method A): Product mass was confirmed m/z 502 $[M+H]^+$ at 1.16 min. $^1$H NMR: (400 MHz, DMSO) 1.28-1.26 (d, 3H, J=7.2Hz), 1.85-1.75 (m, 3H), 2.24 (s, 4H), 3.16 (s, 2H), 3.84 (s, 1H), 4.01 (s, 1H), 4.79-4.72 (m, 1H), 6.66-6.64 (d, 1H, J= 7.2Hz), 6.72 (s, 1H), 7.30-7.23 (dd, 2H, J= 7.6Hz), 7.45-7.35. (m, 4H), 7.64-7.62 (d, 1H, J= 8.0Hz), 7.75-7.73 (d, 1H, J= 8.0Hz), 7.80 (s, 1H), 7.98 (s, 1H), 8.32 (s, 1H), 12.97 (s, 1H).

**Example 18: 2-hydroxy-4-((S)-1-((R)-1-((2'-methyl-4'-sulfamoyl-[1,1'-biphenyl]-3-yl)methyl)pyrrolidine-2-carboxamido)ethyl)benzoic acid (Compound 18)**

Compound 18 was synthesized by a process analogous to Example 15, using Intermediate 10 and 3-methyl-4-(4,4,5,5-tetramethyl-1,3,2-dioxaborolan-2-yl)benzenesulfonamide to give Compound 18. LC/MS: (Method A) m/z 538 $[M+H]^+$ at 1.22 min. $^1$H NMR: (400 MHz, DMSO) 1.27-1.26 (d, 3H, J=6.8Hz), 1.84-1.75 (m, 3H), 2.26 (s, 4H), 3.20 (s, 1H), 3.87-3.80 (bs, 1H), 4.02-3.80 (bs, 1H), 4.77-4.73 (t, 1H, J=7.2Hz), 6.65-6.64 (d, 1H, J= 6.4Hz), 6.72 (s, 1H), 7.46-7.29 (m, 7H), 7.62-7.60 (d, 1H, J= 8.0Hz), 7.69-7.670 (d, 1H, J= 8.0Hz), 7.74 (s, 1H), 8.32-8.31 (m, 1H), 13.08 (s, 1H). (some aliphatic protons merged with DMSO-$d_6$ water peak)

**Example 19: 4-((S)-1-((R)-1-((6-hydroxy-4'-sulfamoyl-[1,1'-biphenyl]-3-yl)methyl)pyrrolidine-2-carboxamido) ethyl)cyclohex-3-ene-1-carboxylic acid (Compound 19)**

Compound 19 was synthesized by a process analogous to Example 12, using Intermediate 5 and 4-(4,4,5,5-tetramethyl-1,3,2-dioxaborolan-2-yl)benzenesulfonamide to give Compound 19. LC/MS (Method D): *m/z* 524 $[M+H]^+$ 1.56 min. $^1$H NMR (400 MHz, Methanol-$d_4$) δ 7.86 (d, J = 8.0 Hz, 2H), 7.82 - 7.77 (m, 2H), 7.59 - 7.54 (m, 2H), 7.39 (d, J = 2.3 Hz, 1H), 7.29 - 7.23 (m, 1H), 7.08 (d, J = 8.0 Hz, 2H), 6.89 (d, J = 8.3 Hz, 1H), 4.91 - 4.85 (m, 1H), 4.43 (d, J = 12.9 Hz, 1H), 4.22 - 4.07 (m, 2H), 3.75 (dt, J = 11.6, 6.0 Hz, 1H), 3.39 - 3.32 (m, 1H), 2.65 - 2.47 (m, 1H), 2.27 - 2.13 (m, 1H), 2.11 - 1.92 (m, 2H), 1.36 (d, J = 7.0 Hz, 3H). Exchangeable protons are absent

**Example 20: 4-((S)-1-((R)-1-((5-hydroxy-2'-methyl-4'-sulfamoyl-[1,1'-biphenyl]-3-yl)methyl)pyrrolidine-2-carboxamido)ethyl)benzoic acid (Compound 20)**

Compound 20 was synthesized by a process analogous to Example 12, using Intermediate 6 and 3-methyl-4-(4,4,5,5-tetramethyl-1,3,2-dioxaborolan-2-yl)benzenesulfonamide to give Compound 20. LC/MS (Method D): *m/z* 538 $[M+H]^+$ 1.69 min. $^1$H NMR (400 MHz, Methanol-$d_4$) δ 7.92 - 7.83 (m, 2H), 7.76 - 7.71 (m, 1H), 7.65 - 7.59 (m, 1H), 7.21 - 7.13 (m, 2H), 7.03 (d, J = 8.1 Hz, 1H), 6.93 (dd, J = 2.3, 1.6 Hz, 1H), 6.85 (t, J = 1.5 Hz, 1H), 6.76 (dd, J = 2.4, 1.4 Hz, 1H), 4.94 (q, J = 7.0 Hz, 1H), 4.45 (d, J = 12.7 Hz, 1H), 4.27 - 4.15 (m, 2H), 3.86 - 3.72 (m, 1H), 3.43 - 3.35 (m, 1H), 2.69 - 2.55 (m, 1H), 2.31 - 2.17 (m, 1H), 2.17 (s, 3H), 2.15 - 1.98 (m, 2H), 1.41 (d, J = 7.0 Hz, 3H). Exchangeable protons are absent.

**Example 21: 4-((S)-1-((2R,4R)-1-((4'-carbamoyl-5-hydroxy-2'-methyl-[1,1'-biphenyl]-3-yl)methyl)-4-hydroxypyrrolidine-2-carboxamido)ethyl)benzoic acid (Compound 21)**

Compound 21 was synthesized by a process analogous to Example 12, using Intermediate 6 and 3-methyl-4-(4,4,5,5-tetramethyl-1,3,2-dioxaborolan-2-yl)benzamide to give Compound 21. LC/MS (Method D): *m/z* 518 $[M+H]^+$ 1.46 min. $^1$H NMR (400 MHz, Methanol-$d_4$) δ 7.91 - 7.84 (m, 2H), 7.71 (dt, J = 2.0, 0.6 Hz, 1H), 7.64 - 7.59 (m, 1H), 7.19 - 7.12 (m, 2H), 6.97 (d, J = 8.0 Hz, 1H), 6.92 (dd, J = 2.3, 1.5 Hz, 1H), 6.89 (t, J = 1.5 Hz, 1H), 6.75 (dd, J = 2.3, 1.5 Hz, 1H), 4.93 (q, J = 7.0 Hz, 1H), 4.59 - 4.55 (m, 1H), 4.52 (d, J = 12.6 Hz, 1H), 4.31 (dd, J = 10.8, 4.2 Hz, 1H), 4.17 (d, J = 12.7 Hz, 1H), 3.79 (d, J = 11.8 Hz, 1H), 3.43 (dd, J = 11.8, 3.8 Hz, 1H), 2.83 (ddd, J = 14.0, 10.8, 4.7 Hz, 1H), 2.19 - 2.08 (m, 4H), 1.42 (d, J = 7.0 Hz, 3H). Exchangeable protons are absent.

**Example 22: 4-((1S)-1-((2R)-1-((2'-hydroxy-4'-sulfamoyl-1',2',3',4',5',6'-hexahydro-[1,1'-biphenyl]-3-yl)methyl) pyrrolidine-2-carboxamido)ethyl)benzoic acid (Compound 22)**

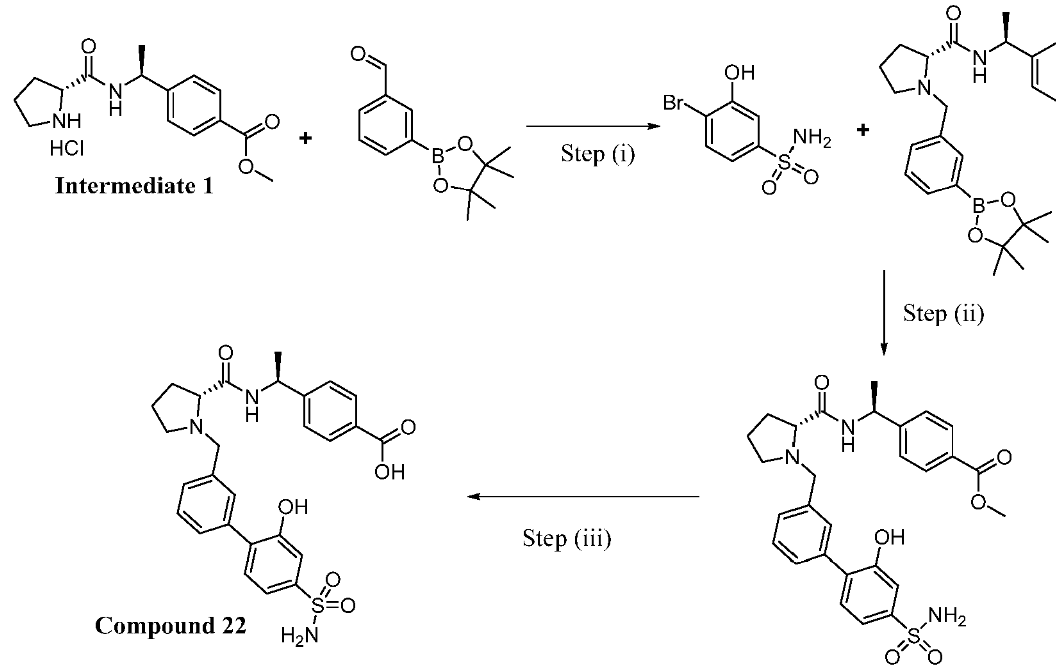


**Step (i):** Intermediate 1-HCl (0.35 g, 1.12 mmol) and 3-(4,4,5,5-tetramethyl-1,3,2-dioxaborolan-2-yl)benzaldehyde (0.31 g, 1.34 mmol) were dissolved in DCM (4 mL) under nitrogen atmosphere. 4 Å molecular sieves (300 mg) were also added to maintain moisture free condition. Potassium acetate (0.11 g, 1.68 mmol) was then added at room temperature and the reaction mixture was allowed to stir for 4 hours at room temperature. The reaction mixture was then cooled to 0°C and sodium triacetoxyborohydride (0.28 g, 1.34 mmol) was added in portions, and the reaction mixture was allowed to stir at room temperature for 16 hours. The solvent was removed *in vacuo* to obtain the crude product, which was purified by reverse phase gradient flash column chromatography (reverse phase, C18 Silica), product eluted at 0% to 22% MeCN to afford methyl 4-((S)-1-((R)-1-(3-(4,4,5,5-tetramethyl-1,3,2-dioxaborolan-2-yl)benzyl)pyrrolidine-2-carboxamido)ethyl)benzoate (0.26 g, 47.18%) as yellow solid. LC/MS: (Method A): m/z 411 $[M+H]^+$, (boronic acid) at 1.22 min. & m/z 493 $[M+H]^+$, at 1.81 min.

**Step (ii)** : methyl 4-((S)-1-((R)-1-(3-(4,4,5,5-tetramethyl-1,3,2-dioxaborolan-2-yl)benzyl)pyrrolidine-2-carboxamido)ethyl)benzoate (0.16 g, 0.32 mmol), 4-bromo-3-hydroxybenzenesulfonamide (0.10 g, 0.39 mmol) and $K_2CO_3$ (0.13 g, 0.97 mmol) were dissolved in toluene (1 mL), ethanol (0.5 mL) and water (0.5 mL). Nitrogen gas was purged at room temperature for 20 minutes, and then $PdCl_2$(dppf)DCM (0.014 g, 0.016 mmol) was added and the reaction mixture was allowed to stir at 90°C for 1.5 hours. The reaction mixture was partitioned between water (70 mL) and EtOAc (70 mL), and the aqueous layer was further extracted with EtOAc (2 x 40 mL). The organic layers were combined and dried ($Na_2SO_4$), the solvent was removed *in vacuo,* and the crude product was purified by reverse phase gradient flash column chromatography (reverse phase, C18 Silica). The product eluted at 0% to 24% 24% MeCN in water to afford methyl 4-((S)-1-((R)-1-((2'-hydroxy-4'-sulfamoyl-[1,1'-biphenyl]-3-yl)methyl)pyrrolidine-2-carboxamido)ethyl)benzoate (0.08 g, 46.96%) as a yellow solid, which was used in the next step without further purification. LC/MS: (Method A): m/z 538 $[M+H]^+$ , at 1.33 min.

**Step (iii):** methyl 4-((S)-1-((R)-1-((2'-hydroxy-4'-sulfamoyl-[1,1'-biphenyl]-3-yl)methyl)pyrrolidine-2-carboxamido)ethyl)benzoate (0.08 g, 0.15 mmol) was dissolved in dioxane (0.5 mL) and water (0.5 mL). LiOH (0.03 g, 0.76 mmol) was added at room temperature and reaction mixture was allowed to stir at room temperature for 3 hours. The reaction mixture was concentrated *in vacuo* to afford the crude product, which was purified using prep HPLC (HPLC method D).

LC/MS: (Method A): m/z 524 $[M+H]^+$ , at 1.18 min. $^1H$ NMR: (400 MHz, $CD_3OD$): δ 1.34-1.35 (d, 3H J= 6.8Hz), 2.05-1.89 (m, 3H), 2.34 (s, 1H), 2.82-2.81 (d, 1H, J= 4.8Hz), 3.38-3.37 (d, 1H, J= 3.2Hz), 3.55-3.53 (d, 1H, J= 6.0Hz), 4.00 (s, 2H), 7.26-7.25 (d, 2H, J= 7.2Hz), 7.45-7.34 (m, 5H), 7.58-7.56 (d, 1H, J= 7.6Hz), 7.64 (s, 1H), 7.91--7.89 (d, 2H, J= 7.2Hz).

**Example 23: (S)-4-(1-(2-((4'-carbamoyl-5-hydroxy-2'-methyl-[1,1'-biphenyl]-3-yl)methyl)-2-azabicyclo[2.1.1] hexane-1-carboxamido)ethyl)benzoic acid (Compound 23)**

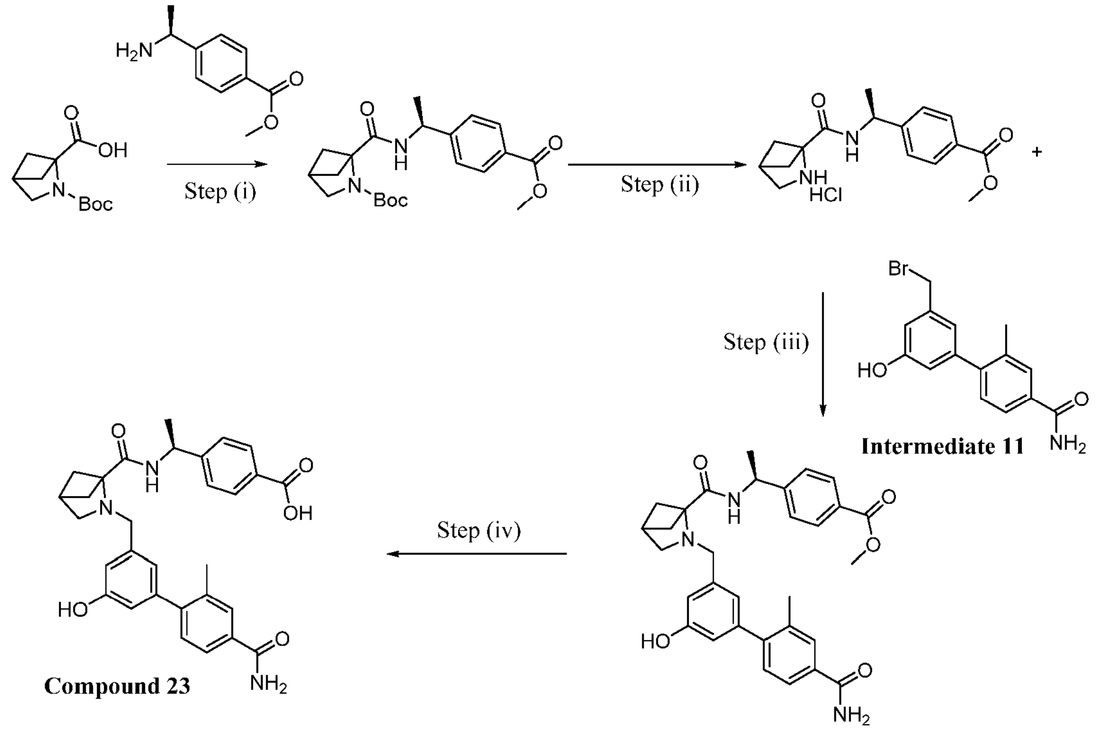


**Step (i):** 2-(*tert*-butoxycarbonyl)-2-azabicyclo[2.1.1]hexane-1-carboxylic acid (0.25 g, 1.10 mmol) was dissolved in MeCN (3 mL) and methyl (S)-4-(1-aminoethyl)benzoate (0.23 g, 1.32 mmol) was added to reaction mixture at room temperature. HATU (0.63 g, 1.65 mmol) was then added and the reaction mixture was allowed to stir at room temperature for 30 min. N, N-Diisopropyethyllamine (0.6 mL, 3.30 mmol) was then added at room temperature and allowed to stir at room temperature for 2 hours. The reaction mixture was partitioned between water (60 mL) and EtOAc (50 mL), and the aqueous layer was further extracted with EtOAc (2 X 30 mL). The organic layers were then combined and dried ($Na_2SO_4$), the solvent was removed *in vacuo,* and the crude product was purified by reverse phase gradient flash column chromatography (C18 silica), product was eluted at 100% MeCN to afford *tert*-butyl (S)-1-((1-(4-(methoxycarbonyl) phenyl)ethyl)carbamoyl)-2-azabicyclo[2.1.1]hexane-2-carboxylate (0.42 g, 98%) as yellow solid. LC/MS: (Method A): m/z 289 $[M+H-Boc]^+$ at 2.23 min.

**Step (ii):** *Tert*-butyl (S)-1-((1-(4-(methoxycarbonyl)phenyl)ethyl)carbamoyl)-2-azabicyclo[2.1.1] hexane-2-carboxylate (0.42 g, 1.08 mmol) was dissolved in dioxane (2 mL) under nitrogen atmosphere, and 4 N HCl in dioxane (4 mL) was then added at room temperature and allowed to stir at room temperature for 3 hours. The solvent was removed in *vacuo* and the crude material was purified by trituration with diethyl ether (5 mL) to afford methyl (S)-4-(1-(2-azabicyclo [2.1.1]hexane-1-carboxamido)ethyl)benzoate hydrochloride (0.34 g, quantitative) as a pale yellow solid. LC/MS: (Method A): m/z 289 (ES+) at 1.00 min.

**Step (iii):** Methyl (S)-4-(1-(2-azabicyclo [2.1.1]hexane-1-carboxamido)ethyl) benzoate hydrochloride (0.26 g, 0.80 mmol) and DIPEA (0.4 mL, 2.40 mmol) were dissolved in MeCN (3 mL) at room temperature. After this, 3'-(bromomethyl)-5'-hydroxy-2-methyl-[1,1'-biphenyl]-4-carboxamide (0.30 g, 0.96 mmol) was added and reaction mixture was stirred at 80°C for 16 hours. The reaction mixture was partitioned between water (80 mL) and EtOAc (80 mL), and the aqueous layer was further extracted with EtOAc (2 x 25 mL). The organic layers were combined and dried ($Na_2SO_4$), the solvent was removed *in vacuo,* and the crude product was purified by reverse phase gradient flash column chromatography (C18 silica). The product was eluted at 0% to 59% MeCN in water to afford methyl (S)-4-(1-(2-((4'-carbamoyl-5-hydroxy-2'-methyl-[1,1'-biphenyl]-3-yl)methyl)-2-azabicyclo [2.1.1]hexane-1-carboxamido)ethyl)benzoate (0.20 g, 48%) as a yellow solid. LC/MS (Method A): m/z 528.17 (ES+) at 1.65 min.

**Step (iv):** Methyl (S)-4-(1-(2-((4'-carbamoyl-5-hydroxy-2'-methyl-[1,1'-biphenyl]-3-yl)methyl)-2-azabicyclo [2.1.1]hexane-1-carboxamido)ethyl)benzoate (0.20 g, 0.39 mmol) was dissolved in dioxane (1 mL) and water (1 mL). LiOH monohydrate (0.084 g, 1.99 mmol) was then added at room temperature and allowed to stir at room temperature for 3 hours. The reaction mixture was acidified with glacial acetic acid (2 mL) to adjust the pH to ~4 and then concentrated *in vacuo.* The crude product was purified by reverse phase gradient flash column chromatography (reverse phase, C18 silica), and the product was eluted at 0% to 36% ACN in water to afford Compound 23 (0.17 g, 85.19%) as an off-white solid. LC/MS (Method A): m/z 514.2 (ES+) at 1.456 min. $^1H$ NMR: (400 MHz, DMSO) δ 1.373-1.355 (d, 3H, J=7.2Hz), 1.791-1.768 (d, 4H, J=9.2Hz), 2.267 (s, 3H), 2.619-2.595 (d, 2H, J=9.6Hz), 2.686-2.666 (d, 1H, J=8.0Hz), 3.475 (s, 2H), 5.041-4.986 (m, 1H), 6.594 (s, 1H), 6.848-6.822 (d, 2H, J=10.4Hz), 7.251-7.231 (d, 1H, J=8.0Hz), 7.341 (s, 1H) ,

7.403-7.383 (d, 2H, J=8.0Hz), 7.735-7.716 (d, 1H, J=7.6Hz), 7.800-7.780 (t, 3H, J=4.0Hz), 7.968 (s, 1H), 8.228-8.208 (d, 1H, J=8.0Hz), 12.979 (s, 1H).

**Example 24: 4-((S)-1-((R)-1-((5-(2-methyl-4-sulfamoylphenyl)pyridin-3-yl)methyl)pyrrolidine-2-carboxamido) ethyl)benzoic acid (Compound 24)**

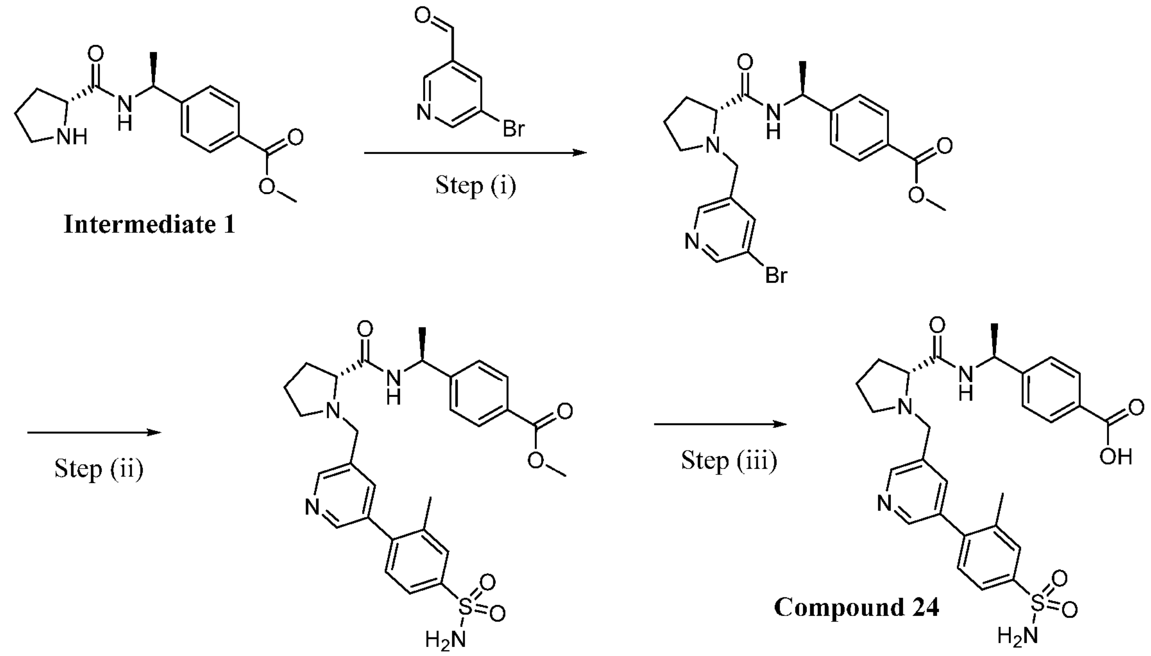


Intermediate 1

Compound 24

**Step (i):** A mixture of 5-bromonicotinaldehyde (356.81 mg, 1.92 mmol) and Intermediate 1 (300.0 mg, 0.96 mmol) in DCM (4.5 mL) was stirred at RT for 1 hour, and then sodium triacetoxyborohydride (426.87 mg, 2.01 mmol) was added and the mixture stirred at RT for 16 hours, after which it was diluted with sat $NaHCO_3$. The organic layers were separated, washed with water and brine, dried (frit), and concentrated. The residue was then purified by flash column chromatography (25 g Biotage® SNAP KP-Sil, 30 mL per min, gradient 30% to 100% ethyl acetate in isohexane) to afford methyl 4-[(1S)-1-[[(2R)-1-[(5-bromo-3-pyridyl)methyl]pyrrolidine-2-carbonyl]amino]ethyl]benzoate (398.1 mg, 0.89 mmol, 93% yield), as a white solid. LC/MS (Method B): *m/z* 446 $[M+H]^+$, 2.12 min.

**Step (ii):** A mixture of methyl 4-[(1S)-1-[[(2R)-1-[(5-bromo-3-pyridyl)methyl]pyrrolidine-2-carbonyl]amino]ethyl] benzoate (200.0 mg, 0.45 mmol), 3-methyl-4-(4,4,5,5-tetramethyl-1,3,2-dioxaborolan-2-yl)benzenesulfonamide (173.11 mg, 0.58 mmol), potassium carbonate (123.86 mg, 0.90 mmol), and tetrakis(triphenylphosphine) palladium (0) (77.67 mg, 0.07 mmol) in 1,4-dioxane (1.8 mL) and water (0.45 mL), was heated to 100 °C for 30 minutes in a microwave reactor. The crude product was diluted with water and EtOAc and the organic layers were separated, washed with water, brine, and dried (frit) before being concentrated *in vacuo.* The residue was purified by flash column chromatography (50 g Biotage® SNAP KP-Sil 80 mL per min, gradient 30% to 100% ethyl acetate in isohexane) to give methyl 4-[(1S)-1-[[(2R)-1-[[5-(2-methyl-4-sulfamoyl-phenyl)-3-pyridyl]methyl] pyrrolidine-2-carbonyl]amino]ethyl]benzoate (231 mg, 96.065%), as an off-white solid. LC/MS (Method B): *m/z* 537 $[M+H]^+$, 1.93 min.

**Step (iii):** To a solution of lithium hydroxide monohydrate (63.22 mg, 1.51 mmol) in water (3.2 mL) and 1,4-dioxane (3.2 mL) was added methyl 4-[(1S)-1-[[(2R)-1-[[5-(2-methyl-4-sulfamoyl-phenyl)-3-pyridyl]methyl]pyrrolidine-2-carbonyl]amino]ethyl]benzoate (231.0 mg, 0.43 mmol), and the resultant mixture left to stir for 3 hours before being checked for completion by LC/MS. The mixture was then concentrated *in vacuo* and the residue purified by reversed phase HPLC (5-35 %, low pH) to afford Compound 24 (136.1 mg, 0.26 mmol, 60.5% yield), as a colourless glass. LC/MS (Method D): *m/z* 523 $[M+H]^+$, 1.51 min. $^1H$ NMR (400 MHz, DMSO-$d_6$) δ 9.83 (br, s, 1H), δ 9.01 (d, J = 7.7 Hz, 1H), 8.66 (d, J = 2.0 Hz, 1H), 8.53 (d, J = 2.1 Hz, 1H), 7.92 (t, J = 2.1 Hz, 1H), 7.83 - 7.78 (m, 2H), 7.77 - 7.73 (m, 1H), 7.71 - 7.64 (m, 1H), 7.36 (s, 2H), 7.22 (d, J = 8.0 Hz, 1H), 7.15 (d, J = 8.1 Hz, 2H), 4.84 (p, J = 7.0 Hz, 1H), 4.51 - 4.42 (m, 2H), 4.27 - 4.22 (m, 1H), 3.69 - 3.55 (m, 1H), 3.44 - 3.25 (m, 1H), 2.17 (s, 3H), 2.15 - 2.06 (m, 1H), 1.91 (tq, J = 15.8, 8.7, 8.2 Hz, 2H), 1.31 (d, J = 7.0 Hz, 3H). Exchangeable proton absent

**Example 25: 4-((S)-1-((2R,4R)-1-((5-(4-carbamoyl-2-methylphenyl)pyridin-3-yl)methyl)-4-hydroxypyrrolidine-2-carboxamido)ethyl)benzoic acid (Compound 25)**

Compound 25 was synthesised by a process analogous to Example 24 using Intermediate 3 and 3-Methyl-4-(4,4,5,5-tetramethyl-1,3,2-dioxaborolan-2-yl)benzamide to afford Compound 25. LC/MS (Method D): *m/z* 503 $[M+H]^+$, 1.21 min. $^1H$ NMR (400 MHz, DMSO-$d_6$) δ 8.80 (d, *J* = 7.8 Hz, 1H), 8.64 (d, *J* = 2.0 Hz, 1H), 8.43 (d, *J* = 2.1 Hz, 1H), 8.00 (t, *J* = 2.1 Hz, 1H), 7.95 (s, 1H), 7.87 - 7.56 (m, 4H), 7.37 (s, 1H), 7.11 - 6.92 (m, 3H), 4.75 (p, *J* = 7.1 Hz, 1H), 4.66 - 4.33 (m, 4H), 4.26 (s, 1H), 3.68 - 3.51 (m, 1H), 3.49 - 3.25 (m, 1H), 2.84 - 2.60 (m, 1H), 2.09 (s, 3H), 1.95 (d, *J* = 13.9 Hz, 1H), 1.28 (d, *J* = 7.0 Hz, 3H). Exchangeable proton absent

**Example 26: 3-(4-carbamoyl-2-methylphenyl)-5-(((R)-2-(((S)-1-(4-carboxyphenyl)ethyl)carbamoyl)pyrrolidin-1-yl)methyl)pyridine 1-oxide (Compound 26)**

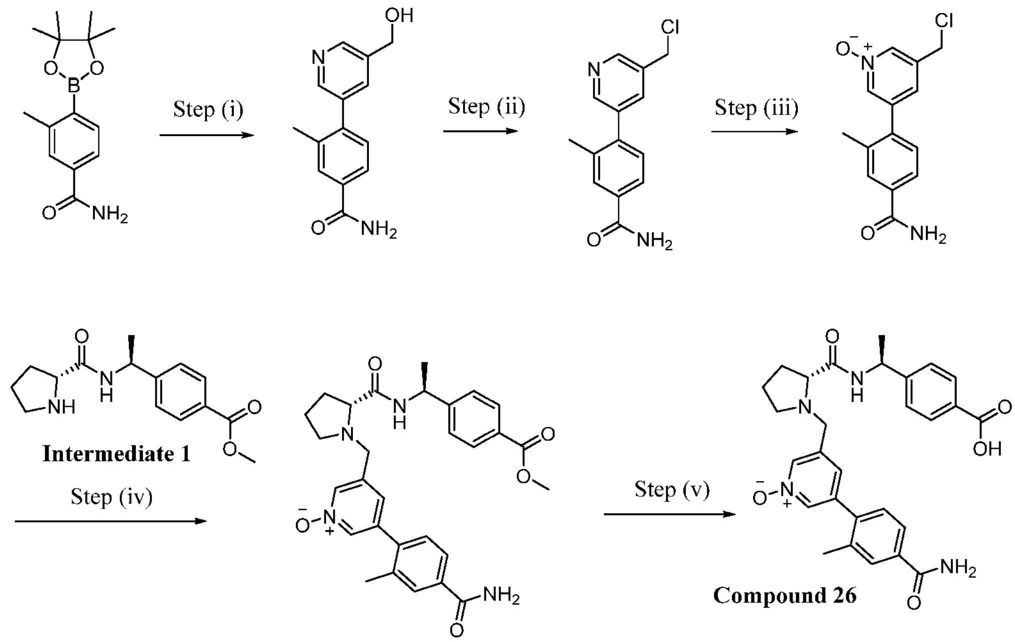


**Step (i):** To a suspension of (4-carbamoyl-2-methylphenyl)boronic acid, pinacol ester (4.00 g, 15.3 mmol, 1.0 eq.), 5-bromo-3-pyridinemethanol (2.88 g, 15.3 mmol, 1 eq.), and $CH_3COOK$ (4.50 g, 45.9 mmol, 3 eq.) in dioxane/$H_2O$ (64 mL/16 mL) under $N_2$, was added Pd(dppf)$Cl_2$(1.12 g, 1.53 mmol, 0.1 eq.). The reaction mixture was stirred at 85 °C for 16 hours, and the mixture was then filtered and concentrated. The residue was purified by column chromatography on silica gel (eluent - DCM:MeOH = 20:1 → 10:1) to afford 4-(5-(hydroxymethyl)pyridin-3-yl)-3-methylbenzamide (2.5 g, 68%) as a yellow solid. LC/MS: (Method E): m/z 243 $[M+H]^+$, 0.80 min.

**Step (ii):** To a solution of 4-(5-(hydroxymethyl)pyridin-3-yl)-3-methylbenzamide (1.20 g, 4.9 mmol, 1.0 eq.) in DCM (30.00 mL), was added $SOCl_2$ (5.83 g, 49.0 mmol, 10.0 eq.). The mixture was stirred at RT for 1 hour, filtrated and concentrated, and the residue was purified by column chromatography on silica gel (eluent - DCM:MeOH = 20:1 → 10:1) to afford 4-(5-(chloromethyl)pyridin-3-yl)-3-methylbenzamide (1.0 g, 78.7%) as a yellow solid. LC/MS: (Method E): m/z 261 $[M+H]^+$, 2.73 min.

**Step (iii):** To a suspension of 4-(5-(chloromethyl)pyridin-3-yl)-3-methylbenzamide (500.0 mg, 1.92 mmol, 1.0 eq.) in DCM (15 mL), was added m-CPBA (663.5 mg, 3.84 mmol, 2.0 eq.). The reaction mixture was stirred at RT for 1 hour, concentrated *in vacuo,* and the residue purified by column chromatography on silica gel (eluent - DCM:MeOH = 20:1 → 15:1) to afford 3-(4-carbamoyl-2-methylphenyl)-5-(chloromethyl)pyridine 1-oxide (500 mg, 94%) as a light yellow solid. LC/MS: (Method E): m/z 277 $[M+H]^+$, 2.30 min.

**Step (iv):** To a solution of 3-(4-carbamoyl-2-methylphenyl)-5-(chloromethyl)pyridine 1-oxide (200 mg, 0.72 mmol, 1.0 eq) in MeCN (12 mL) and DMF (2 mL) was added Intermediate 1 (226 mg, 0.72 mmol, 1.0 eq.), $NaHCO_3$ (182 mg, 2.17 mmol, 3.0 eq.), and NaI (108 mg, 0.72 mmol, 1.0 eq.). The resulting mixture was stirred at 70 °C for 4 hours and then filtrated and concentrated, and the residue purified by column chromatography on silica gel (eluent - DCM:MeOH = 30:1 → 15:1) to give 3-(4-carbamoyl-2-methylphenyl)-5-(chloromethyl)pyridine 1-oxide (311 mg, yield: 89%) as a white solid.

LC/MS: (Method E): m/z 517 $[M+H]^+$, 2.50 min.

**Step (v):** To a solution of compound 3-(4-carbamoyl-2-methylphenyl)-5-(chloromethyl)pyridine 1-oxide (330 mg, 0.60 mmol, 1.0 eq.) in methanol (10 mL), was added LiOH (1.8 mL, 1.80 mmol, 3.0 eq.). The mixture was stirred at RT for 2 days and then diluted with water (5 mL), acidified with 1 N HCl to pH ~ 5, and the solvent removed *in vacuo.* The residue was purified by prep-HPLC (Method E) to afford Compound 26 (120 mg, yield: 40.0%) as a white solid. LC/MS: (Method E): m/z 503$[M+H]^+$, 1.20 min. 1H NMR (400 MHz, Methanol-d4): δ 8.51-8.48 (m, 1H), 8.18 (s, 1H), 7.87 (dd, J = 8.0, 2.0 Hz, 2H), 7.80 (s, 1H), 7.73 (d, J = 8.0 Hz, 1H), 7.63-7.60 (m, 1H), 7.23 (d, J = 8.0 Hz, 2H), 6.98 (d, J = 7.6 Hz, 1H), 4.65-4.59 (m, 1H), 4.42 - 4.28 (m, 2H), 3.90 (s, 1H), 3.48-3.43 (m, 1H), 2.76-2.69 (m, 1H), 2.34-2.27 (m, 1H), 2.21-2.14 (m, 4H), 2.13-2.07 (m, 1H), 1.44 (d, J = 7.2 Hz, 3H).

**Example 27: 4-(1-(2-((2'-methyl-4'-sulfamoyl-[1,1'-biphenyl]-3-yl)methyl)-2-azabicyclo[2.1.1]hexane-1-carboxamido)cyclopropyl)benzoic acid (Compound 27)**

Compound 27 was synthesized by a process analogous to Example 23 using methyl 4-(1-aminocyclopropyl) benzoate and Intermediate 12 to give Compound 27 LC/MS: (Method A): 546 $[M+H]^+$ at 1.40min. Chiral HPLC: Product purity was confirmed at 11.73min 1H NMR: (400MHz, DMSO) 1.13 (m, 2H), 1.23 (m, 2H), 1.85-1.78 (m, 4H), 2.32 (s, 3H), 2.67 (s, 3H), 3.63 (s, 2H), 7.17-7.15 (d, 2H, J=8Hz), 7.26-7.25 (d, 1H, J=6.4Hz), 7.50-7.37 (m, 6H), 7.76-7.69 (m, 4H), 8.61 (s, 1H), 12.83 (bs, 1H).

**Example 28: (S)-4-(1-(2-((4'-carbamoyl-2'-methyl-[1,1'-biphenyl]-3-yl)methyl)-2-azabicyclo[2.1.1]hexane-1-carboxamido)ethyl)benzoic acid (Compound 28)**

Compound 28 was synthesized by a process analogous to Example 23 using Intermediate 13 to give Compound 28 LC/MS: (Method A): m/z 498 (ES+) at 1.27min.

Chiral HPLC: Product purity was confirmed at 8.83min.

1H NMR: (400MHz, DMSO): 1.39-1.37 (d, 3H, J= 7.2Hz), 1.79 (m, 4H), 2.28 (s, 3H), 2.70-2.61 (m, 3H), 3.57 (s, 2H), 5.06-5.02 (t, 1H, J= 7.4Hz), 7.29-7.24 (m, 2H), 7.46-7.36 (m, 6H), 7.82-7.75 (m, 4H), 7.97 (s, 1H), 8.30 (s, 1H), 12.80 (s, 1H).

**Example 29: 4-(1-(2-((4'-carbamoyl-2'-methyl-[1,1'-biphenyl]-3-yl)methyl)-2-azabicyclo[2.1.1]hexane-1-carboxamido)cyclopropyl)benzoic acid (Compound 29)**

Compound 29 was synthesized by a process analogous to Example 23 using methyl 4-(1-aminocyclopropyl) benzoate and Intermediate 13 to give Compound 29 LC/MS: (Method A): m/z 510 $[M+H]^+$ at 1.23 min.

1H NMR: (400MHz, DMSO): 1.14 (s, 2H), 1.24 (s, 2H), 1.78 (d, 2H, J= 3.6Hz), 1.85(s, 2H), 2.29 (s, 3H), 2.61 (s, 1H), 2.67 (s, 2H), 3.62 (s, 2H), 7.18-7.30 (m, 4H), 7.36-7.48 (m, 4H), 7.74 (d, 3H, J= 8.0Hz), 7.82 (s, 1H), 7.98 (s, 1H), 8.65 (s, 1H), 12.82 (s, 1H).

**Example 30: (R)-4-(1-(1-((2'-methyl-4'-sulfamoyl-[1,1'-biphenyl]-3-yl)methyl)pyrrolidine-2-carboxamido)cyclopropyl)benzoic acid (Compound 30)**

Compound 30 was synthesized by a process analogous to Example 23 using (tert-butoxycarbonyl)-D-proline, methyl 4-(1-aminocyclopropyl)benzoate and Intermediate 12 to give Compound 30. LC/MS: (Method A): m/z 534 $[M+H]^+$, at 1. 18min.

Chiral HPLC: Product purity was confirmed at 11.23 min.

1H NMR: (400MHz, DMSO): 1.06-1.04 (d, 2H, J= 4.8Hz), 1.19-1.18 (m, 2H), 1.78-1.77 (d, 3H, J= 2.8Hz), 2.14-2.12 (m, 1H), 2.29 (s, 3H), 2.38-2.36 (m, 1H), 3.12-3.11 (m, 2H), 3.62-3.59 (d, 1H, J= 12.8Hz), 3.85-3.82 (d, 1H, J= 13.2Hz), 7.11-7.09 (d, 2H, J= 8.4Hz), 7.28-7.27 (d, 1H, J= 6.8Hz), 7.44-7.35 (m, 6H), 7.7o-7.67 (m, 1H), 7.78-7.75 (q, 3H), 8.48 (s, 1H), 12.77 (s, 1H).

**Example 31: (R)-4-(1-(1-((4'-carbamoyl-2'-methyl-[1,1'-biphenyl]-3-yl)methyl) pyrrolidine-2-carboxamido)cyclopropyl)benzoic acid (Compound 31)**

Compound 31 was synthesized by a process analogous to Example 23 using (tert-butoxycarbonyl)-D-proline, methyl 4-(1-aminocyclopropyl)benzoate and Intermediate 12 to give Compound 31. LC/MS: (Method A): m/z 498 $[M+H]^+$ at 1.12min.

Chiral HPLC: Product purity was confirmed at 8.98min

1H NMR: (400MHz, DMSO) 1.20 (m, 1H), 1.23 (m, 1H), 1.77 (s, 3H), 2.08-2.10(m, 1H), 2.27 (s, 3H), 2.67 (s, 2H),

3.04-3.34 (m, 3H), 3.60 (d, 1H, J = 13.2Hz), 3.82 (d, 1H, J= 12.8Hz), 7.06 (d, 2H, J= 8.4Hz), 7.26 (d, 2H, J= 7.6Hz), 7.35-7.42 (m, 4H), 7.74 (d, 3H, J= 8Hz), 7.80 (s, 1H), 7.99 (s, 1H), 8.45 (s, 1H), 12.78 (s, 1H).

**Example 32: (S)-4-(1-(2-((2'-methyl-4'-sulfamoyl-[1,1'-biphenyl]-3-yl)methyl)-2-azabicyclo[2.1.1]hexane-1-carboxamido)ethyl)benzoic acid (Compound 32)**

Compound 32 was synthesized by a process analogous to Example 23 using Intermediate 12 to give Compound 32. LC/MS: (Method A): Product m/z 534 (ES+) at 1.27min.

Chiral HPLC: product purity was confirmed at 12.16min.

1H NMR: (400MHz, DMSO) 1.39-1.37 (d, 3H, J= 7.2Hz), 1.82-1.79 (m, 4H), 2.31 (s, 3H), 2.18-2.61 (m, 3H), 3.61-3.53 (m, 2H), 7.26-7.24 (m, 1H), 7.47-7.37 (m, 8H), 7.72-7.70 (m, 1H), 7.80-7.76 (m, 3H), 8.28-8.26 (d, 1H, J= 8.4Hz), 12.86 (s, 1H).

**Example 33: 4-((S)-1-((R)-1-((4',5-dihydroxy-2'-methyl-[1,1'-biphenyl]-3-yl)methyl)pyrrolidine-2-carboxamido)ethyl)benzoic acid (Compound 33)**

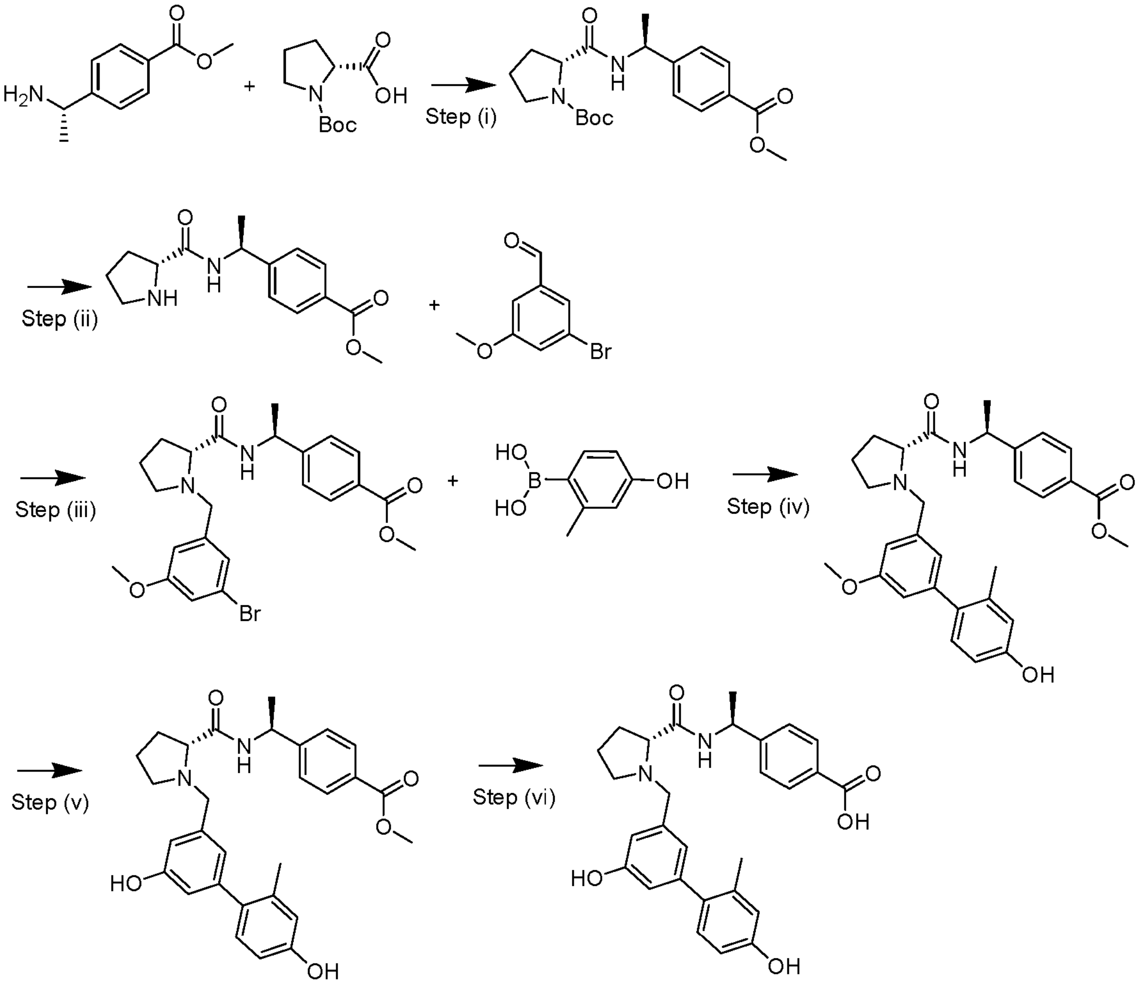


Compound 33

**Step (i):** To a solution of (tert-butoxycarbonyl)-D-proline (5.0 g, 27.90mmol) in DMF (50 mL) was added HATU (21.22 g, 55.8 mmol, 2 *eq)* and DIPEA (24.15 ml, 139.5 mmol, 5 *eq*), the mixture was stirred at 25°C for 0.5hr. Methyl (S)-4-(1-aminoethyl) benzoate (6.01 g, 27.90mmol, 1 *eq*) was added and the resulting mixture was stirred at 25°C for 1hr. After completion the reaction mixture was diluted with $H_2O$ (500 mL) and extracted with EA (2×150 mL), combined organic layers were dried over anhydrous sodium sulfate, filtered and concentrated under reduced pressure to give residue. The residue was purified by flash silica gel chromatography Eluent of 0~50% Ethyl acetate/ hexenes gradient to give the tert-butyl (R)-2-(((S)-1-(4-(methoxycarbonyl) phenyl)ethyl)carbamoyl)pyrrolidine-1-carboxylate (8.5 g, 79.31%) as yellow oil.

TLC: (5.0:5.0, EA / hexene, RF: 0.7).

Mass (ESI +ve): 277.0 [M-100].

LCMS: 98.91% (LCMS Method H), RT: 2.949 mins, 254 nm.

1H NMR: (400 MHz, DMSO): 1.20-1.25(m, 9H),1.34-1.47(m, 3H), 1.78-2.13(m,4H), 3.27-3.35(m,2H), 3.83(m,3H), 4.06-4.15(m,1H), 4.93-5.00(m,1H), 7.4-7.52 (m,2H), 7.8-7.90(m,2H), 8.36-8.38(d, J=6.8Hz,1H).

**Step (ii):** To a solution of tert-butyl (R)-2-(((S)-1-(4-(methoxycarbonyl) phenyl) ethyl) carbamoyl) pyrrolidine-1-carboxylate (8.5 g, 2.85 mmol, 1 *eq)* in DCM (100 mL) was added HCl in dioxane (4.0 M, 85 mL). The mixture was stirred at 15 °C for 1 hr. Starting material was consumed completely, then the reaction mixture was concentrated under reduced pressure to remove HCl/Dioxane. Dioxane (30 ml) was further added to the residue and concentrated under reduced pressure to give the methyl 4-((S)-1-((R)-pyrrolidine-2-carboxamido) ethyl) benzoate (7.5 g, crude, HCL salt) as a brown solid.

TLC: (5.0:5.0, EA / hexene, RF: 0.8).

Mass (ESI +ve): 277.0 [M+1].

LCMS: 89.10% (LCMS Method H), and RT: 2.184mins, 230 nm.

1H NMR: (400 MHz, DMSO): 1.26-1.41(m, 6H),1.89(s, 3H), 2.33-2.35(m,2H),3.63-3.90(m,9H),3.18(m,3H),4.47-3.84(m,6H),4.21(m,1H)4.97-5.0(m,1H),7.50-7.52(d, J=4.4Hz, 2H),7.90-7.94(d J=16.0Hz ,2H),8.51(s,1H), 9.29-9.30(d,1H).10.03(s,1H).

HPLC Analysis: 99.41% 5.81RT, 240.0 nm, 4.0 nm

**Step (iii):** To a solution of methyl 4-((S)-1-((R)-pyrrolidine-2-carboxamido) ethyl) benzoate (7.5 g, 27.14mmol ,1 eq, HCl), 3-bromo-5-methoxybenzaldehyde (7.01 g, 32.57mmol, 1.2 *eq*) in DCE (40 mL) and DMF (40 mL) was added TEA (3.76ml, 27.14 mmol, 1 *eq*) and the mixture was stirred at 30°C for 1hr. AcOH (1.63ml, 27.14mmol,1 *eq)* and $NaBH_3CN$ (3.41g, 54.28mmol, 2 *eq*) were added and the resulting mixture was stirred at 30°C for 12hr. Starting material was consumed completely and one main peak with desired mass was detected. Then the mixture was diluted with $H_2O$ (500 mL), adjusted pH to 9 with $NaHCO_3$ solid and extracted with EA (2×200 mL). The combined organic layers were dried over anhydrous sodium sulphate, filtered and concentrated under reduced pressure to give residue. The residue was purified by flash silica gel chromatography Eluent of 0~10% MEOH/ DCM gradient to give the pure compound methyl 4-((S)-1-((R)-1-(3-bromo-5-methoxybenzyl) pyrrolidine-2-carboxamido) ethyl) benzoate (5.40 g, 39.76%) as white solid.

TLC: (1.0:9.0, MeOH / DCM, RF: 0.2).

Mass (ESI +ve): 475.2 [M+1].

LCMS: 95.77% (LCMS Method H), RT: 3.656 mins, 202 nm.

1H NMR: (400 MHz, DMSO): 6.92(s,1H),7.038(s,1H),7.14(s,1H), 7.42-7.44 (d, *J* = 8.0 Hz, 2 H), 7.89-7.91 (d, *J* = 8.0 Hz, 2 H), 8.18-8.20(d, *J* = 8.0 Hz, 1 H), 1.10-1.14(m, 4 H), 1.34-1.36 (d, 3 H), 1.65-1.73(m, 3H),2.020-2.068(m, 1 H), 2.33-2.36(m, 1H), 2.72-3.10(m, 6H), 3.47-3.50 (d, *J* =12.0 Hz, 1H), 2.70-3.85 (m, 6 H), 4.86-4.90 (m, 1 H).

1H NMR: (400 MHz, CDCl3): 6.79(s,1H),7.017(s,1H), 7.092 (s,1H), 7.36-7.39(d, *J* = 8.0Hz, 2 H), 7.68 (s,1 H), 8.037-8.058(d, *J* = 8.0 Hz, 2 H), 1.16-1.35(m,6H),1.36-1.46(s,3H), 1.70-1.84(m, 3 H),2.24 (m, 1H), 2.44-2.46(s, 1H),2.64(s,1H),2.84-3.14(m,5H), 3.26(m, 1H),3.54-3.57(m,1H), 3.72-4.002(m. 7H), 5.056-5.10 (m, 1 H).

HPLC Analysis: 99.59 %, RT,6.57 mins, at 239.0 nm.

**Step (iv):** To a solution of methyl 4-((S)-1-((R)-1-(3-bromo-5-methoxybenzyl) pyrrolidine-2-carboxamido) ethyl) benzoate (5.3 g, 11.15mmol 1 eq), (4-hydroxy-2-methylphenyl) boronic acid (2.55 g, 16.72mmol ,1.5 eq) in dioxane (70 mL) and $H_2O$ (15 mL) was added $K_2CO_3$ (3.078 g, 11.15mmol ,2 eq) and Pd(dppf)$Cl_2$ (0.816 g, 1.11mmol 0.1 *eq).* The mixture was stirred at 90 °C for 12 hr under $N_2$. Starting material was consumed completely and one new spot formed. The reaction mixture was diluted with $H_2O$ (100 mL) and extracted with EA (2×100 mL). The combined organic layers were dried over anhydrous sodium sulfate, filtered and concentrated under reduced pressure to give residue . The residue was purified by flash silica gel chromatography Eluent of 0~05% MeOH/ DCM gradient to give the methyl 4-((S)-1-((R)-1-((4'-hydroxy-5-methoxy-2'-methyl-[1,1'-biphenyl]-3-yl) methyl) pyrrolidine-2-carboxamido) ethyl) benzoate (3.5 g, 66%) as a white solid.

TLC: (5.0:5.0, EA / hexene, RF: 0.2).

Mass (ESI +ve): 503.2 [M+1].

LCMS: 92.39% (LCMS Method H), RT: 3.296mins, 230nm.

1H NMR: (400 MHz, DMSO): 6.62-6.70(m,3H),6.82-6.85 (d, *J* = 11.2 Hz, 2 H), 7.00-7.02 (d, *J* = 8.0 Hz, 1 H), 7.40-7.42(d, *J* = 8.0 Hz, 2 H), 7.86-7.88(d, *J* = 8.0 Hz, 2 H), 8.14-8.16(d, *J* = 8.0 Hz, 1 H), 1.23-1.33(m, 4 H),1.72 (m, 3 H), 2.05(m, 1H),2.16(s,3H),2.33-2.39(m, 1 H), 3.087-3.097(m, 2H),3.49-3.52(m,1H), 3.76-3.94(m, 7H), 4.85-4.90 (m, 1 H).

1H NMR: (400 MHz, CDCl3): 6.75-6.85(m,5H),7.08-7.10 (d,1H), 7.30-7.39(m,2H), 7.87-8.06(m,3H), 1.30-1.48(m,3H),1.72-1.89 (m,4H), 2.24(s, 4H),2.48(m,1H),3.18(m, 1 H), 3.34-3.35(m, 2H),3.49-3.52(m,1H), 3.62-3.66(m, 1H),3.85-3.95(m,6H).

HPLC Analysis: 89.40% RT 7.31 mins, at 239.0 nm.

**Step (v):** To a solution of methyl 4-((S)-1-((R)-1-((4'-hydroxy-5-methoxy-2'-methyl-[1,1'-biphenyl]-3-yl) methyl)

pyrrolidine-2-carboxamido) ethyl) benzoate (3.7 g,7.36 mmol, 1 eq) in DCM (100 mL) was added dropwise BBr3 (1.0 M solution in DCM) (40.52 ml, 5.5 eq) at -60°C. After addition, the mixture was stirred at this temperature for 30 min, and then the resulting mixture was stirred at 15°C for 2 hr. Starting material was consumed completely. Then reaction mixture was quenched by addition MeOH (200 mL) at -60°C, then the mixture was concentrated under reduced pressure to give the residue. The residue was purified by flash silica gel chromatography Eluent of 0~20% MeOH/ DCM gradient to give the methyl 4-((S)-1-((R)-1-((4',5-dihydroxy-2'-methyl-[1,1'-biphenyl]-3-yl) methyl) pyrrolidine-2-carboxamido) ethyl) benzoate (2.80 g, 77.85%) as a white solid.

TLC: (4.0:1.0:5.0 EA / MEOH/DCM, RF: 0.4).

Mass (ESI +ve): 489.2 [M+1].

LCMS: 84.75 % (LCMS Method H), RT: 2.921mins, 254.0 nm.

1H NMR: (400 MHz, DMSO): 6.57-6.58(m,2H), 6.64(d,1H), 6.80-6.84(m,3H),7.17-7.23 (d, *J* = 8.0 Hz,2H), 7.81-7.89(d, *J* = 8.0 Hz, 2 H), 9.01-9.028(d, J= 7.2Hz, 1 H), 1.35-1.37(m, 3 H),1.90-1.93(m, 2 H), 2.05-2.15(m, 4H),2.70(m,1H),3.18(s, 4 H), 3.32(m, 1H),3.60-3.62(s,1H), 4.23-4.31(m,3H), 4.88-4.31 (m, 1 H).

CHIRAL HPLC: 85.25%, RT 8.23mins, at 234.0 nm.

**Step (vi):** To a solution of methyl 4-((S)-1-((R)-1-((4',5-dihydroxy-2'-methyl-[1,1'-biphenyl]-3-yl) methyl) pyrrolidine-2-carboxamido) ethyl) benzoate (2.80 g, 5.73mmol) in EtOH (10mL) and THF (30 mL) was added LiOH•$H_2O$ (2.40 g, 57.31mmol) in $H_2O$ (30 mL). The mixture was stirred at RT for 15hr. RM LCMS showed starting material remained. Then again LiOH•$H_2O$ (2.65g, 63.04mmol) was added and the mixture was stirred at RT for 24hr. Starting material was consumed completely then reaction mixture was concentrated under reduced pressure to remove EtOH and THF. Then the mixture was diluted with $H_2O$ (20 mL), adjusted pH to 5 with HCl solution (2.0 M) and the mixture was concentrated under reduced pressure to give crude product. The crude product was purified by prep-HPLC (Method F) to give 4-((S)-1-((R)-1-((4',5-dihydroxy-2'-methyl-[1,1'-biphenyl]-3-yl)methyl)pyrrolidine-2-carboxamido)ethyl)benzoic acid (Compound 33) (500 mg, 18.38%) as a white solid.

TLC: (1.0:9.0, Methanol / DCM, RF: 0.1).

Mass (ESI +ve): 475.0 [M+H].

LCMS: 100.0% (LCMS Method I), RT: 2.747mins, 304.0 nm.

LCMS: 100.0% (LCMS Method H), RT: 2.080 mins, 304.0 nm.

HPLC Analysis: 100.0% [RT 2.85 mins, at 220.0 nm].

1H NMR: (400 MHz, MeOD): 6.63-6.70(m,5H),6.95-6.98(d, J=8.0Hz,1H), 7.288-7.307(d, J = 7.6 Hz,2H),7.94-7.97(d, J = 8.0 Hz,2H), 1.35-1.39(m, 3 H),1.87-1.97(m, 3 H), 2.17(m, 3H),2.34(m,1H),2.79-2.813(m, 1H), 3.40(m, 1H),3.53-3.55(m,1H), 3.86-3.95(m,1H).

1H NMR: (400 MHz, DMSO): 6.57-6.71(m,5H),6.976-6.996(d, J=8.0Hz,1H),7.368-7.388(d, J =8.0 Hz,2H),7.865-7.885(d, J = 8.0 Hz,2H),8.052-8.072(d, J = 8.0 Hz,1H), 9.40(s,1H),1.25-1.33(m, 3 H),1.70-1.73(m, 3 H), 2.066(m, 2H),2.166(s,3H),3.028(s, 1H), 3.08-3.12(m, 1H),3.70-3.77(m,2H), 3.86-4.90(m,1H).

**Example 34: 4-((S)-1-((R)-1-((4'-carbamoyl-5-hydroxy-2'-methyl-[1,1'-biphenyl]-3-yl)methyl)pyrrolidine-2-carboxamido)ethyl)-2-hydroxybenzoic acid (Compound 34)**

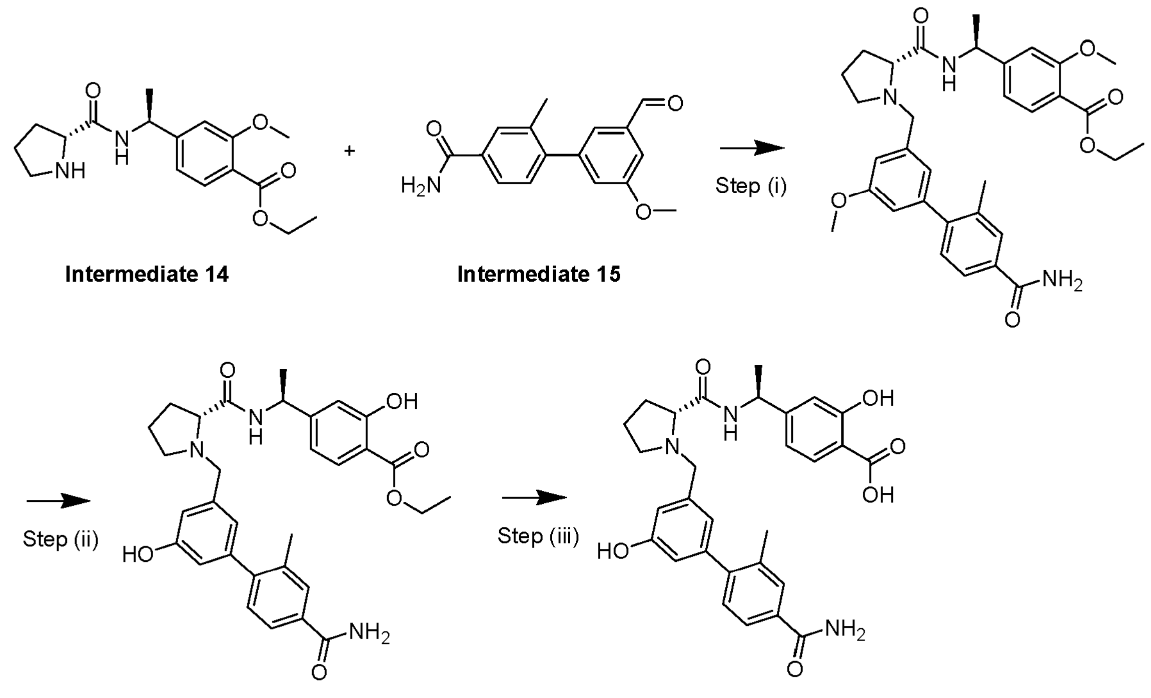


Intermediate 14

Intermediate 15

Compound 34

**Step (i):** To a solution of Intermediate 14 (160 mg, 448.38 umol, 1 *eq,* HCl), Intermediate 15 (144.89 mg, 538.05 umol, 1.2 *eq*) in DCE (2.5 mL) and DMF (2.5 mL) was added TEA (45.37 mg, 448.38 umol, 62.41 uL, 1 *eq*) and the mixture was stirred at 30°C for 1hr. AcOH (26.93 mg, 448.38 umol, 25.64 uL, 1 *eq*) and $NaBH_3CN$ (56.35 mg, 896.75 umol, 2 *eq*) were added, then the resulting mixture was stirred at 30°C for 12hr. LC-MS showed Intermediate 14 was consumed completely and one main peak with desired mass was detected. Then the combined mixture were diluted with $H_2O$ (20 mL), adjusted pH to 9 with $NaHCO_3$ solid and extracted with EtOAc (30 mL * 2). The combined organic layers were dried over anhydrous sodium sulfate, filtered and concentrated under reduced pressure to give residue. The residue was purified by prep-HPLC (HPLC method G) to give the compound c1 (102 mg, 174.07 umol, 38.82% yield, 97.9% purity) as yellow solid, which was confirmed by HNMR.

LCMS: $[M+1]^+$ = 574.2.

SFC: Rt = 1.404 min, ee = 100%

$^1$HNMR: (MeOD, 400 MHz) δ 7.80 (s, 1 H), 7.75 - 7.71 (m, 1 H), 7.67 (d, *J* = 8.0 Hz, 1H), 7.29 (d, *J* = 7.6 Hz, 1 H), 6.98 (d, *J* = 11.2 Hz, 2 H), 6.91 - 6.85 (m, 2 H), 6.79 (s, 1 H), 4.85 - 4.81 (m, 1 H), 4.30 (q, *J* = 6.8, 2 H), 3.86 - 3.71 (m, 8 H), 3.27 - 3.17 (m, 2 H), 2.61 - 2.49 (m, 1 H), 2.30 (s, 3 H), 2.27 - 2.13 (m, 1 H), 1.88 - 1.74 (m, 3 H), 1.39 - 1.29 (m, 6 H).

**Step (ii):** To a solution of compound c1 (230 mg, 400.92 umol, 1 *eq)* in DCM (8 mL) was added dropwise $BBr_3$ (502.20 mg, 2.00 mmol, 193.15 uL, 5 *eq)* in DCM (2 mL) at - 60°C. After addition, the mixture was stirred at this temperature for 30 min, and then the resulting mixture was stirred at 15°C for 2 hr. LC-MS showed compound c1 was consumed completely. The reaction mixture was quenched by addition MeOH (5 mL) at -60°C, then the mixture was warmed to room temperature and concentrated under reduced pressure to give the compound c2 (220 mg, crude) as yellow solid.

LCMS: $[M+1]^+$ = 546.2.

**Step (iii):** To a solution of compound c2 (220 mg, 403.21 umol, 1 *eq*) in EtOH (1 mL) and THF (5 mL) was added LiOH•$H_2O$ (50.76 mg, 1.21 mmol, 3 *eq*) in $H_2O$ (1 mL). The mixture was stirred at 30°C for 15hr. LCMS showed compound c2 remained. LiOH•$H_2O$ (50.76 mg, 1.21 mmol, 3 *eq*) was added and the mixture was stirred at 30°C for 15hr. LC-MS showed 9.1% of compound c2 remained and one main peak with desired mass was detected. The reaction mixture was concentrated under reduced pressure to remove EtOH and THF. Then the mixture was diluted with $H_2O$ (3 mL), adjusted pH to 5 by HCl (2M) and the mixture was concentrated under reduced pressure to give residue. The residue was purified by prep-HPLC (HPLC method H) to give 4-((S)-1-((R)-1-((4'-carbamoyl-5-hydroxy-2'-methyl-[1,1'-biphenyl]-3-yl)methyl) pyrrolidine-2-carboxamido)ethyl)-2-hydroxybenzoic acid (Compound 34) (111.7 mg, 214.09 umol, 53.10% yield, 99.2% purity) as a white solid, which was confirmed by HNMR.

LCMS (LCMS Method J): 1.25 min., m/z 518.34 [MH+]

SFC: Rt = 1.499 min, ee = 100%

$^1$HNMR (MeOD, 400 MHz) *δ* 7.79 - 7.71 (m, 2 H), 7.66 (dd, *J* = 1.6, 8.0 Hz, 1 H), 7.09 (d, *J* = 8.0 Hz, 1 H), 6.91 - 6.86 (m, 1 H), 6.82 (s, 1 H), 6.77 - 6.74 (m, 1 H), 6.69 (d, *J*= 1.2 Hz, 1 H), 6.58 (dd, *J* = 1.6, 8.0 Hz, 1 H), 4.84 - 4.80 (m, 1 H), 4.28 - 4.17 (m, 1 H), 4.14 - 4.06 (m, 1 H), 3.92 (dd, *J* = 4.8, 8.4 Hz, 1 H), 3.67 - 3.56 (m, 1 H), 3.20 - 3.06 (m, 1 H), 2.57 -2.42 (m, 1H),

2.22 (s, 3 H), 2.16 - 1.91 (m, 3 H), 1.36 (d, *J* = 7.2 Hz, 3 H).

**Example 35: 4-((S)-1-((R)-1-((4',5-dihydroxy-2'-methyl-[1,1'-biphenyl]-3-yl)methyl)pyrrolidine-2-carboxamido) ethyl)-2-hydroxybenzoic acid (Compound 35)**

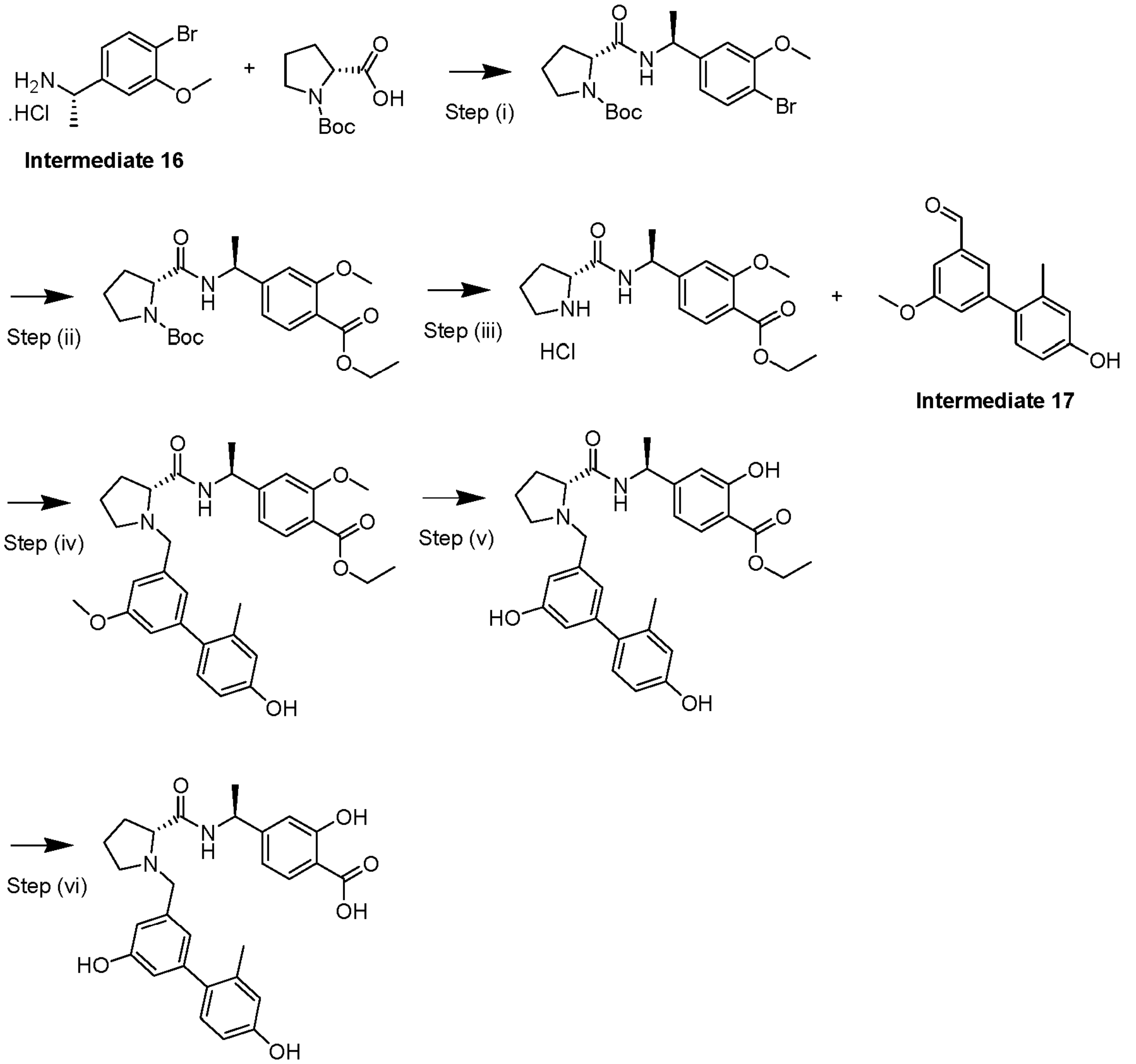


Intermediate 16

Intermediate 17

**Step (i):** To a solution of (tert-butoxycarbonyl)-D-proline (7.250 g, 0.027 mol, 1.0 *eq)* in DMF (72 mL) was added HATU (15.50 g, 0.04 mol, 1.5 *eq)* to a reaction mixture and reaction mixture was stirred for at 0°C for 15 min, addition DIPEA (13.94 ml, 0.08 mol, 3 *eq)* to a reaction mixture at 0°C, the mixture was stirred at RT for 30 min. , added (S)-1-(4-bromo-3-methoxyphenyl)ethan-1-amine.HCl salt (Intermediate 16) (5.85 g, 0.027 mol, 1 *eq,* HCl) was added and the resulting mixture was stirred at 25°C for overnight. LC-MS showed Intermediate 16 was consumed completely and one main peak with desired mass was detected. The reaction mixture was diluted with cooled $H_2O$ (800 mL) and extracted with EA (3×300 mL). The combined organic layers were dried over anhydrous sodium sulphate, filtered and concentrated under reduced pressure to give residue. The residue was purified by flash silica gel chromatography (Normal-Phase silica at 0 to 40.0% EA/Hexane) to give the tert-butyl (R)-2-(((S)-1-(4-bromo-3-methoxyphenyl) ethyl) carbamoyl) pyrrolidine-1-carboxylate (11.4 g, 98.10% yield, 96% purity) as a colourless gum.

TLC: (5.0:5.0, Hexane / EtOAc, RF: 0.4).

LCMS: 96.31% (LCMS Method H), RT: 3.161 mins, 254.0 nm.

Chiral HPLC:80.05% at 4.23 RT.

$^1$H NMR: (400 MHz, CDCl3) δ: 7.47-7.39 (s, 3H), 6.91-6.73 (m, 3H), 5.04 (s, 2H), 4.34 (s, 2H), 3.96-3.86 (s, 5H), 3.46-3.3 (m, 4H), 2.99 (s, 1H), 2.91 - 2.89 (s, 1H), 2.84-2.83 (s, 7H), 2.40-2.31 (m, 1H), 2.19-2.08 (m, 2H), 1.89- 1.63 (s, 5H), 1.58-1.22 (m, 25H).

**Step (ii):** To a solution of tert-butyl (R)-2-(((S)-1-(4-bromo-3-methoxyphenyl) ethyl) carbamoyl) pyrrolidine-1-carboxylate (11.4 g, 0.026 mol, 1 *eq)* in EtOH (120 mL) was added TEA (8.08 g, 0.08 mol, 11.1mL, 3 *eq)* and Pd(dppf) $Cl_2.CH_2Cl_2$ (2.12 g, 0.002 mol, 0.1 *eq).* The mixture was stirred at 100 °C for 16 hr under CO gas (125 PSI). On TLC showed tert-butyl (R)-2-(((S)-1-(4-bromo-3-methoxyphenyl) ethyl) carbamoyl) pyrrolidine-1-carboxylate was consumed completely and one main peak with desired mass was detected. The reaction mixture was filtered and the filtrate was concentrated under reduced pressure to give residue. The reaction mixture was diluted with $H_2O$ (1200 mL) and extracted with EA (3×400 mL). The combined organic layers were dried over anhydrous sodium sulphate, filtered and concentrated under reduced pressure to give residue. The residue was purified by flash silica gel chromatography (Normal-Phase silica at 0 to 49.0% EA/Hexane) to give the tert-butyl (R)-2-(((S)-1-(4-(ethoxycarbonyl)-3-methoxyphenyl) ethyl) carbamoyl) pyrrolidine-1-carboxylate (7.25 g, 59.37% yield) as a colourless gum.

TLC: (5.0:5.0, Hexane / EtOAc, RF: 0.4).

Mass (ESI +ve): 321.2 [M-100+1].

LCMS: 89.69 % (LCMS Method H), RT: 2.999 mins, 254.0 nm.

Chiral HPLC:98.91% at 6.16 RT.

$^1H$ NMR: (400 MHz, DMSO-D2O) δ:8.38-8.29 (m, 1H), 7.57-7.55 (s, 1H), 7.12-6.96 (m, 2H), 4.97-4.88 (m, 1H), 4.25-4.21 (d, 2H), 4.04-4.02 (s, 1H), 3.85-3.80 (d, 3H), 2.11 (m, 1H), 1.77 (s, 3H), 1.38 (s, 8H), 1.28-1.17 (m, 11H).

**Step (iii):** To a solution of tert-butyl (R)-2-(((S)-1-(4-(ethoxycarbonyl)-3-methoxyphenyl) ethyl) carbamoyl) pyrrolidine-1-carboxylate (7.25 g, 0.017 mol, 1 *eq)* in Dioxane (20 ml) was added HCl/dioxane (4.0 M, 120 mL). The mixture was stirred at RT for 1.5 hr. On TLC showed tert-butyl (R)-2-(((S)-1-(4-(ethoxycarbonyl)-3-methoxyphenyl) ethyl) carbamoyl) pyrrolidine-1-carboxylate was consumed completely and one main peak with desired mass was detected. The reaction mixture was concentrated under reduced pressure to remove HCl/Dioxane, added Dioxane (40 ml*2) reaction mixture was concentrated under reduced pressure to remove HCl/Dioxane to give the ethyl 2-methoxy-4-((S)-1-((R)-pyrrolidine-2-carboxamido) ethyl) benzoate. HCL salt (5.5 g, crude) as a brown gum.

TLC: (5.0:5.0, Hexane / EtOAc, RF: 0.2).

LCMS: 98.97 % (LCMS Method K), RT: 1.057 mins, 254.0 nm.

Chiral HPLC: 97.97% at 6.19 RT.

$^1H$ NMR: (400 MHz, DMSO-D2O) δ: 9.79 (s, 1H), 9.23-9.21 (d, 1H), 8.54 (s, 1H), 7.61-7.59 (d, 1H), 7.16 (s, 1H), 7.01-6.99 (d, 1H), 4.99-4.95 (m, 1H), 4.27-4.21 (q, 3H), 3.85 (s, 3H), 3.58 (s, 2H), 3.22-3.18 (m, 3H), 2.70 (s, 3H), 2.37-2.35 (m, 1H), 1.91 (s, 3H), 1.42-1.40 (d, 3H), 1.26-1.32 (t, 4H).

**Step (iv):** A mixture of ethyl 2-methoxy-4-((S)-1-((R)-pyrrolidine-2-carboxamido)ethyl)benzoate.HCL salt (5.50 g, 0.015 mol, 1 eq) was in DCE (50 ml), addition of TEA (4.66 g, 6.14 ml, 0.046 mol, 3 eq) at 0°C the mixture was stirred at 0°C for 10 minute, then after 4'-hydroxy-5-methoxy-2'-methyl-[1,1'-biphenyl]-3-carbaldehyde (Intermediate 17) (3.73 g, 0.015 mol, 1 eq) added in reaction mixture and mixture was stirred at 60°C for 3hr, After 3hr $NaBH_3CN$ (2.86 g, 0.046 mol, 3 eq) was added and then the resulting mixture was stirred at 30°C for overnight. LC-MS showed ethyl 2-methoxy-4-((S)-1-((R)-pyrrolidine-2-carboxamido)ethyl)benzoate.HCL salt was consumed completely and one main peak with desired mass was detected. Then the mixture was diluted with $H_2O$ (600 mL), extracted with EA (400 mL * 3). The combined organic layers were dried over anhydrous sodium sulphate, filtered and concentrated under reduced pressure to give residue. The residue was purified by normal phase column chromatography (Normal-Phase neutral activated alumina at 5.0% to 30.0% Methanol/DCM) to give ethyl 4-((S)-1-((R)-1-((4'-hydroxy-5-methoxy-2'-methyl-[1,1'-biphenyl]-3-yl) methyl) pyrrolidine-2-carboxamido) ethyl)-2-methoxybenzoate (3.77 g, 44.72% yield, 81.66% purity) as an off-white solid.

TLC: (7.0:3.0, Hexane / EtOAc, RF: 0.4).

Mass (ESI +ve): 547.33 (M+1)

LCMS: 81.66% (LCMS Method H), RT: 3.382 min, 254 nm.

CHIRAL HPLC: 83.20% RT 8.08 mins at 232.0 nm.

$^1H$ NMR: (400 MHz, DMSO-D2O) δ: 1.21-1.1.32 (m, 6H), 1.72-1.75 (m, 3H), 2.01-2.06 (m, 1H), 2.18 (s, 3H), 2.35-2.39 (m, 1H), 3.05-3.13 (m, 2H), 3.52-3.56 (m, 1H), 3.79-3.81 (m, 6H), 4.21-4.26 (m, 2H), 4.83-4.86 (m, 1H), 6.64-6.69 (m, 2H), 6.73 (s, 1H), 6.84-6.87 (d, 2H, J=10 Hz), 6.91-6.93 (d, 1H, J=7.6 Hz), 7.02-7.07 (m, 2H), 7.55-7.57 (d, 1H, J=7.6 Hz), 8.14-8.16 (d, 1H, J=7.6 Hz), 9.37 (s, 1H).

**Step (v):** To a solution of ethyl 4-((S)-1-((R)-1-((4'-hydroxy-5-methoxy-2'-methyl-[1,1'-biphenyl]-3-yl) methyl) pyrrolidine-2-carboxamido) ethyl)-2-methoxybenzoate (3.77 g, 0.006 mol, 1 *eq*) in DCM (108 mL) was added $BBr_3$ (1.0 M solution in DCM) (8.63 g, 34.42 ml, 0.034 mol, 5 *eq*) to a reaction mixture at -78°C, the mixture was stirred at -78°C for 1hr. Then the resulting mixture was stirred at RT for 2hr. On TLC showed ethyl 4-((S)-1-((R)-1-((4'-hydroxy-5-methoxy-2'-methyl-[1,1'-biphenyl]-3-yl) methyl) pyrrolidine-2-carboxamido) ethyl)-2-methoxybenzoate was consumed completely and one main peak with desired mass was detected. The reaction mixture was quenched by addition MeOH (197.5 mL) slowly dropwise at -78°C, then the mixture was warmed to room temperature and concentrated under reduced pressure to give the ethyl 4-((S)-1-((R)-1-((4',5-dihydroxy-2'-methyl-[1,1'-biphenyl]-3-yl) methyl) pyrrolidine-2-carboxamido) ethyl)-2-hydroxybenzoate (5.04 g, crude) as yellow solid.

TLC: (8.0:2.0, Hexane / EtOAc, RF: 0.2).
Mass (ESI +ve): 519.4 [M+1].
LCMS: 52.0% (LCMS Method L), RT: 2.838 mins, 254 nm.
**Step (vi):** To a solution of ethyl 4-((S)-1-((R)-1-((4',5-dihydroxy-2'-methyl-[1,1'-biphenyl]-3-yl) methyl) pyrrolidine-2-carboxamido) ethyl)-2-hydroxybenzoate (5.04 g, 0.009 mol, 1 eq) in EtOH (35 mL) and THF (170 mL) was added LiOH•$H_2O$ (3.05 g, 0.07 mol, 7.5 eq) in $H_2O$ (35 mL). The mixture was stirred at 30 °C for 7 days. LC-MS showed ethyl 4-((S)-1-((R)-1-((4',5-dihydroxy-2'-methyl-[1,1'-biphenyl]-3-yl) methyl) pyrrolidine-2-carboxamido) ethyl)-2-hydroxybenzoate was consumed completely and one main peak with desired mass was detected. The reaction mixture was concentrated under reduced pressure to remove EtOH and THF. Then the mixture was cool down at 0°C diluted with $H_2O$ (20 mL), adjusted pH to 5 by HCl (2.0 M) at 0°C and solid was followed then filter through vacuum pump, the mixture was concentrated under reduced pressure to give residue. The residue was purified by prep-HPLC (HPLC method I) to give 4-((S)-1-((R)-1-((4',5-dihydroxy-2'-methyl-[1,1'-biphenyl]-3-yl)methyl)pyrrolidine-2-carboxamido)ethyl)-2-hydroxybenzoic acid (Compound 35) (472.5 mg, 9.91% yield, 100.0% purity) as a white solid.
TLC: (1.0:9.0, Methanol / DCM, RF: 0.1).
Mass (ESI +ve): - 491.2 (M+1)
LCMS: 100.0% (LCMS Method L), RT: 2.415 min, 304.0 nm.
LCMS: 99.60% (LCMS Method H), RT: 2.156 min, 304.0, nm.
Chiral HPLC:100.0% at 6.75 RT.
$^1$H NMR: (400 MHz, MeOD) δ: 7.76-7.78 (d, 1H), 6.86-6.88 (d, 1H), 6.79-6.80 (d, 2H), 6.72 (s, 2H), 6.66 (s, 1H), 6.60 (s, 2H), 4.85-4.87 (m, 1H), 4.18-4.21 (d, 1H), 4.08-4.11 (d, 1 H), 3.94 (s, 1H), 3.60 (s, 1H), 3.15 (s, 1H), 2.49 (s, 1H), 2.12 (s, 4H), 1.99 (s, 2H), 1.37-1.39 (d, 3 H).
$^1$H NMR: (400 MHz, DMSO-D2O) δ: 9.52 (s, 1H), 9.35 (s, 1H), 8.14-8.31 (m, 1H), 7.64-7.66 (d, 1H), 6.93-6.95 (d, 1H), 6.72 (s, 3H), 6.11-6.65 (m, 4H), 4.76-4.79 (m, 1H), 3.90 (s, 1H), 3.72 (s, 1H), 3.2-3.5 (m, 3H), 2.33-2.36 (s, 1H), 2.12 (s, 3H), 1.75-1.85 (m, 3H), 1.29-1.28 (d, 4H).

## Biological Activity Assay

***Cloning, Baculovirus generation, large scale infection of HEK293 cells and membrane preparation:*** Human prostaglandin E2 receptor 4 ($EP_4$) was cloned into pBacMam expression vector (GeneScript, UK). Transposition of $EP_4$ DNA was performed using Invitrogen's Bac-to-Bac Baculovirus Expression Systems. P0 baculovirus was generated by transfecting SF9 Cells with bacmid DNA using Cellfectin II transfection reagent (ThermoFisher Scientific, UK). Following P0 generation P1 virus was then generated ready for large scale infection and membrane preparation. HEK293 cells were grown in DMEM (ThermoFisher Scientific, UK), supplemented with 10% heat inactivated fetal bovine serum (FBS). Cells were infected at a seeding density of 3.5 million cells/ mL in 500 $cm^3$ flasks at 5% v/v $EP_4$ Bacman. Expression was carried out over a 36 hour period at 37°C with 5% $CO_2$. The cells were removed using PBS and a cell scrapper. The cell culture was centrifuged at 2500 RPM for 10 mins at 4°C. The supernatant was then poured off and the pellet stored at - 80°C. The pellet was defrosted and re-suspended in 15 mL of homogenising buffer (20 m M HEPES, 10 m M EDTA, pH 7.4). Then homogenised in mechanical homogeniser (VMR) for 10 seconds. The membrane was centrifuged in centrifuge tubes at 40,000 g for 15 mins at 4°C. The supernatant was poured away and re-suspended in 15 mL of homogenising buffer and homogenised for 20 seconds. The membrane was then centrifuged at 40,000 g for 45 mins at 4°C. The membrane was then re-suspended in 3 mL of storage buffer (20 mM HEPES, 0.1 mM EDTA, pH 7.4) mixing well. The resulting membranes were then stored at - 80°C.
***cAMP Gs Functional Assay:*** cAMP production following $EP_4$ receptor activation was determined using the Homogeneous Time-Resolved Fluorescence (HTRF) cAMP dynamic-2 assay (Cisbio, France). HEK293 cells were transfected using a 0.5 % EP4 Bacmam virus for 36 hours, before dissociating the cells, and freezing at -150°C.
On the day of testing, increasing concentration of test compounds, alongside positive controls (10 μM $PGE_2$ (Tocris, Abingdon, UK)) and negative control (DMSO (Sigma-Aldrich, UK) were added to a ProxiPlate-384 Plus, White 384-shallow well Mircoplate, (PerkinElmer, USA) using the ECHO dispense.
Cells were defrosted in a water bath and resuspended in DMEM supplemented with 10% FBS before centrifuging at 1200 RPM for 5 mins to form a pellet. The pellet was resuspended in assay buffer (DMEM + 0.5 mM IBMX (Tocris, Abingdon, UK)) to 0.5 x $10^6$ cells/mL. Cell suspension, for a final assay concentration of 5000 cell/well was added using the multidrop to the pre-dispensed assay plate. The plate was then incubated at 37°C for 30 mins, with 5% $CO_2$. The cAMP production was determined as manufacturer's instructions, before plates were read on a PheraStar fluorescence plate reader (BMG LabTech, Germany).
The $pEC_{50}$ values were calculated from the mid-point of the curve using dotmatics as shown in Table 2.

Table 2- $EP_4$ $pEC_{50}$ and Emax values

| Example | cAMP human $EP_4$ $pEC_{50}$ | cAMP human $EP_4$ Emax |
|---|---|---|
| 1 | 8.7 | 81 |
| 2 | 8.8 | 99 |
| 3 | 7.9 | 100 |
| 4 | 6.8 | 98 |
| 5 | 7.2 | 80 |
| 6 | 6.3 | 91 |
| 7 | <5 | NA |
| 8 | 7.5 | 74 |
| 9 | 6.7 | 96 |
| 10 | 8.7 | 96 |
| 11 | 7.7 | 92 |
| 12 | 8.1 | 89 |
| 13 | 9.6 | 99 |
| 14 | 6.8 | 100 |
| 15 | 7.5 | 96 |
| 16 | 7.7 | 98 |
| 17 | 7.2 | 97 |
| 18 | 8.5 | 95 |
| 19 | 7.0 | 90 |
| 20 | 9.2 | 98 |
| 21 | 8.1 | 96 |
| 22 | 6.9 | 85 |
| 23 | 9.8 | 91 |
| 24 | 7.6 | 99 |
| 25 | 5.9 | 91 |
| 26 | <6 | NA |
| 27 | 8.2 | 85 |
| 28 | 7.6 | 76 |
| 29 | 8.2 | 91 |
| 30 | 9.3 | 99 |
| 31 | 10.7 | 98 |
| 32 | 9.4 | 90 |
| 33 | 10.5 | 97 |
| 34 | 9.7 | 100 |
| 35 | 10.3 | 97 |

## UNIDIRECTIONAL CACO-2 CELL PERMEABILITY ASSAY

Caco-2 cells (ECACC) were seeded onto 24-well Transwell plates at 2 x $10^5$ cells per well and used in confluent monolayers after a 21 day culture at 37 °C under 5% $CO_2$. Test compounds were incubated at 10 $\mu$M, 0.2% DMSO final, n=2 in assay buffer (Hanks balanced salt solution supplemented with 25 mM HEPES, adjusted to pH 6.5). Hanks balanced

salt solution supplemented with 25 mM HEPES, adjusted to pH 7.4 (0.2% DMSO final) was used for the basolateral chamber (as receiver).

Incubations were performed at 37 °C, with samples removed from both donor and acceptor chambers at T=0 and 1 hour and compound analysed by mass spectrometry (LC-MS/MS) including an analytical internal standard (0.5 μM carbamazepine).

Apparent permeability ($P_{app}$) values are shown in Table 3 and were determined from the relationship:

$$P_{app} = \frac{Compound_{Acceptor\ T=end} / (Compound_{Donor} \times V_{Donor})}{Incubation\ Time} \times \frac{V_{Donor}}{Area \times 60 \times 10^{-6}\ cm/s}$$

Where V is the volume of each Transwell compartment (apical 125 μL, basolateral 600 μL), and concentrations are the relative MS responses for compound (normalized to internal standard) in the donor chamber before incubation and acceptor chamber at the end of the incubation Area = area of cells exposed for drug transfer (0.33 $cm^2$).

Lucifer Yellow (LY) was added to the apical buffer in all wells to assess viability of the cell layer. As LY cannot freely permeate lipophilic barriers, a high degree of LY transport indicates poor integrity of the cell layer and wells with a LY $P_{app}$ > 10 x $10^{-6}$ cm/s were rejected.

Compound recovery from the wells was determined from MS responses (normalized to internal standard) in donor and acceptor chambers at the end of incubation compared to response in the donor chamber pre-incubation.

**Table 3 - Mean apparent permeability ($P_{app}$) values**

| Example | Caco-2 Mean $P_{app}$ A-B ($10^{-6}$ cm/s) |
|---|---|
| 1 | 14.5 |
| 2 | 1.14 |
| 3 | 1.18 |
| 4 | 0.26 |
| 5 | 0.36 |
| 6 | 0.85 |
| 7 | 7.71 |
| 8 | 2.1 |
| 9 | 0.33 |
| 10 | 1.84 |
| 11 | 0.18 |
| 12 | 0.26 |
| 13 | 0.34 |
| 14 | 0.19 |
| 15 | <0.1 |
| 16 | 0.17 |
| 18 | 0.11 |
| 19 | <0.12 |
| 20 | <0.1 |
| 21 | <0.11 |
| 22 | <0.1 |
| 23 | 0.245 |
| 24 | 0.16 |
| 27 | <0.28 |

(continued)

| Example | Caco-2 Mean $P_{app}$ A-B ($10^{-6}$ cm/s) |
|---|---|
| 28 | <0.12 |
| 29 | <0.11 |
| 30 | 0.29 |
| 31 | 0.45 |
| 33 | 0.32 |
| 34 | 0.24 |
| 35 | <0.1 |

## Claims

**1.** A compound of Formula I:

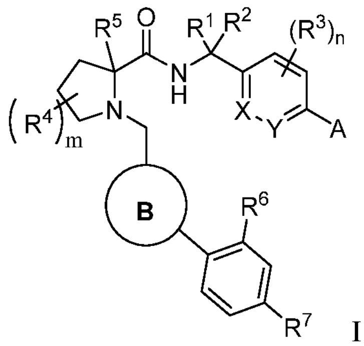

I

or a pharmaceutically acceptable salt, solvate, hydrate, tautomer or optical isomer thereof, wherein;

A is OR', C(O)R', $CO_2R'$, C(O)N(R')$_2$, C(O)N(R')S(O)$_2$R', S(O)$_2$R', S(O)$_2$OR', $SO_2N(R')_2$, $C_{1-8}$ alkyl, cycloalkyl, heterocycloalkyl, aryl, or heteroaryl;
Ring B is aryl or heteroaryl;
X and Y are each independently CR" or N, wherein at least one of X and Y is CH; $R^1$ and $R^2$ are each independently H, $C_{1-6}$ alkyl, $C_{1-6}$ alkoxy, or $R^1$ and $R^2$, together with the carbon atom to which they are attached, form a $C_{3-6}$ cycloalkane-1,1-diyl ring;
each $R^3$ is independently selected from H, OR', COOR', C(O)R', halo, or $C_{1-6}$ alkyl; $R^4$ is H, $C_{1-6}$ alkyl, halo, CN, $NO_2$, or OR';
$R^5$ is H or $C_{1-6}$ alkyl; or $R^4$ and $R^5$, together with the pyrrolidine ring to which they are attached, form a $C_{1-6}$ alkylene linker;
$R^6$ is H, $C_{1-6}$ alkyl, $C_{1-3}$ alkoxy optionally substituted with 1-3 fluorine atoms, halo, CN, $NO_2$, OR', $CO_2R'$, or C(O)R';
$R^7$ is OR', OC(O)R', OC(O)OR', $CO_2R'$, CON(R')$_2$, $SO_2N(R')_2$, $SO_2R'$, $OSO_2R'$, or $OSO_2N(R')_2$;
each R' is independently H, $C_{1-6}$ alkyl, or $C_{3-6}$ cycloalkyl;
each R" is H, $C_{1-6}$ alkyl, halo, or OR': and
n and m are each independently 0, 1, 2, or 3;
wherein at each occurrence, alkyl, alkylene, and cycloalkyl are each optionally and independently substituted with up to 3 instances of OH, SH, CN, $NO_2$, COOH, halo, or $COOC_{1-4}$ alkyl;
wherein at each occurrence, heterocycloalkyl, aryl, and heteroaryl are each optionally and independently substituted with up to 3 instances of OR', SR', CN, $NO_2$, $CO_2R'$, halo, $C_{1-4}$ alkyl, or oxo.

**2.** The compound according to claim 1, wherein:

(i) Ring B is a 6 membered aryl or a 5-6 membered heteroaryl; each optionally and independently substituted with up to 3 instances of OR', SR', CN, $NO_2$, $CO_2R'$, halo, or $C_{1-4}$ alkyl; or

(ii) Ring B is phenyl, which is optionally substituted with up to three instances of OH, or a 6 membered heteroaryl comprising one or two nitrogen atoms, wherein each nitrogen atom is optionally oxidized.

**3.** The compound according to claim 1 or 2, which is a compound of Formula (1):

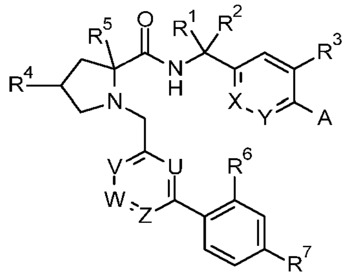
(1)

or a pharmaceutically acceptable salt, solvate, hydrate, tautomer or optical isomer thereof, wherein;

U, V, W, and Z are each independently selected from the group consisting of CH, COH, N or $N^+$-$O^-$, wherein at least three of U, V, W, and Z are CH.

**4.** The compound according to any one of claims 1-3, wherein $R^4$ is H, OH, F or is joined to $R^5$ to form a $CH_2$ bridge; preferably wherein $R^4$ is H, OH or is joined to $R^5$ to form a $CH_2$ bridge.

**5.** The compound according to any one of claims 1-4, wherein $R^5$ is H or is joined to $R^4$ to form a $CH_2$ bridge; preferably wherein $R^5$ is H.

**6.** The compound according to any one of claims 1-5, wherein A is selected from the group consisting of:

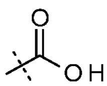 , 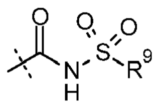 , 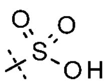 , 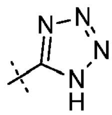 , 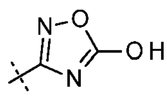 , 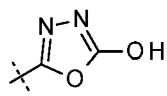 ,

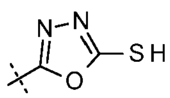 , 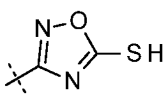 , 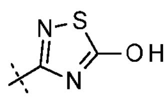 , 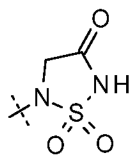 , 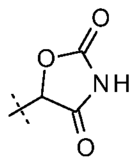 ;

wherein $R^9$ is $C_{1-3}$ alkyl or a $C_{3-6}$ cycloalkyl ring; preferably wherein A is:

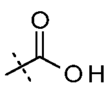 or 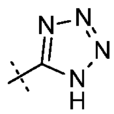 .

**7.** The compound according to claim 3, which is a compound of Formula (2a):

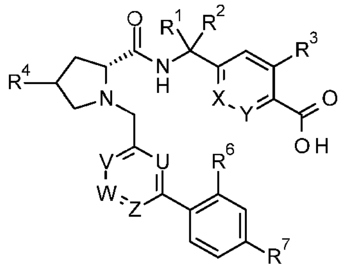

(2a),

or a pharmaceutically acceptable salt, solvate, hydrate, tautomer or optical isomer thereof, wherein U, V, W, X, Y, Z, $R^1$, $R^2$, $R^3$, $R^4$, $R^6$, and $R^7$ are the same as defined in claim 3.

**8.** The compound according to any one of claims 1-7, wherein $R^1$ and $R^2$ are independently H or $C_{1-3}$ alkyl optionally substituted with 1-3 fluorine atoms.

**9.** The compound according to any one of claims 1-7, wherein $R^1$ is H or methyl or is joined to $R^2$ to form a cyclopropane-1,1-diyl ring; more preferably wherein $R^1$ is methyl; and/or wherein $R^2$ is H.

**10.** The compound according to claim 3, which is a compound of Formula (3a):

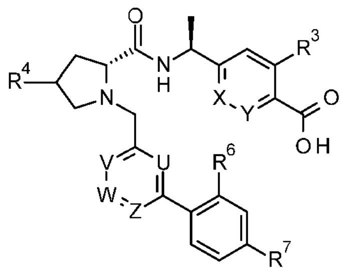

(3a),

or a pharmaceutically acceptable salt, solvate, hydrate, tautomer or optical isomer thereof, wherein U, V, W, X, Y, Z, $R^3$, $R^4$, $R^6$, and $R^7$ are the same as defined in claim 3.

**11.** The compound according to any one of claims 1-10, wherein $R^3$ is H, OH or F; preferably wherein $R^3$ is H or OH; more preferably wherein $R^3$ is H.

**12.** The compound according to any one of claims 1-11, wherein $R^4$ is H, OH or F; preferably wherein $R^4$ is H or OH; more preferably wherein $R^4$ is H.

**13.** The compound according to any one of claims 1-12, wherein $R^6$ is H, OH, CN, halo, $C_{1-3}$ alkoxy optionally substituted with 1-3 fluorine atoms or $C_{1-3}$ alkyl optionally substituted with 1-3 fluorine atoms; preferably wherein $R^6$ is H, OH, CN or methyl; more preferably wherein $R^6$ is methyl.

**14.** The compound according to any one of claims 1-13, wherein $R^7$ is OH, $CO_2H$, $CONH_2$, $SO_2NH_2$ or $OSO_2NH_2$; preferably wherein $R^7$ is $CONH_2$ or $SO_2NH_2$.

**15.** The compound according to any one of claims 1-14, wherein X and Y are each independently selected from the group consisting of CH, CF, COH or N; preferably wherein X is CH and Y is CH or COH; more preferably wherein X and Y are both CH.

**16.** The compound according to any one of claims 3-15, wherein U, V, W and Z are CH, or wherein U, V and Z are CH and W is COH.

**17.** The compound according to claim 1, wherein the compound is selected from:

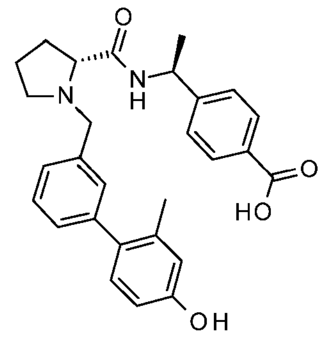

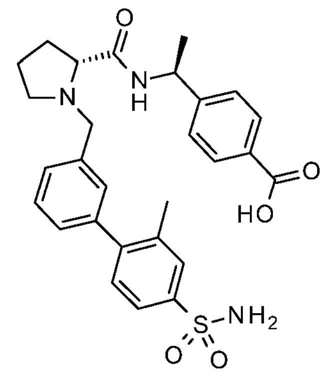

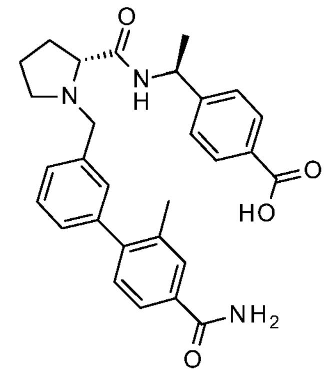

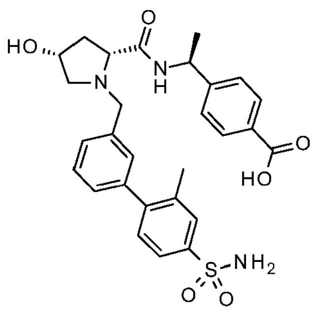

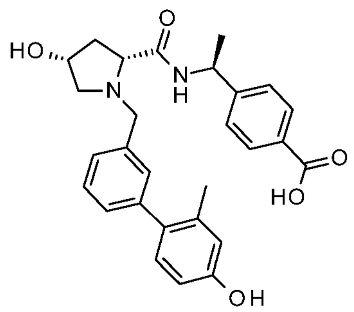

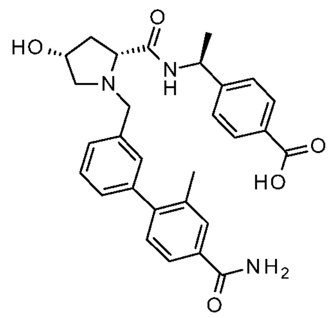

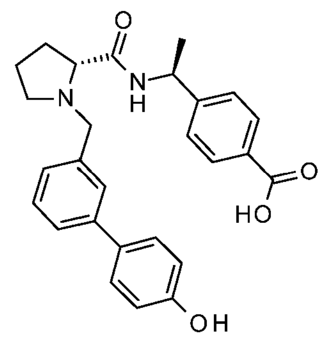

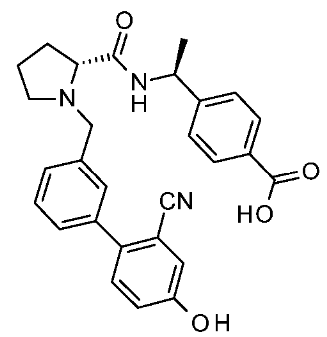

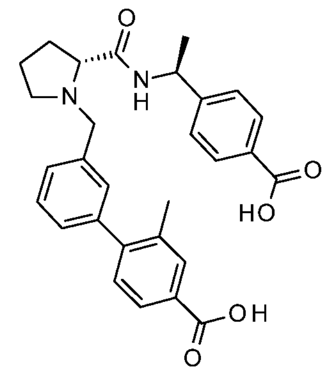

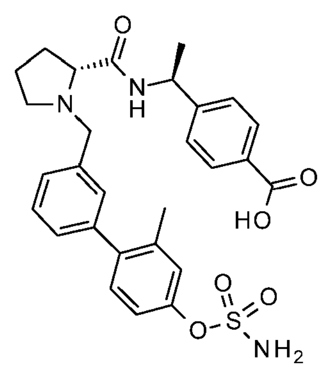

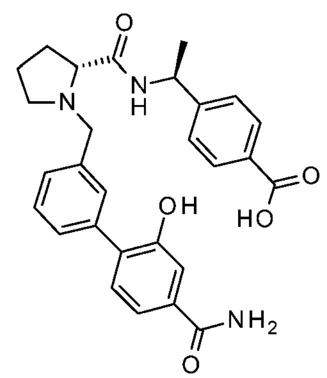

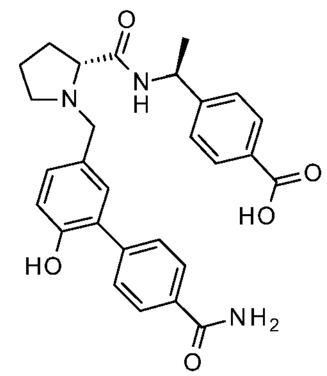

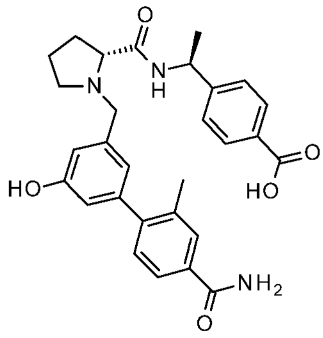

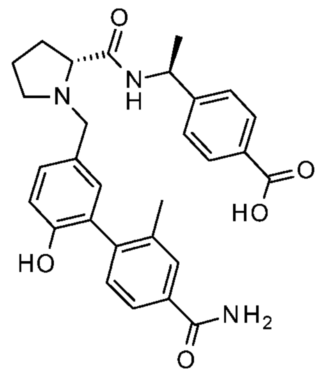

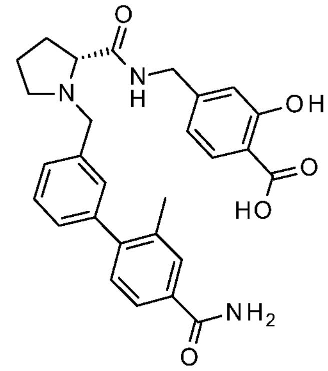

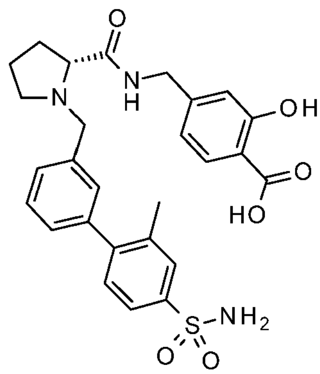

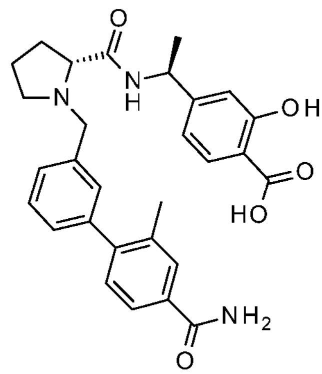

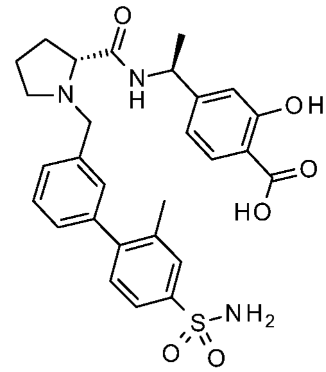

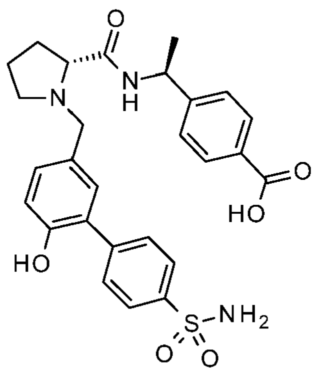

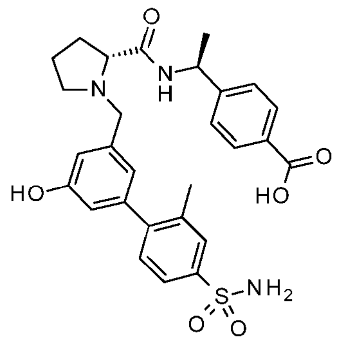

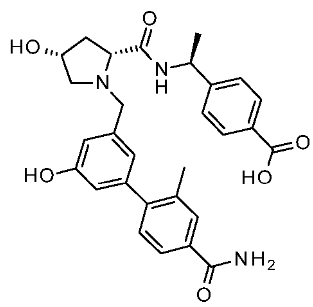

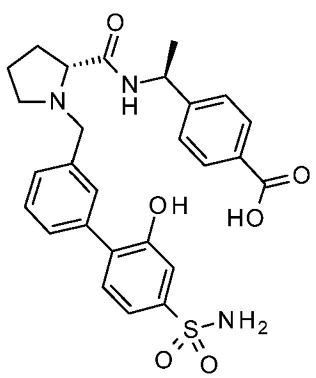

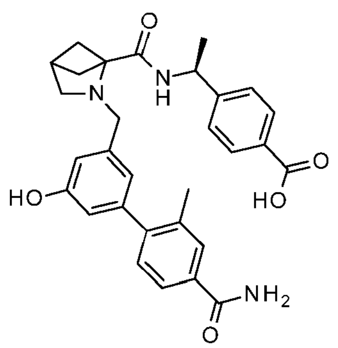

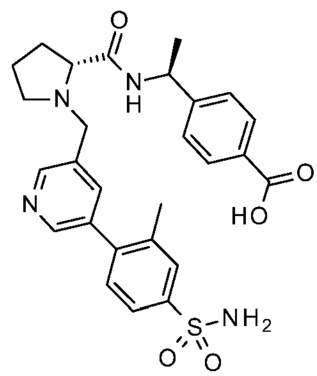

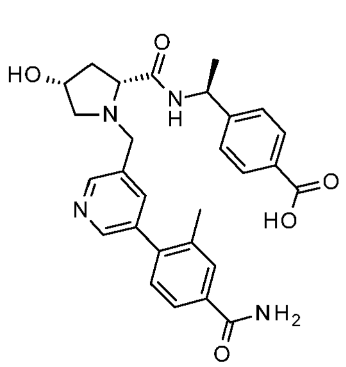

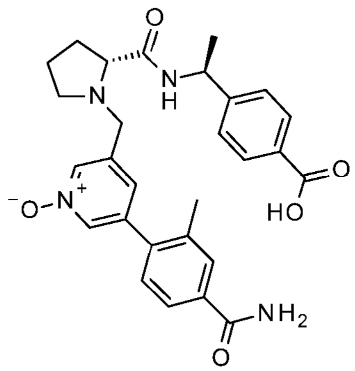

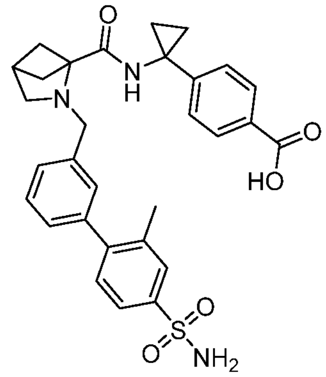

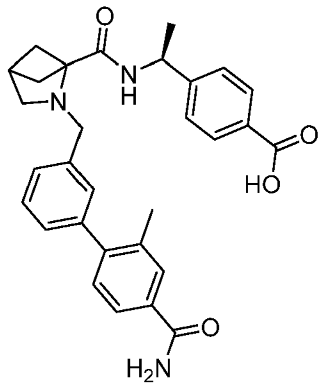

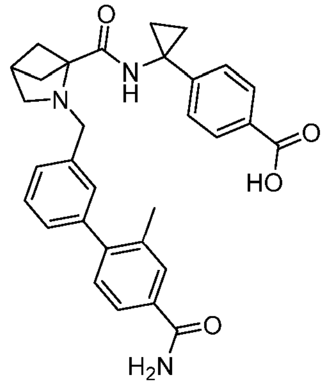

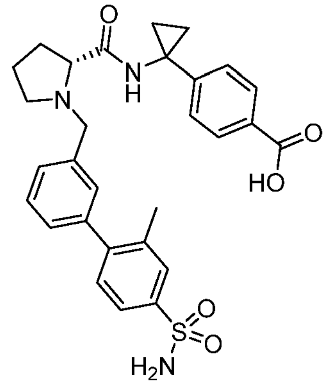

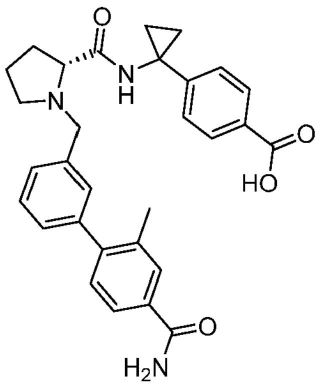

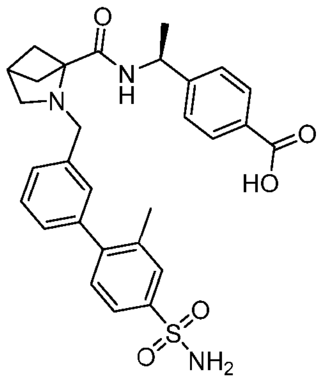

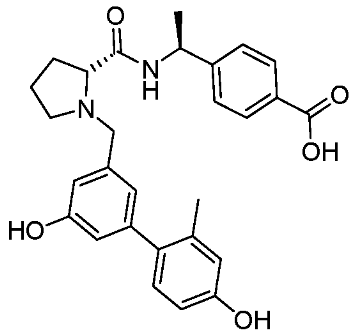

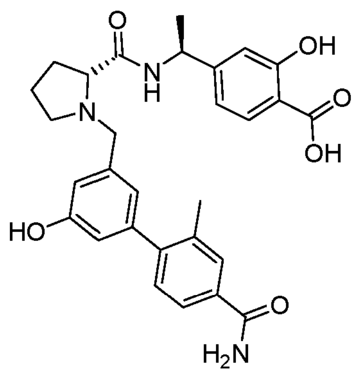

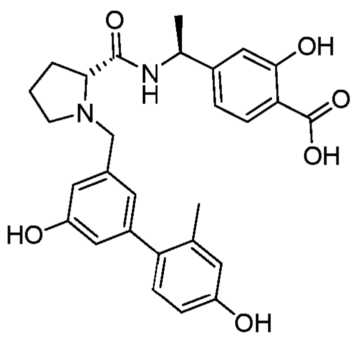

or a pharmaceutically acceptable salt, solvate, hydrate or tautomer thereof.

**18.** A pharmaceutical composition comprising a compound, pharmaceutically acceptable salt, solvate, hydrate, tautomer

or optical isomer according to any one of claims 1-17, and a pharmaceutically acceptable excipient; preferably wherein the composition further comprises at least one additional therapeutic agent selected from the group consisting of aminosalicylates, corticosteroids, immunomodulators and combinations thereof.

**19.** A kit comprising a compound, pharmaceutically acceptable salt, solvate, hydrate, tautomer or optical isomer according to any one of claims 1-17 and at least one additional therapeutic agent selected from the group consisting of aminosalicylates, corticosteroids, immunomodulators and combinations thereof.

**20.** A compound according to any one of claims 1-17, a composition according to claim 18, or a kit according to claim 19 for use in medicine.

**21.** A compound according to any one of claims 1-17, a composition according to claim 18, or a kit according to claim 19, for use in the treatment of an EP4 receptor mediated disease, wherein the EP4 receptor mediated disease is a gastrointestinal disorder or a pulmonary disease or condition.

**22.** The compound, composition or kit for use according to claim 21, wherein:

(i) the gastrointestinal disorder is selected from the group consisting of constipation disorders, constipation-predominant irritable bowel syndrome, mixed type irritable bowel syndrome, chronic idiopathic constipation, gastrointestinal symptoms associated with Parkinson's disease, gastrointestinal symptoms associated with cystic fibrosis, intestinal dysmotility, postoperative ileus, food allergy or food intolerance, celiac disease, gastrointestinal motility disorders, functional gastrointestinal disorders, drug induced enteropathy, NSAID induced gastric and intestinal injury, chemotherapy induced mucositis, gastroesophageal reflux disease (GERD), duodenogastric reflux, diarrhoeal diseases, immune mediated gastrointestinal diseases, Crohn's disease, ulcerative colitis, inflammatory bowel disease, and ischemic colitis; and/or
(ii) the pulmonary disease or condition is selected from chronic obstructive pulmonary diseases, asthma, chronic bronchitis, cystic fibrosis, emphysema, chronic idiopathic cough, hyperactive airway disorder, and idiopathic pulmonary fibrosis.

## Patentansprüche

**1.** Verbindung der Formel I:

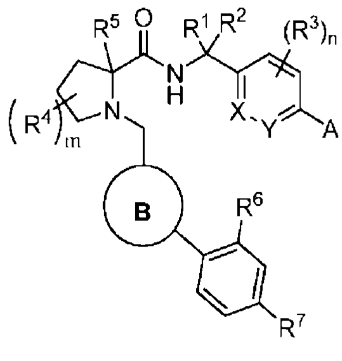

I

oder ein pharmazeutisch annehmbares Salz, Solvat, Hydrat, Tautomer oder optisches Isomer dieser, wobei;

A OR', C(O)R', $CO_2R'$, $C(O)N(R')_2$, $C(O)N(R')S(O)_2R'$, $S(O)_2R'$, $S(O)_2OR'$, $SO_2N(R')_2$, $C_{1-8}$-Alkyl, Cycloalkyl, Heterocycloalkyl, Aryl, oder Heteroaryl ist;
Ring B Aryl oder Heteroaryl ist;
X und Y jeweils unabhängig voneinander CR" oder N sind, wobei mindestens eines von X und Y CH ist;
$R^1$ und $R^2$ jeweils unabhängig voneinander H, $C_{1-6}$-Alkyl, $C_{1-6}$-Alkoxy sind, oder $R^1$ und $R^2$ zusammen mit dem Kohlenstoffatom, an das sie gebunden sind, einen $C_{3-6}$-Cycloalkan-1,1-diyl-Ring bilden;
jedes $R^3$ unabhängig voneinander aus H, OR', COOR', C(O)R', Halogen oder $C_{1-6}$-Alkyl ausgewählt ist;
$R^4$ H, $C_{1-6}$ -Alkyl, Halogen, CN, $NO_2$, oder OR' ist;
$R^5$ H oder $C_{1-6}$-Alkyl ist; oder $R^4$ und $R^5$ zusammen mit dem Pyrrolidinring, an den sie gebunden sind, einen $C_{1-6}$-Alkylen-Linker bilden;

$R^6$ H, $C_{1-6}$-Alkyl, $C_{1-3}$-Alkoxy, das optional mit 1-3 Fluoratomen substituiert ist, Halogen, CN, $NO_2$, OR’, $CO_2R'$, oder C(O)R’ ist;
$R^7$ OR’, OC(O)R’, OC(O)OR’, $CO_2R'$, $CON(R')_2$, $SO_2N(R')_2$, $SO_2R'$, $OSO_2R'$, oder $OSO_2N(R')_2$ ist;
jedes R’ unabhängig H, $C_{1-6}$ -Alkyl, oder $C_{3-6}$ -Cycloalkyl ist;
jedes R" H, $C_{1-6}$ -Alkyl, Halogen oder OR’ ist; und
n und m jeweils unabhängig voneinander 0, 1, 2 oder 3 sind;
wobei bei jedem Vorkommen Alkyl, Alkylen und Cycloalkyl jeweils optional und unabhängig voneinander mit bis zu 3 Resten von OH, SH, CN, $NO_2$, COOH, Halogen oder $COOC_{1-4}$ -Alkyl substituiert sind;
wobei bei jedem Vorkommen Heterocycloalkyl, Aryl und Heteroaryl jeweils optional und unabhängig voneinander mit bis zu 3 Resten von OR’, SR’, CN, $NO_2$, $CO_2R'$, Halogen, $C_{1-4}$-Alkyl, oder Oxo substituiert sind.

**2.** Verbindung nach Anspruch 1, wobei:

(i) Ring B ein 6-gliedriges Aryl oder ein 5-6-gliedriges Heteroaryl ist; jeweils optional und unabhängig voneinander mit bis zu 3 Resten von OR’, SR’, CN, $NO_2$, $CO_2R'$, Halogen, oder $C_{1-4}$ -Alkyl substituiert; oder
(ii) Ring B Phenyl ist, das optional mit bis zu drei OH-Gruppen substituiert ist, oder ein 6-gliedriges Heteroaryl, das ein oder zwei Stickstoffatome umfasst, wobei jedes Stickstoffatom optional oxidiert ist.

**3.** Verbindung nach Anspruch 1 oder 2, die eine Verbindung der Formel (1) ist:

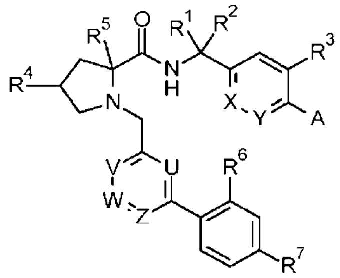

(1)

oder ein pharmazeutisch annehmbares Salz, Solvat, Hydrat, Tautomer oder optisches Isomer dieser, wobei;
U, V, W und Z jeweils unabhängig voneinander aus der Gruppe ausgewählt sind, die aus CH, COH, N oder $N^+$-$O^-$ besteht, wobei mindestens drei von U, V, W und Z CH sind.

**4.** Verbindung nach einem der Ansprüche 1-3, wobei $R^4$ H, OH oder F ist oder mit $R^5$ verbunden ist, um eine $CH_2$-Brücke zu bilden; wobei $R^4$ vorzugsweise H oder OH ist oder mit $R^5$ verbunden ist, um eine $CH_2$-Brücke zu bilden.

**5.** Verbindung nach einem der Ansprüche 1-4, wobei $R^5$ H ist oder mit $R^4$ verbunden ist, um eine $CH_2$-Brücke zu bilden; wobei $R^5$ vorzugsweise H ist.

**6.** Verbindung nach einem der Ansprüche 1-5, wobei A ausgewählt ist aus der Gruppe bestehend aus:

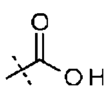 , 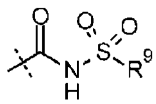 , 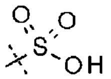 , 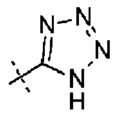 , 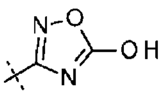 , 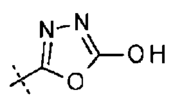 ,

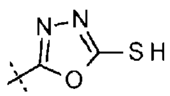 , 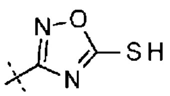 , 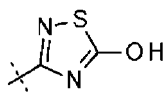 , 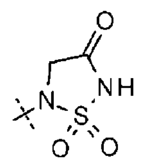 , 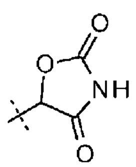 ;

**wobei** $R^9$ $C_{1-3}$-Alkyl oder ein $C_{3-6}$ -Cycloalkylring ist; vorzugsweise wobei A Folgendes ist:

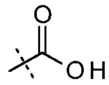

oder

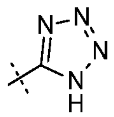

.

**7.** Verbindung nach Anspruch 3, die eine Verbindung der Formel (2a) ist:

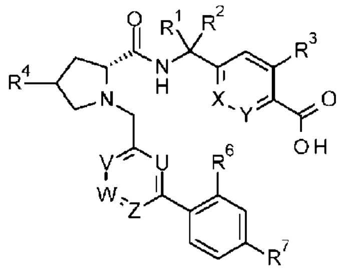

(2a),

oder ein pharmazeutisch annehmbares Salz, Solvat, Hydrat, Tautomer oder optisches Isomer dieser, wobei U, V, W, X, Y, Z, $R^1$, $R^2$, $R^3$, $R^4$, $R^6$, und $R^7$ die in Anspruch 3 definierte Bedeutung haben.

**8.** Verbindung nach einem der Ansprüche 1-7, wobei $R^1$ und $R^2$ unabhängig voneinander H oder $C_{1-3}$ -Alkyl sind, das optional mit 1 bis 3 Fluoratomen substituiert ist.

**9.** Verbindung nach einem der Ansprüche 1-7, wobei $R^1$ H oder Methyl ist oder mit $R^2$ verbunden ist, um einen Cyclopropan-1,1-diyl-Ring zu bilden; wobei $R^1$ noch bevorzugter Methyl ist; und/oder wobei $R^2$ H ist.

**10.** Verbindung nach Anspruch 3, die eine Verbindung der Formel (3a) ist:

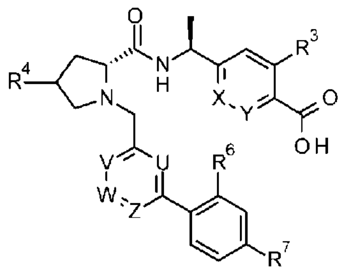

(3a),

oder ein pharmazeutisch annehmbares Salz, Solvat, Hydrat, Tautomer oder optisches Isomer dieser, wobei U, V, W, X, Y, Z, $R^3$, $R^4$, $R^6$, und $R^7$ die in Anspruch 3 definierte Bedeutung haben.

**11.** Verbindung nach einem der Ansprüche 1-10, wobei $R^3$ H, OH oder F ist; wobei $R^3$ vorzugsweise H oder OH ist; wobei $R^3$ noch bevorzugter H ist.

**12.** Verbindung nach einem der Ansprüche 1-11, wobei $R^4$ H, OH oder F ist; wobei $R^4$ vorzugsweise H oder OH ist; wobei $R^4$ noch bevorzugter H ist.

**13.** Verbindung nach einem der Ansprüche 1-12, wobei $R^6$ H, OH, CN, Halogen, $C_{1-3}$-Alkoxy, das optional mit 1-3 Fluoratomen substituiert ist, oder $C_{1-3}$ -Alkyl, das optional mit 1-3 Fluoratomen substituiert ist, ist; wobei $R^6$ vorzugsweise H, OH, CN oder Methyl ist; wobei $R^6$ noch bevorzugter Methyl ist.

**14.** Verbindung nach einem der Ansprüche 1-13, wobei $R^7$ OH, $CO_2H$, $CONH_2$, $SO_2NH_2$ oder $OSO_2NH_2$ ist; wobei $R^7$ vorzugsweise $CONH_2$ oder $SO_2NH_2$ ist.

**15.** Verbindung nach einem der Ansprüche 1-14, wobei X und Y jeweils unabhängig voneinander aus der Gruppe ausgewählt sind, die aus CH, CF, COH oder N besteht; wobei vorzugsweise X CH ist und Y CH oder COH ist; noch

bevorzugter wobei X und Y beide CH sind.

**16.** Verbindung nach einem der Ansprüche 3-15, wobei U, V, W und Z CH sind oder wobei U, V und Z CH sind und W COH ist.

**17.** Verbindung nach Anspruch 1, wobei die Verbindung ausgewählt ist aus:

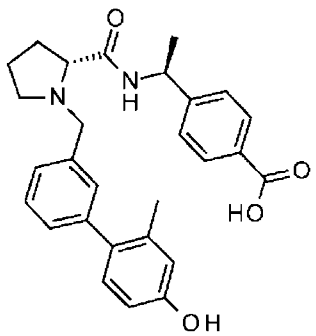
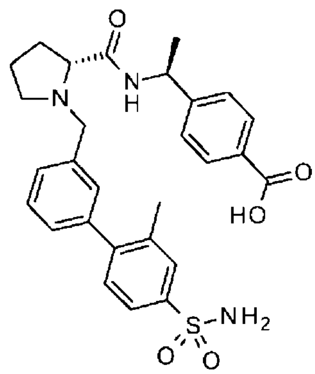
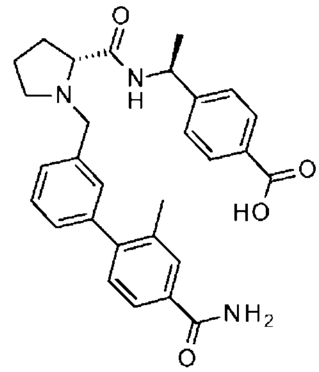
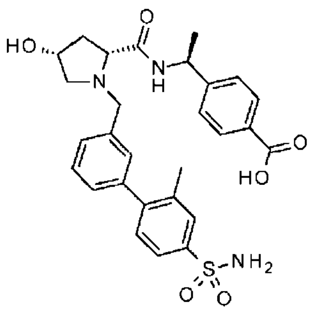
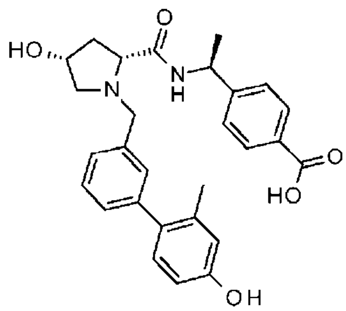
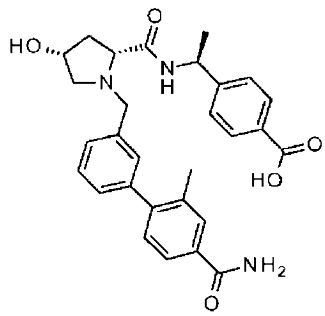
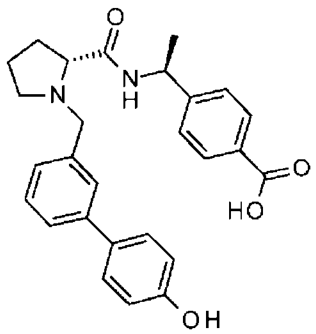
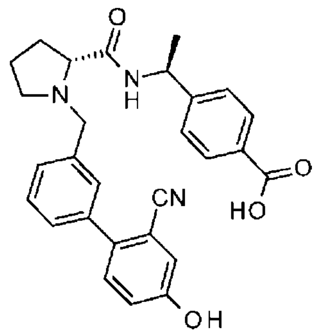
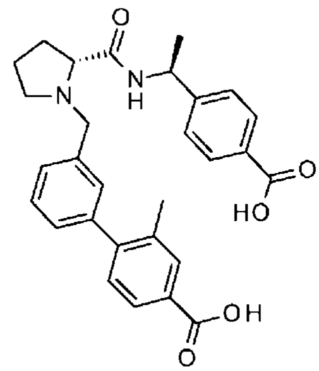
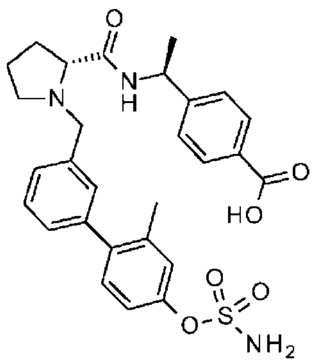
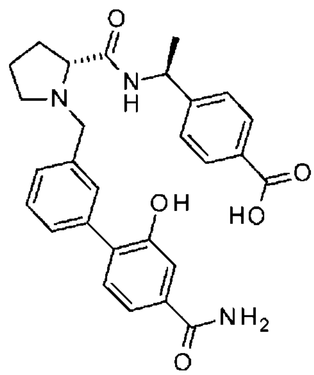
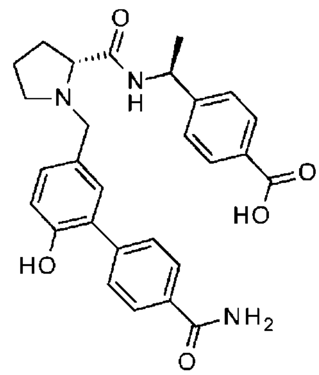

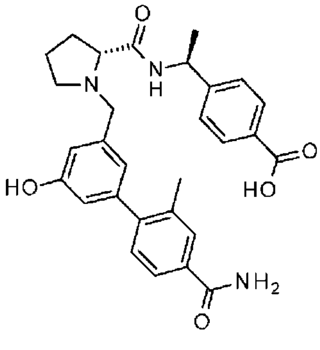
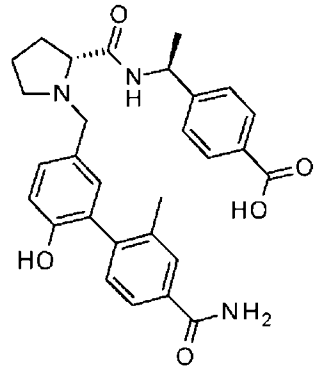
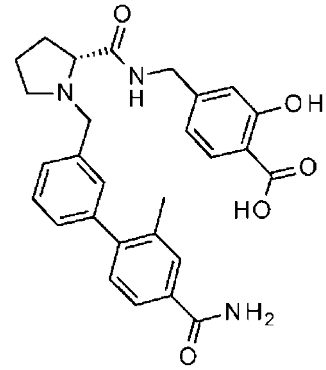

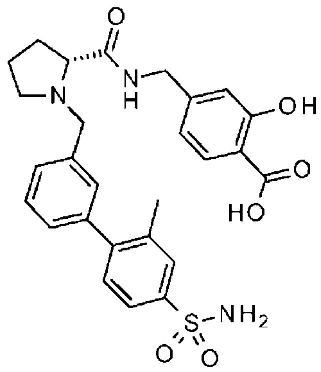
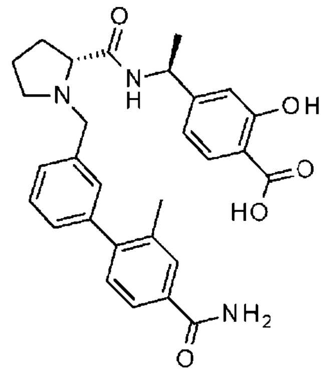
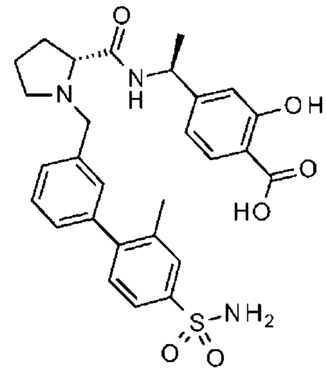

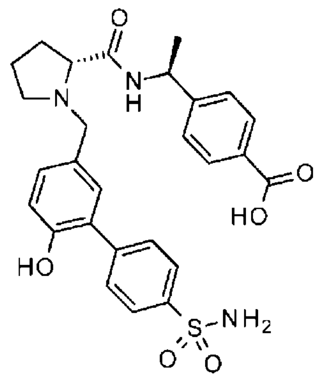
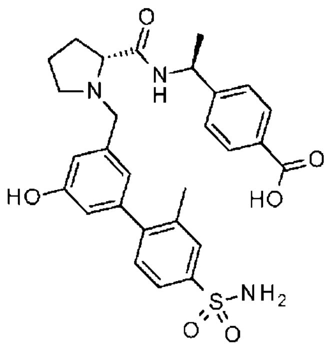
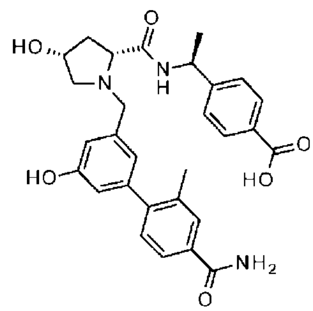

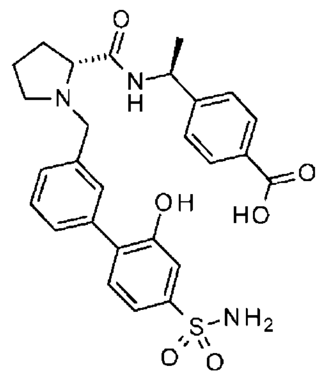
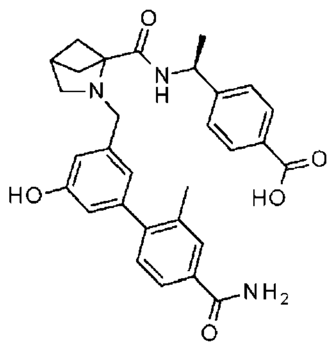
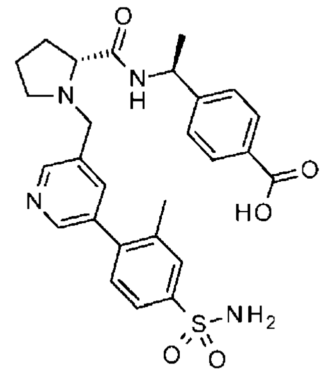

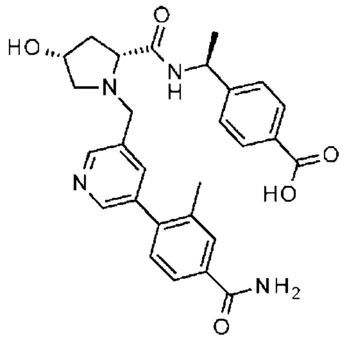
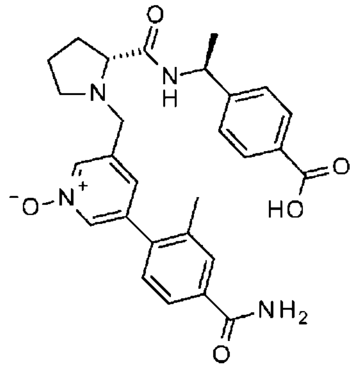
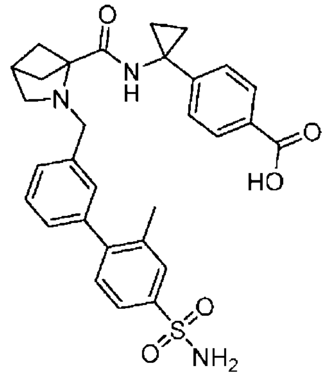
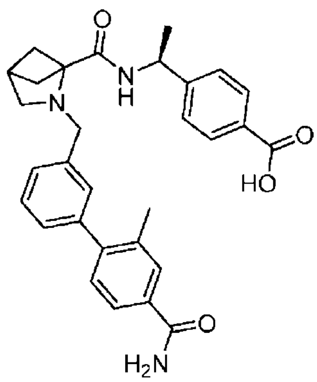
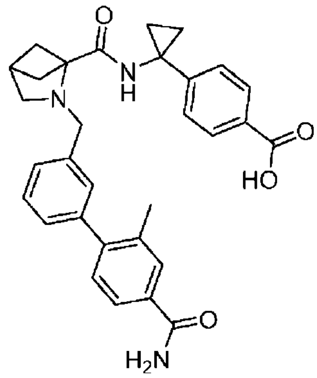
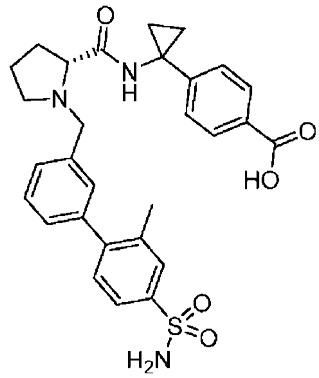
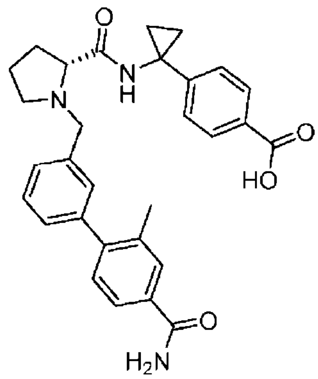
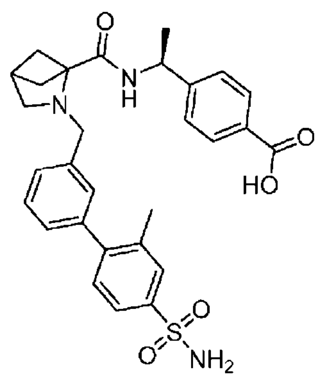
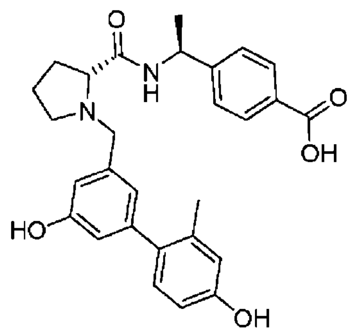
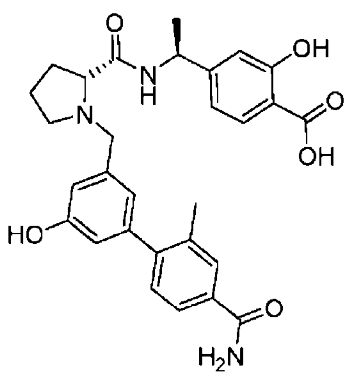
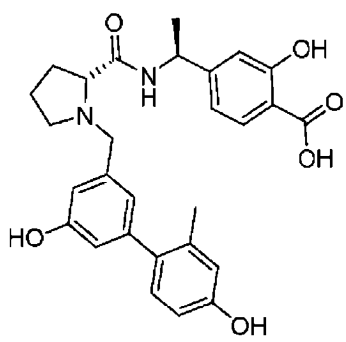

oder ein pharmazeutisch annehmbares Salz, Solvat, Hydrat oder Tautomer dieser.

**18.** Pharmazeutische Zusammensetzung, umfassend eine Verbindung, ein pharmazeutisch annehmbares Salz, ein Solvat, ein Hydrat, ein Tautomer oder ein optisches Isomer nach einem der Ansprüche 1-17, und einen pharmazeutisch annehmbaren Hilfsstoff; wobei die Zusammensetzung vorzugsweise weiter mindestens einen zusätzlichen therapeutischen Wirkstoff umfasst, der aus der Gruppe ausgewählt ist, die aus Aminosalicylaten, Kortikosteroiden, Immunmodulatoren und Kombinationen dieser besteht.

**19.** Kit, umfassend eine Verbindung, ein pharmazeutisch annehmbares Salz, ein Solvat, ein Hydrat, ein Tautomer oder ein optisches Isomer nach einem der Ansprüche 1-17 und mindestens einen weiteren therapeutischen Wirkstoff, ausgewählt aus der Gruppe bestehend aus Aminosalicylaten, Kortikosteroiden, Immunmodulatoren und Kombinationen dieser.

**20.** Verbindung nach einem der Ansprüche 1-17, Zusammensetzung nach Anspruch 18, oder Kit nach Anspruch 19 zur Verwendung in der Medizin.

**21.** Verbindung nach einem der Ansprüche 1-17, Zusammensetzung nach Anspruch 18, oder Kit nach Anspruch 19, zur Verwendung bei der Behandlung einer durch den EP4-Rezeptor vermittelten Erkrankung, wobei es sich bei der durch den EP4-Rezeptor vermittelten Erkrankung um eine Magen-Darm-Erkrankung oder eine Lungenerkrankung bzw. einen Lungenzustand handelt.

**22.** Verbindung, Zusammensetzung oder Kit zur Verwendung nach Anspruch 21, wobei:

(i) die Magen-Darm-Erkrankung aus der Gruppe ausgewählt ist, die aus Verstopfungsstörungen, dem verstopfungsdominanten Reizdarmsyndrom, dem gemischten Reizdarmsyndrom, chronischer idiopathischer Verstopfung, Magen-Darm-Symptomen im Zusammenhang mit der Parkinson-Krankheit, Magen-Darm-Symptomen im Zusammenhang mit Mukoviszidose, Darmmotilitätsstörungen, postoperativem Ileus, Nahrungsmittelallergien oder -unverträglichkeiten, Zöliakie, Störungen der Magen-Darm-Motilität, funktionellen Magen-Darm-Erkrankungen, medikamenteninduzierter Enteropathie, durch NSAIDs verursachten Magen- und Darmschäden, durch Chemotherapie verursachter Mukositis, gastroösophagealer Refluxkrankheit (GERD), duodenogastralem Reflux, Durchfallerkrankungen, immunvermittelten Magen-Darm-Erkrankungen, Morbus Crohn, Colitis ulcerosa, entzündlichen Darmerkrankungen und ischämischer Kolitis besteht; und/oder
(ii) die Lungenerkrankung oder der Lungenzustand ausgewählt ist aus chronisch obstruktiven Lungenerkrankungen, Asthma, chronischer Bronchitis, Mukoviszidose, Emphysem, chronischem idiopathischem Husten, hyperaktiver Atemwegserkrankung und idiopathischer Lungenfibrose.

## Revendications

**1.** Composé de Formule I :

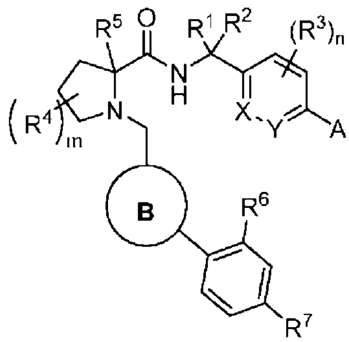

I

ou sel pharmaceutiquement acceptable, solvate, hydrate, tautomère ou isomère optique de celui-ci, dans lequel :

A est OR', C(O)R', $CO_2R'$, $C(O)N(R')_2$, $C(O)N(R')S(O)_2R'$, $S(O)_2R'$, $S(O)_2OR'$, $SO_2N(R')_2$, alkyle en $C_{1-8}$, cycloalkyle, hétérocycloalkyle, aryle, ou hétéroaryle ;
le cycle B est aryle ou hétéroaryle ;
X et Y sont chacun indépendamment CR" ou N, dans lequel au moins un de X et Y est CH ;
$R^1$ et $R^2$ sont chacun indépendamment H, alkyle en $C_{1-6}$, alcoxy en $C_{1-6}$, ou $R^1$ et $R^2$, conjointement avec l'atome de carbone auquel ils sont attachés, forment un cycle cycloalcane-1,1-diyle en $C_{3-6}$ ;
chaque $R^3$ est sélectionné indépendamment parmi H, OR', COOR', C(O)R', halo, ou alkyle en $C_{1-6}$ ;

$R^4$ est H, alkyle en $C_{1-6}$, halo, CN, $NO_2$, ou OR' ;
$R^5$ est H ou alkyle en $C_{1-6}$ ; ou $R^4$ et $R^5$, conjointement avec le cycle pyrrolidine auquel ils sont attachés, forment un agent de liaison alkylène en $C_{1-6}$ ;
$R^6$ est H, alkyle en $C_{1-6}$, alcoxy en $C_{1-3}$ facultativement substitué par 1 à 3 atomes de fluor, halo, CN, $NO_2$, OR', $CO_2R'$, ou C(O)R' ;
$R^7$ est OR', OC(O)R', OC(O)OR', $CO_2R'$, $CON(R')_2$, $SO_2N(R')_2$, $SO_2R'$, $OSO_2R'$, ou $OSO_2N(R')_2$ ;
chaque R' est indépendamment H, alkyle en $C_{1-6}$, ou cycloalkyle en $C_{3-6}$ ;
chaque R" est H, alkyle en $C_{1-6}$, halo, ou OR' ; et
n et m sont chacun indépendamment 0, 1, 2, ou 3 ;
dans lequel, à chaque occurrence, alkyle, alkylène et cycloalkyle sont chacun facultativement et indépendamment substitués par jusqu'à 3 instances de OH, SH, CN, $NO_2$, COOH, halo, ou alkyle en $COOC_{1-4}$ ;
dans lequel, à chaque occurrence, hétérocycloalkyle, aryle et hétéroaryle sont chacun facultativement et indépendamment substitués par jusqu'à 3 instances de OR', SR', CN, $NO_2$, $CO_2R'$, halo, alkyle en $C_{1-4}$, ou oxo.

**2.** Composé selon la revendication 1, dans lequel :

(i) le cycle B est un aryle à 6 chaînons ou un hétéroaryle à 5 à 6 chaînons ; chacun étant facultativement et indépendamment substitué par jusqu'à 3 instances de OR', SR', CN, $NO_2$, $CO_2R'$, halo, ou alkyle en $C_{1-4}$ ; ou
(ii) le cycle B est phényle, lequel est facultativement substitué par jusqu'à trois instances de OH, ou un hétéroaryle à 6 chaînons comprenant un ou deux atomes d'azote, dans lequel chaque atome d'azote est facultativement oxydé.

**3.** Composé selon la revendication 1 ou la revendication 2, qui est un composé de formule (1) :

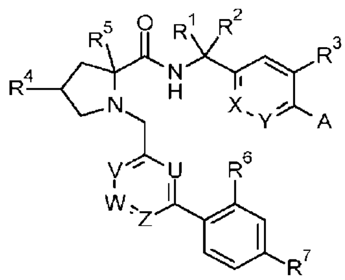
(1)

ou sel pharmaceutiquement acceptable, solvate, hydrate, tautomère ou isomère optique de celui-ci, dans lequel : U, V, W, et Z sont chacun indépendamment sélectionnés dans le groupe consistant en CH, COH, N ou $N^+$-$O^-$, dans lequel au moins trois parmi U, V, W, et Z sont CH.

**4.** Composé selon l'une quelconque des revendications 1 à 3, dans lequel $R^4$ est H, OH, F ou est relié à $R^5$ pour former un pont $CH_2$ ; de préférence dans lequel $R^4$ est H, OH ou est relié à $R^5$ pour former un pont $CH_2$.

**5.** Composé selon l'une quelconque des revendications 1 à 4, dans lequel $R^5$ est H ou est relié à $R^4$ pour former un pont $CH_2$ ; de préférence dans lequel $R^5$ est H.

**6.** Composé selon l'une quelconque des revendications 1 à 5, dans lequel A est sélectionné dans le groupe consistant en :

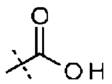 , 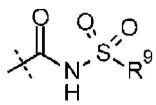 , 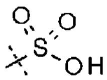 , 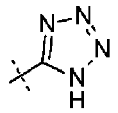 , 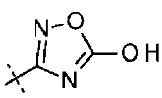 , 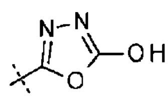 ,

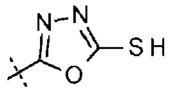, 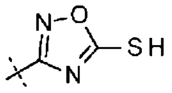, 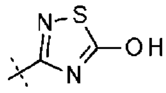, 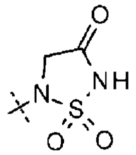, 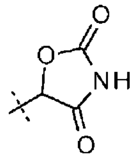 ;

dans lequel $R^9$ est alkyle en $C_{1-3}$ ou un cycle cycloalkyle en $C_{3-6}$ ; de préférence dans lequel A est :

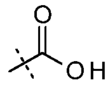 ou 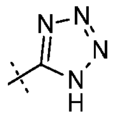 .

**7.** Composé selon la revendication 3, qui est un composé de formule (2a) :

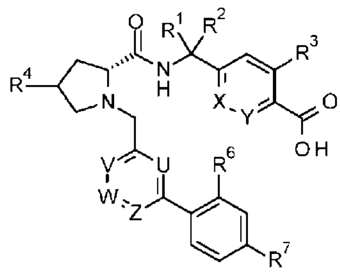 (2a),

ou un sel pharmaceutiquement acceptable, solvate, hydrate, tautomère ou isomère optique de celui-ci, dans lequel U, V, W, X, Y, Z, $R^1$, $R^2$, $R^3$, $R^4$, $R^6$, et $R^7$ sont les mêmes que ceux définis dans la revendication 3.

**8.** Composé selon l'une quelconque des revendications 1 à 7, dans lequel $R^1$ et $R^2$ sont indépendamment H ou alkyle en $C_{1-3}$ facultativement substitué par 1 à 3 atomes de fluor.

**9.** Composé selon l'une quelconque des revendications 1 à 7, dans lequel $R^1$ est H ou méthyle ou est relié à $R^2$ pour former un cycle cyclopropane-1,1-diyle ; de manière davantage préférée dans lequel $R^1$ est méthyle ; et/ou dans lequel $R^2$ est H.

**10.** Composé selon la revendication 3, qui est un composé de formule (3a) :

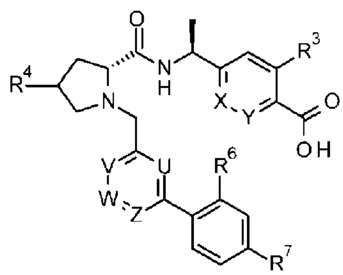 (3a),

ou un sel pharmaceutiquement acceptable, solvate, hydrate, tautomère ou isomère optique de celui-ci, dans lequel U, V, W, X, Y, Z, $R^3$, $R^4$, $R^6$, et $R^7$ sont les mêmes que ceux définis dans la revendication 3.

**11.** Composé selon l'une quelconque des revendications 1 à 10, dans lequel $R^3$ est H, OH ou F ; de préférence dans lequel $R^3$ est H ou OH ; de manière davantage préférée dans lequel $R^3$ est H.

**12.** Composé selon l'une quelconque des revendications 1 à 11, dans lequel $R^4$ est H, OH ou F ; de préférence dans lequel $R^4$ est H ou OH ; de manière davantage préférée dans lequel $R^4$ est H.

**13.** Composé selon l'une quelconque des revendications 1 à 12, dans lequel $R^6$ est H, OH, CN, halo, alcoxy en $C_{1-3}$ facultativement substitué par 1 à 3 atomes de fluor ou alkyle en $C_{1-3}$ facultativement substitué par 1 à 3 atomes de fluor ; de préférence dans lequel $R^6$ est H, OH, CN ou méthyle ; de manière davantage préférée dans lequel $R^6$ est méthyle.

**14.** Composé selon l'une quelconque des revendications 1 à 13, dans lequel $R^7$ est OH, $CO_2H$, $CONH_2$, $SO_2NH_2$ ou $OSO_2NH_2$ ; de préférence dans lequel $R^7$ est $CONH_2$ ou $SO_2NH_2$.

**15.** Composé selon l'une quelconque des revendications 1 à 14, dans lequel X et Y sont chacun indépendamment sélectionnés dans le groupe consistant en CH, CF, COH ou N ; de préférence dans lequel X est CH et Y est CH ou COH ; de manière davantage préférée dans lequel X et Y sont tous les deux CH.

**16.** Composé selon l'une quelconque des revendications 3 à 15, dans lequel U, V, W et Z sont CH, ou dans lequel U, V et Z sont CH et W est COH.

**17.** Composé selon la revendication 1, dans lequel le composé est sélectionné parmi :

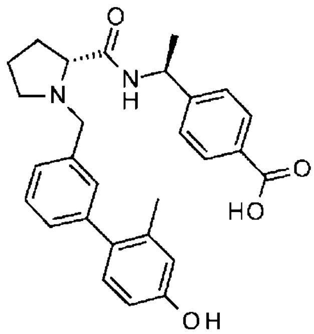
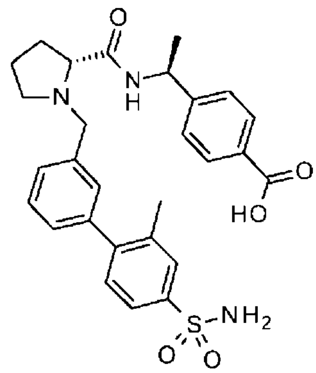
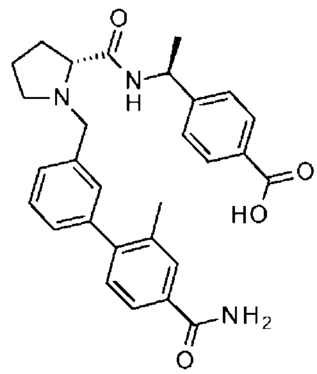

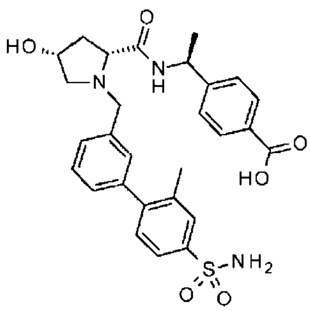
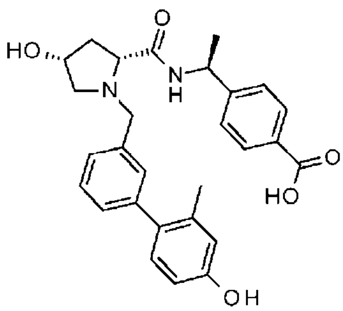
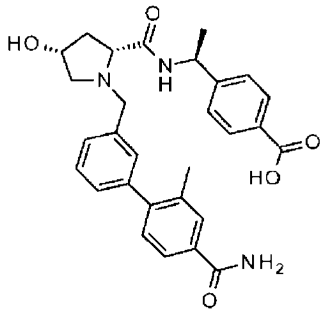

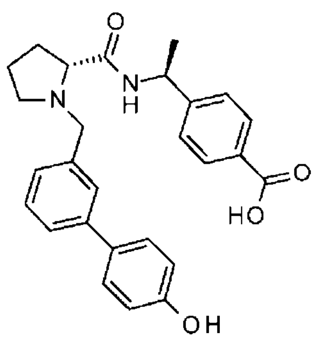
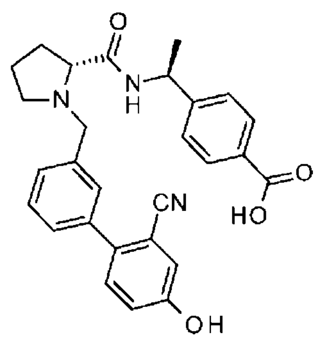
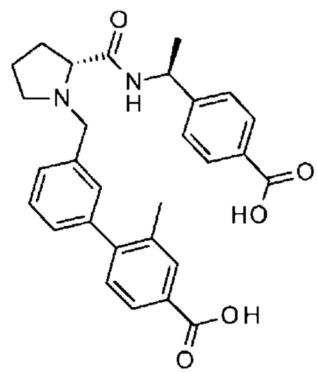

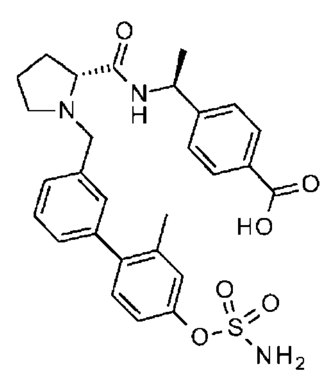
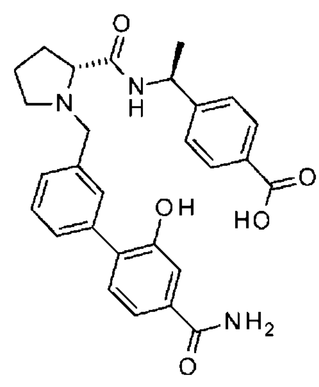
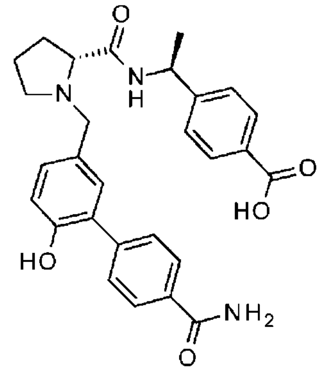

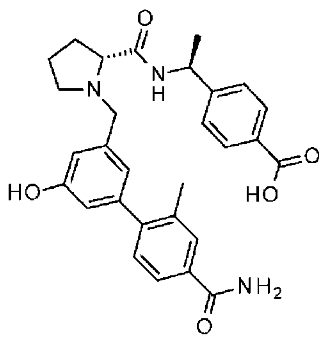
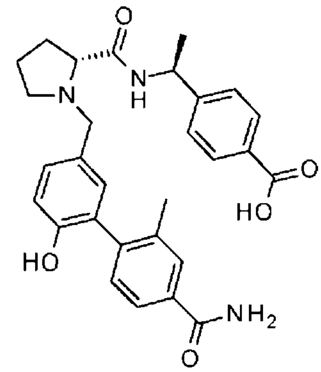
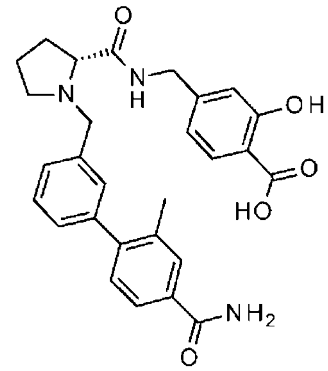

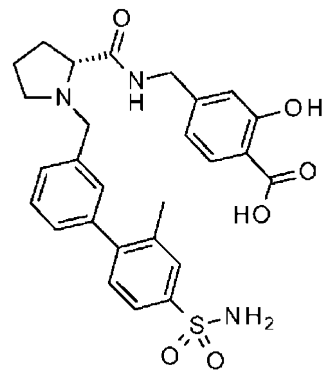
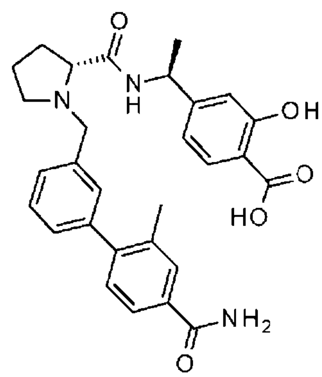
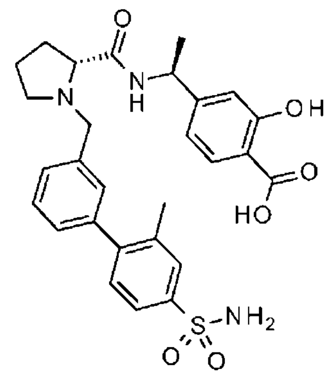

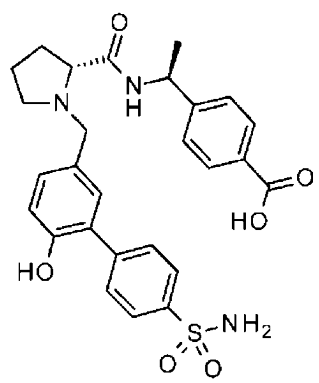
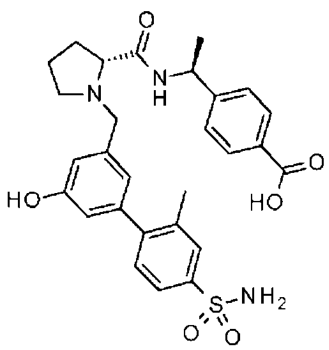
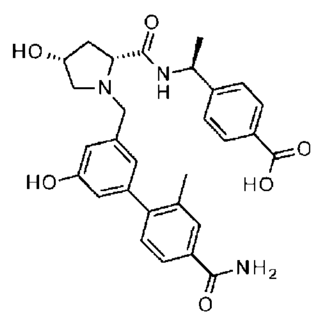

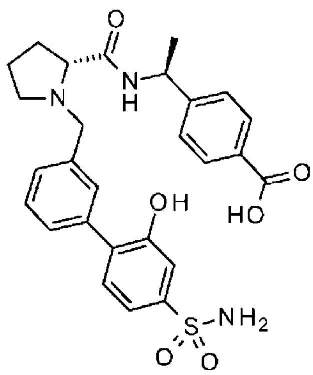
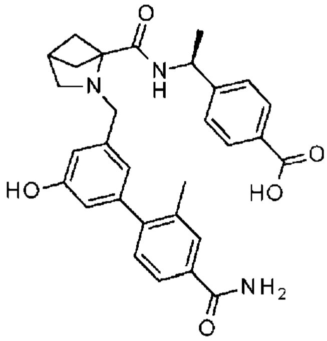
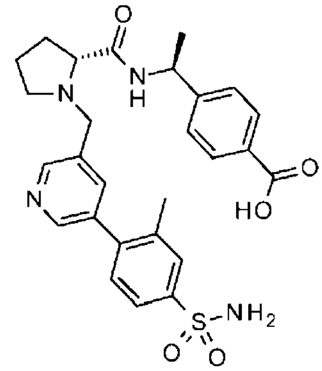

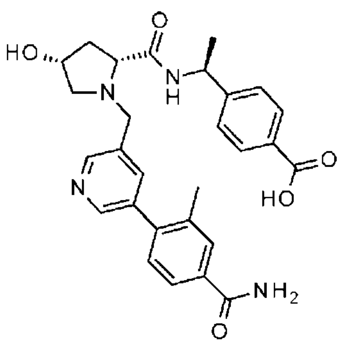
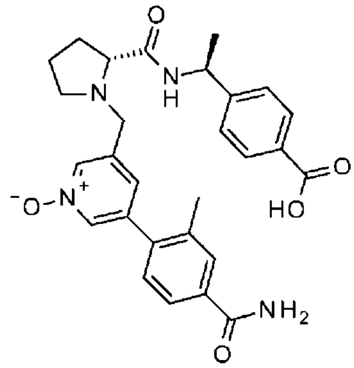
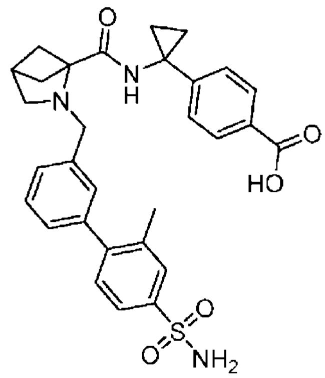

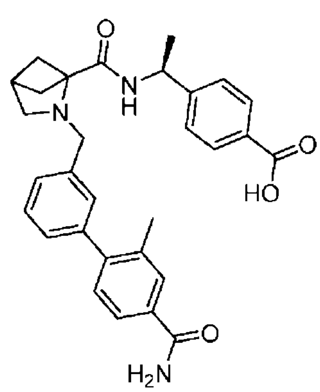
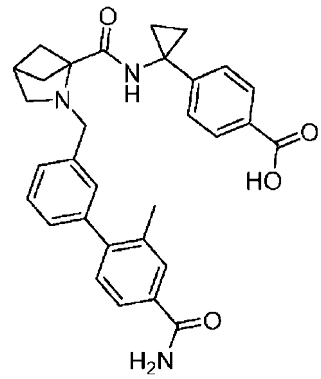
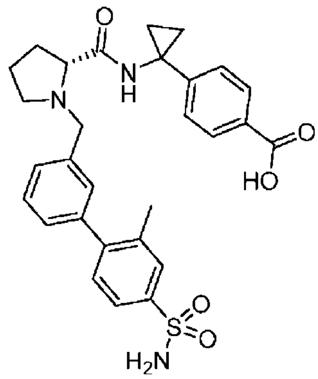

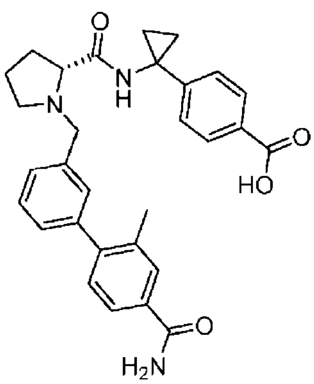
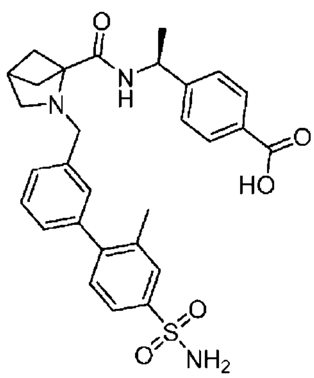
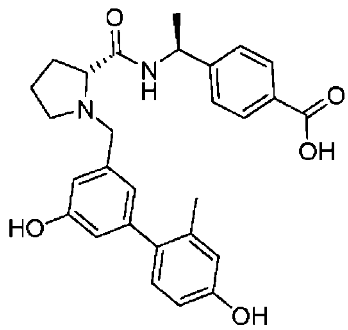

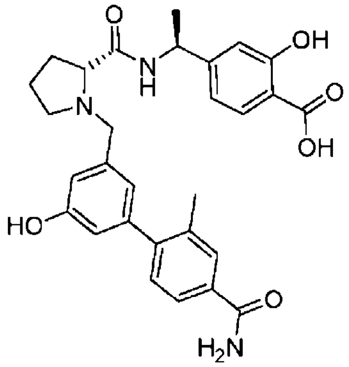
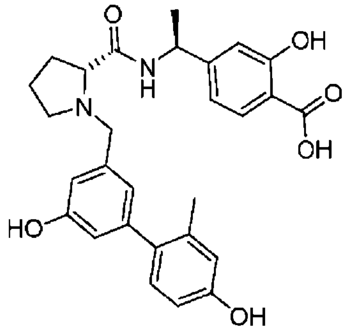

ou sel pharmaceutiquement acceptable, solvate, hydrate, ou tautomère de celui-ci.

**18.** Composition pharmaceutique comprenant un composé, sel pharmaceutiquement acceptable, solvate, hydrate, tautomère ou isomère optique selon l'une quelconque des revendications 1 à 17, et un excipient pharmaceutiquement acceptable ; de préférence dans laquelle la composition comprend en outre au moins un agent thérapeutique additionnel sélectionné dans le groupe consistant en aminosalicylates, corticostéroïdes, immunomodulateurs et des combinaisons de ceux-ci.

**19.** Kit comprenant un composé, sel pharmaceutiquement acceptable, solvate, hydrate, tautomère ou isomère optique selon l'une quelconque des revendications 1 à 17 et au moins un agent thérapeutique additionnel sélectionné dans le groupe consistant en aminosalicylates, corticostéroïdes, immunomodulateurs et des combinaisons de ceux-ci.

**20.** Composé selon l'une quelconque des revendications 1 à 17, composition selon la revendication 18, ou kit selon la revendication 19 pour une utilisation en médecine.

**21.** Composé selon l'une quelconque des revendications 1 à 17, composition selon la revendication 18, ou kit selon la revendication 19, pour une utilisation dans le traitement d'une maladie médiée par le récepteur EP4, dans lequel la maladie médiée par le récepteur EP4 est un trouble gastrointestinal ou une maladie ou affection pulmonaire.

**22.** Composé, composition ou kit pour une utilisation selon la revendication 21, dans lequel :

(i) le trouble gastro-intestinal est sélectionné dans le groupe consistant en troubles de constipation, syndrome de l'intestin irritable à prédominance constipation, syndrome de l'intestin irritable de type mixte, constipation idiopathique chronique, symptômes gastro-intestinaux associés à la maladie de Parkinson, symptômes gastro-intestinaux associés à la fibrose kystique, dysmotilité intestinale, iléus postopératoire, allergie alimentaire ou intolérance alimentaire, maladie cœliaque, troubles de la motilité gastro-intestinale, troubles gastro-intestinaux fonctionnels, entéropathie induite par des médicaments, lésion gastrique et intestinale induite par des AINS, mucosite induite par une chimiothérapie, maladie de reflux gastro-œsophagien (GERD), reflux duodénogastrique, maladies diarrhéiques, maladies gastro-intestinales médiées par le système immunitaire, maladie de Crohn, colite ulcéreuse, maladie inflammatoire de l'intestin, et colite ischémique ; et/ou
(ii) la maladie ou affection pulmonaire est sélectionnée parmi les maladies pulmonaires obstructives chroniques, l'asthme, la bronchite chronique, la fibrose cystique, l'emphysème, la toux chronique idiopathique, le trouble des voies aériennes hyperactif, et la fibrose pulmonaire idiopathique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013004291 A1


### Non-patent literature cited in the description


- **A HONDA et al.** *J. Biol. Chem.*, 1993, vol. 268, 7759-7762
- **MIYOSHI, H. et al.** *EMBO J.*, 2017, vol. 36, 5-24
- **KABASHIMA, K. et al.** *J. Clin. Invest.*, 2002, vol. 109, 883-893
- **WATANABE, Y. et al.** *Eur. J. Pharmacol.*, 2015, vol. 754, 179-189
- **NITTA, M. et al.** *Scand. J. Immunol.*, 2002, vol. 56, 66-75
- **NAKASE, H. et al.** *Inflamm. Bowel Dis.*, 2010, vol. 16, 731-733
- **BUCKLEY, J. et al.** *Thorax*, 2011, vol. 66, 1029-1035
- **HONDA, A. et al.** *Eur. J. Pharmacol.*, 2016, vol. 775, 130-137
- **BRYN et al.** Solid-State Chemistry of Drugs. SSCI, Inc of West Lafayette, 1999
- **HOLFORD, N.H.G.** ; **SCHEINER, L.B.** *Clin. Pharmacokinet.*, 1981, vol. 6, 429-453
- **LOEWE, S.** ; **MUISCHNEK, H.** *Arch. Exp. Pathol Pharmacol.*, 1926, vol. 114, 313-326
- **CHOU, T.C.** ; **TALALAY, P.** *Adv. Enzyme Regul.*, 1984, vol. 22, 27-55